# EUROPÄISCHE PATENTSCHRIFT

(11) **EP 1 501 361 B1**
(45) Veröffentlichungstag und Bekanntmachung des Hinweises auf die Patenterteilung: **30.09.2009**
(21) Anmeldenummer: 03725066.9
(22) Anmeldetag: 22.04.2003
(51) Int. Cl.: A01N 43/80, C07D 413/04, C07D 417/04

(54) **3-HETEROARYLSUBSTITUIERTE 5-METHYLOXYMETHYL-ISOXAZOLINE ALS HERBIZIDE**
3-HETEROARYL SUBSTITUTED 5-METHYLOXYMETHYL ISOXAZOLINES USED AS HERBICIDES
ISOXAZOLINES 5-METHYLOXYMETHYLIQUES A SUBSTITUTION 3-HETEROARYLE UTILISEES COMME HERBICIDES

(30) Priorität: 25.04.2002 DE 10218620
(43) Veröffentlichungstag der Anmeldung: 02.02.2005
(73) Patentinhaber: BASF SE, 67056 Ludwigshafen (DE)
(72) Erfinder: WAGNER, Oliver, 67433 Neustadt (DE); RACK, Michael, 69123 Heidelberg (DE); WITSCHEL, Matthias, 67098 Bad Dürkheim (DE); ZAGAR, Cyrill, 68167 Mannheim (DE); LANDES, Andreas, 67354 Römerberg-Heiligenstein (DE)
(86) Internationale Anmeldenummer: PCT/EP2003/004136
(87) Internationale Veröffentlichungsnummer: WO 2003/090539

(56) Entgegenhaltungen:
- EP-A- 0 334 120
- WO-A-02/19825
- JP-A- 2001 158 787

## Beschreibung

Die vorliegende Erfindung betrifft 3-heteroarylsubstituierte Isoxazoline der Formel I 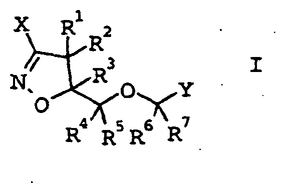 in der die Variablen die folgenden Bedeutungen haben:
- X: 6-gliedriges Heteroaryl mit ein bis vier Stickstoffatomen,
das partiell oder vollständig halogeniert ist und/oder ein bis drei Substituenten aus folgender Gruppe trägt:
Cyano, Nitro, Hydroxy, C₁-C₈-Alkyl, C₃-C₆-Cycloalkyl, C₃-C₆-Halogencycloalkyl, C₂-C₈-Alkenyl, C₂-C₈-Alkiriyl, C₁-C₈-Halogenalkyl, C₂-C₆-Halogenalkenyl, C₂-C₆-Halogenalkinyl, C₁-C₈-Cyanoalkyl, C₂-C₈-Cyanoalkenyl, C₁-C₈-Alkoxy, C₁-C₈-Halogenalkoxy, C₁-C₈-Alkoxy-C₁-C₈-alkyl, C₁-C₈-Alkoxy-C₁-C₈-alkoxy, Amino, C₁-C₈-Alkylamino, Di(C₁-C₈-alkyl)amino, C₁-C₈-Alkylcarbonylamino, N-C₁-C₈-Alkylcarbonyl-N-C₁-C₈-alkylamino, C₁-C₈-Alkoxycarbonylamino, N-C₁-C₈-Alkoxycarbonyl-N-C₁-C₈-alkylamino, Amino-C₁-C₈-alkyl, C₁-C₈-Alkylamino-C₁-C₈-alkyl, Di(C₁-C₈alkyl)amino-C₁-C₈-alkyl, C₁-C₈-Alkylthio, C₁-C₈-Halogenalkylthio, C₁-C₈-Alkylthio-C₁-C₈-alkyl, C₁-C₈-Alkylsulfinyl, C₁-C₈-Halogenalkylsulfinyl, C₁-C₈-Alkylsulfonyl, C₁-C₈-Halogenalkylsulfonyl, C₁-C₈-Alkylsulfonyl-C₁-C₈-alkyl, C₁-C₈-Alkylcarbonyl, C₁-C₈-Alkoxycarbonyl, C₁-C₈-Alkoxycarbonyl-C₁-C₈-alkyl, Aminocarbonyl, C₁-C₈-Alkylaminocarbonyl, Di(C₁-C₈-alkyl)aminocarbonyl, Aminocarbonyl-C₁-C₈-alkyl, C₁-C₈-Alkylaminocarbonyl-C₁-C₈-alkyl, Di(C₁-C₈-alkyl)aminocarbonyl-C₁-C₈-alkyl, Carboxyl, Carboxy-C₁-C₈-alkyl, Phenyl, Phenoxy, Phenylthio, Phenylamino, Phenyl-C₁-C₈-alkyl, Phenyl-C₁-C₈-alkoxy, 3- bis 6-gliedriges Heterocyclyl und 5- oder 6-gliedriges Heteroaryl, wobei die acht letztgenannten Reste ihrerseits partiell oder vollständig halogeniert sein können und/oder ein bis drei Substituenten aus der Gruppe Nitro, Cyano, C₁-C₈-Alkyl, C₁-C₈-Alkoxy, C₁-C₈-Halogenalkyl, C₁-C₈-Halogenalkoxy, C₁-C₈-Alkylthio, C₁-C₈-Halogenalkylthio, C₁-C₈-Alkylsulfinyl, C₁-C₈-Halogenalkylsulfinyl, C₁-C₈-Alkylsulfonyl und C₁-C₈-Halogenalkylsulfonyl tragen können;
- R¹ - R⁷: Wasserstoff, C₁-C₆-Alkyl oder C₁-C₆-Halogenalkyl;
- Y: Aryl, das partiell oder vollständig halogeniert sein kann und/oder ein bis drei Substituenten aus der Gruppe Cyano, Nitro, C₁-C₆-Alkyl, C₂-C₆-Alkenyl, C₂-C₆-Alkinyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkoxycarbonyl, C₁-C₆-Alkoxycarbonyl-C₁-C₄-alkoxy, C₁-C₆-Alkylsulfonyl, Phenyl, Phenoxy und Phenylcarbonyl, Benzo[1,4]dioxonyl, Benzo[1,3]dioxolanyl, 2,3-Dihydrobenzofuranyl oder Benzimidazol; oder
5-gliedriges Heteroaryl mit ein bis vier Stickstoffatomen, oder mit ein bis drei Stickstoffatomen und einem Sauerstoff- oder Schwefelatom, oder mit einem Sauerstoffoder Schwefelatom; oder 6-gliedriges Heteroaryl mit ein bis vier Stickstoffatomen;
wobei die Heterocyclen partiell oder vollständig halogeniert sein können und/oder ein bis drei Substituenten aus der Gruppe Cyano, C₁-C₆-Alkyl, C₁-C₆-Halogenalkyl, C₁-C₆-Alkoxy-C₁-C₄-alkyl, C₁-C₆-Alkoxycarbonyl und C₁-C₆-Alkylsulfonyl tragen können;
sowie deren landwirtschaftlich brauchbaren Salze.

Außerdem betrifft die Erfindung Verfahren und Zwischenprodukte zur Herstellung von Verbindungen der Formel I, Mittel welche diese enthalten sowie die Verwendung dieser Derivate oder diese enthaltende Mittel zur Schadpflanzenbekämpfung.

Aus der Literatur, beispielsweise aus EP 514 987 sind 3-phenylsubstituierte Isoxazoline bekannt. In EP 334 120 werden Isoxazoline, welche in 3-Position durch einen unsubstituierten Heterocyclus subsitituiert sind, offenbart. Weiterhin werden in JP 2001/158787 3-Pyrazolylsubstituierte Isoxazoline beschrieben. Aus WO 02/19825 und KR 2002/19751 sind thienylsubstituierte Isoxazoline bekannt. In JP 09/143171 werden Isoxazoline, welche in 3-Position durch eine Carbonyl- oder Oximgruppe substituiert sind, genannt.

Die herbiziden Eigenschaften der bisher bekannten Verbindungen bzw. die Verträglichkeiten gegenüber Kulturpflanzen können jedoch nur bedingt befriedigen. Es lag daher dieser Erfindung die Aufgabe zugrunde, neue, insbesondere herbizid wirksame, Verbindungen mit verbesserten Eigenschaften zu finden.

Demgemäß wurden die 3-heteroarylsubstituierten Isoxazoline der Formel I sowie deren herbizide Wirkung gefunden.

Ferner wurden herbizide Mittel gefunden, welche die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Die Verbindungen der Formel I können je nach Substitutionsmuster ein oder mehrere Chiralitätszentren enthalten und liegen dann als Enantiomeren oder Diastereomerengemische vor. Gegenstand der Erfindung sind sowohl die reinen Enantiomeren oder Diastereomeren als auch deren Gemische.

Die Verbindungen der Formel I können auch in Form ihrer landwirtschaftlich brauchbaren Salze vorliegen, wobei es auf die Art des Salzes in der Regel nicht ankommt. Im allgemeinen kommen die Salze derjenigen Kationen oder die Säureadditionssalze derjenigen Säuren in Betracht, deren Kationen, beziehungsweise Anionen, die herbizide Wirkung der Verbindungen I nicht negativ beeinträchtigen.

Es kommen als Kationen insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, wobei hier gewünschtenfalls ein bis vier Wasserstoffatome durch C₁-C₄-Alkyl, Hydroxy-C₁-C₄-alkyl, C₁-C₄-Alkoxy-C₁-C₄-alkyl, Hydroxy-C₁-C₄-alkoxy-C₁-C₄-alkyl, Phenyl oder Benzyl ersetzt sein können, vorzugsweise Ammonium, Dimethylammonium, Diisopropylammonium, Tetramethylammonium, Tetrabutylammonium, 2-(2-Hydroxyeth-1-oxy)eth-1-ylammonium, Di (2-hydroxyeth-1-yl) ammonium, Trimethylbenzylammonium, des weiteren Phosphoniumionen, Sulfoniumionen, vorzugsweise Tri(C₁-C₄-alkyl)sulfonium und Sulfoxoniumionen, vorzugsweise Tri(C₁-C₄-alkyl)sulfoxonium, in Betracht.

Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat und Butyrat.

Die für die Substituenten R¹-R⁷ oder als Reste an Aryl- bzw. Heteroarylringen genannten organischen Molekülteile stellen Sammelbegriffe für individuelle Aufzählungen der einzelnen Gruppenmitglieder dar. Sämtliche Kohlenwasserstoffketten, also alle Alkyl-, Halogenalkyl-, Cyanoalkyl-, Alkoxy-, Halogenalkoxy-, Alkycarbonyloxy, Alkylthio-, Halogenalkylthio-, Alkylsulfinyl-, Halogenalkylsulfinyl-, Alkylsulfonyl-, Halogenalkylsulfonyl-, Alkylcarbonyl, Alkoxycarbonyl-, Halogenalkoxycarbonyl, Alkylamino, Dialkylamino-, Alkylcarbonylamino-, N-Alkylcarbonyl-N-alkylamino, Alkoxyalkyl-, Alkoxyalkoxy, Alkylthioalkyl-, Dialkylaminoalkyl- und Alkoxycarbonylalkyl-Teile können geradkettig oder verzweigt sein. Sofern nicht anders angegeben tragen halogenierte Substituenten vorzugsweise ein bis fünf gleiche oder verschiedene Halogenatome. Die Bedeutung Halogen steht jeweils für Fluor, Chlor, Brom oder Iod.

Ferner bedeuten beispielsweise:
- C₁-C₄-Alkyl sowie die Alkylteile von C₁-C₄-Alkoxy-C₁-C₄-alkyl, C₁-C₄-Alkylthio-C₁-C₄-alkyl, C₁-C₄-Alkylsulfonyl-C₁-C₄-alkyl, C₁-C₄-Alkylcarbonyl, C₁-C₄-Alkylcarbonylamino, N-C₁-C₄-Alkylcarbonyl-N-C₁-C₄-alkylamino, N-C₁-C₄-Alkoxycarbonyl-N-C₁-C₄-alkylamino, Amino-C₁-C₄-alkyl, C₁-C₄-Alkylamino-C₁-C₄-alkyl, Di (C₁-C₄-alkyl)amino-C₁-C₄-alkyl, Carboxy-C₁-C₄-alkyl, C₁-C₄-Alkoxycarbonyl-C₁-C₄-alkyl, Aminocarbonyl-C₁-C₄-alkyl, C₁-C₄-Alkylaminocarbonyl-C₁-C₄-alkyl, Di(C₁-C₄-alkyl)aminocarbonyl-C₁-C₄-alkyl und Phenyl-C₁-C₄-alkyl: z.B. Methyl, Ethyl, Propyl, 1-Methylethyl, Butyl, 1-Methylpropyl, 2-Methylpropyl und 1,1-Dimethylethyl;
- C₁-C₆-Alkyl: C₁-C₄-Alkyl, wie voranstehend genannt, sowie die Alkylteile von C₁-C₆-Alkoxy-C₁-C₆-alkyl, C₁-C₆-Alkylthio-C₁-C₆-alkyl, C₁-C₆-Alkylsulfonyl-C₁-C₆-alkyl, C₁-C₆-Alkylcarbonyl, C₁-C₆-Alkylcarbonylamino, N-C₁-C₆-Alkylcarbonyl-N-C₁-C₆-alkylamino, N-C₁-C₆-Alkoxycarbonyl-N-C₁-C₆-alkylamino, Amino-C₁-C₆-alkyl, C₁-C₆-Alkylamino-C₁-C₆-alkyl, Di(C₁-C₆-alkyl)amino-C₁-C₆-alkyl, Carboxy-C₁-C₆-alkyl, C₁-C₆-Alkoxycarbonyl-C₁-C₆-alkyl, Aminocarbonyl-C₁-C₆-alkyl, C₁-C₆-Alkylaminocarbonyl-C₁-C₆-alkyl, Di(C₁-C₆-alkyl)aminocarbonyl-C₁-C₆-alkyl und Phenyl-C₁-C₆-alkyl, sowie z.B. Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl und 1-Ethyl-3-methylpropyl;
- C₁-C₈-Alkyl: C₁-C₆-Alkyl, wie voranstehend genannt, sowie die Alkylteile von C₁-C₈-Alkoxy-C₁-C₈-alkyl, C₁-C₈-Alkylthio-C₁-C₈-alkyl, C₁-C₈-Alkylsulfonyl-C₁-C₈-alkyl, C₁-C₈-Alkylcarbonyl, C₁-C₈-Alkylcarbonylamino, N-C₁-C₈-Alkylcarbonyl-N-C₁-C₈-alkylamino, N-C₁-C₈-Alkoxycarbonyl-N-C₁-C₈- alkylamino, Amino-C₁-C₈-alkyl, C₁-C₈-Alkylamino-C₁-C₈-alkyl, Di (C₁-C₈-alkyl)amino-C₁-C₈-alkyl, Carboxy-C₁-C₈-alkyl, C₁-C₈-Alkoxycarbonyl-C₁-C₈-alkyl, Aminocarbonyl-C₁-C₈-alkyl, C₁-C₈-Alkylaminocarbonyl-C₁-C₈-alkyl, Di(C₁-C₈-alkyl)aminocarbonyl-C₁-C₈-alkyl und Phenyl-C₁-C₈-alkyl, sowie z.B. Heptyl, 2-Methylhexyl, 3-Methylhexyl, 2,2-Dimethylpentyl, 2,3-Dimethylpentyl, 2,4-Dimethylpentyl, 3,3-Dimethylpentyl, 2,2,3-Trimethylbutyl, Octyl, 2-Methylheptyl, 3-Methylheptyl, 4-Methylheptyl, 2,2-Diemthylhexyl, 2,3-Dimethylhexyl, 2,4-Dimethylhexyl, 3,3-Dimethylhexyl, 2,2,3-Trimethylpentyl, 2,3,3-Trimethylpentyl, 2,3,4-Trimethylpentyl und 2,2,3,3-Tetramethylbutyl;
- C₃-C₆-Cycloalkyl: Cyclopropyl, Cyclobutyl, Cyclopentyl und Cyclohexyl;
- C₃-C₆-Halogencycloalkyl: C₃-C₆-Cycloalkyl, wie voranstehend genannt, der partiell oder vollständig duch Fluor, Chlor, Brom und/oder Jod substituiert ist, also z. B. 2,2-Difluorcyclopropyl, 2,2-Dichlorcyclopropyl, 3-Fluorcyclopentyl, 3-Chlorcyclopentyl, 3,3-Difluorcyclopentyl, 3,3-Dichlorcyclopentyl, 4-Fluorcyclohexyl, 4-Chlorcyclohexyl, 4,4-Difluorcyclohexyl und 4,4-Dichlorcyclohexyl;
- drei- bis sechsgliedriges Heterocyclyl: monocyclischer, gesättigter oder partiell ungesättigter Kohlenwasserstoff mit drei bis sechs Ringliedern wie voranstehend genannt, welche neben Kohlenstoffatomen ein bis vier Stickstoffatome, oder ein bis drei Stickstoffatome und ein Sauerstoff- oder Schwefelatom, oder ein Sauerstoff- und/oder ein Schwefelatom oder 2 Sauerstoff- oder 2 Schwefelatome enthalten können und welche über ein C-Atom oder ein N-Atom verknüpft sein können, z.B.
   3- bis 4-gliedriges Heterocyclyl:
   2-Oxrianyl, 2-Oxetanyl, 3-Oxetanyl, 2-Aziridinyl, 3-Thiethanyl, 1-Azetinyl, 2-Azetinyl;
   5-gliedriges gesättigtes Hererocyclyl, verknüpft über ein C-Atom:
   2-Tetrahydrofuranyl, 3-Tetrahydrofuranyl, 1,3-Dioxolan-2-yl, 1,3-Dioxolan-5-yl, 2-Tetrahydrothienyl, 3-Tetrahydrothienyl, 1,3-Dithiolan-2-yl, 1,3-Dithiolan-5-yl, 1,2-Dithiolan-3-yl, 1,2-Dithiolan-4-yl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl;
   5-gliedriges gesättigtes Hererocyclyl, verknüpft über ein N-Atom:
   1-Pyrrolidinyl, 2-Isothiazolidinyl, 2-Isothiazolidinyl, 1-Pyrazolidinyl, 3-Oxazolidinyl, 3-Thiazolidinyl, 1-Imidazolidinyl, 1,2,4-Triazolidin-1-yl, 1,2,4-Oxadiazolidin-2-yl, 1,2,4-Oxadiazolidin-4-yl, 1,2,4-Thiadiazolidin-2-yl, 1,2,4-Thiadiazolidin-4-yl;
   5-gliedriges partiell ungesättigtes Hererocyclyl; verknüpft über ein C-Atom:
   2,3-Dihydrofur-2-yl, 2,3-Dihydrofur-3-yl, 2,3-Dihydrofur-4-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 4,5-Dihydropyrrol-2-yl, 4,5-Dihydropyrrol-3-yl, 2,5-Dihydropyrrol-2-yl, 2,5-Dihydropyrrol-3-yl, 4,5-Dihydroisoxazol-3-yl, 2,5-Dihydroisoxazol-3-yl, 2,3-Dihydroisoxazol-3-yl, 4,5-Dihydroisoxazol-4-yl, 2,5-Dihydroisoxazol-4-yl, 2,3-Dihydroisoxazol-4-yl, 4,5-Dihydroisoxazol-5-yl, 2,5-Dihydroisoxazol-5-yl, 2,3-Dihydroisoxazol-5-yl, 4,5-Dihydroisothiazol-3-yl, 2,5-Dihydroisothiazol-3-yl, 2,3-Dihydroisothiazol-3-yl, 4,5-Dihydroisothiazol-4-yl, 2,5-Dihydroisothiazol-4-yl, 2,3-Dihydroisothiazol-4-yl, 4,5-Dihydroisothiazol-5-yl, 2,5-Dihydroisothiazol-5-yl, 2,3-Dihydroisothiazol -5-yl, 2,3-Dihydropyrazol-3-yl, 2,3-Dihydropyrazol-4-yl, 2,3-Dihydropyrazol-5-yl, 3,4-Dihydropyrazol-3-yl, 3,4-Dihydropyrazol-4-yl, 3,4-Dihydropyrazol-5-yl, 4,5-Dihydropyrazol-3-yl, 4,5-Dihydropyrazol-4-yl, 4,5-Dihydropyrazol-5-yl, 2,3-Dihydroimidazol-2-yl, 2,3-Dihydroimidazol-4-yl, 2,3-Dihydroimidazol-5-yl, 4,5-Dihydroimidazol-2-yl, 4,5-Dihydroimidazol-4-yl, 4,5-Dihydroimidazol-5-yl, 2,5-Dihydroimidazol-2-yl, 2,5-Dihydroimidazol-4-yl, 2,5-Dihydroimidazol-5-yl, 2,3-Dihydrooxozol-2-yl, 2,3-Dihydrooxazol-4-yl, 2,3-Dihydrooxazol-5-yl, 3,4-Dihydrooxazol-2-yl, 3,4-Dihydrooxazol-4-yl, 3,4-Dihydrooxazol-5-yl, 2,3-Dihydrothiazol-2-yl, 2,3-Dihydrothiazol-4-yl, 2,3-Dihydrothiazol-5-yl, 3,4-Dihydrothiazol-2-yl, 3,4-Dihydrothiazol-4-yl, 3,4-Dihydrothiazol-5-yl;
   5-gliedriges partiell ungesättigtes Hererocyclyl verknüpft über ein N-Atom:
   4,5-Dihydropyrrol-1-yl, 2,5-Dihydropyrrol-1-yl, 4,5-Dihydroisoxazol-2-yl, 2,3-Dihydroisoxazol-1-yl, 4,5-Dihydroisothiazol-1-yl, 2,3-Dihydroisothiazol-1-yl, 2,3-Dihydropyrazol-1-yl, 2,3-Dihydropyrozol-2-yl, 4,5-Dihydropyrazol-1-yl, 3,4-Dihydropyrazol-1-yl, 2,3-Dihydroimidazol-1-yl, 2,3-Dihydroimidozol-3-yl, 4,5-Dihydroimidazol-1-yl, 2,5-Dihydroimidazol-1-yl, 2,3-Dihydrooxazol-3-yl, 3,4-Dihydrooxazol-3-yl, 2,3-Dihydrothiazol-3-yl, 3,4-Dihydrothiazol-3-yl;
   6-gliedriges gesättigtes Heterocyclyl verknüpft über ein C-Atom:
   2-Piperidinyl, 3-Piperidinyl, 4-Piperidinyl, 1,3-Dioxan-5-yl, 1,3-Dithian-5-yl, 2-Tetrahydropyranyl, 4-Tetrahydropyranyl, 2-Tetrahydrothiopyranyl, 4-Tetrahydrothiopyranyl, 3-Hexahydropyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydrotriazin-2-yl, 1,2,4-Hexahydrotriazin-3-yl, Tetrahydro-1,3-oxazin-2-yl, Tetrahydro-1,3-oxazin-6-yl, 2-Morpholinyl, 3-Morpholinyl;
   6-gliedriges gesättigtes Heterocyclyl verknüpft über ein N-Atom:
   1-Piperidinyl, 1-Hexahydropyridazinyl, 1-Hexahydropyrimidinyl, 1-Piperazinyl, 1,3,5-Hexahydrotriazin-1-yl, 1,2,4-Hexahydrotriazin-1-yl, Tetrahydro-1,3-oxazin-1-yl, 1-Morpholinyl;
   6-gliedriges partiell ungesättigtes Heterocyclylverknüpft über ein C-Atom:
   2H-Pyran-2-yl, 2H-Pyran-3-yl, 2H-Pyran-4-yl, 2H-Pyran-5-yl, 2H-Pyran-6-yl, 2H-Thiopyran-2-yl, 2H-Thiopyran-3-yl, 2H-Thiopyran-4-yl, 2H-Thiopyran-5-yl, 2H-Thiopyran-6-yl, 5,6-Dihydro-4H-1,3-Oxazin-2-yl;
- C₂-C₆-Alkenyl: z.B. Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;
- C₂-C₈-Alkenyl: C₂-C₆-Alkenyl wie voranstehend genannt, sowie z.B. 1-Heptenyl, 2-Heptenyl, 3-Heptenyl, 2-Methyl-1-hexenyl, 2-Methyl-2-hexenyl, 2-Methyl-3-hexenyl, 2-Methyl-4-hexenyl, 2-Methyl-5-hexenyl, 3-Methyl-1-hexenyl, 3-Methyl-2-hexenyl, 3-Methyl-3-hexenyl, 3-Methyl-4-hexenyl, 3-Methyl-5-hexenyl, 2,2-Dimethyl-3-pentenyl, 2,2-Dimethyl-4-pentenyl, 2,3-Dimethyl-1-pentenyl, 2,3-Dimethyl-2-pentenyl, 2,3-Dimethyl-3-pentenyl, 2,3-Dimethyl-4-pentenyl, 2,4-Dimethyl-1-pentenyl. 2,4-Dimethyl-2-pentenyl, 3,3-Dimethyl-1-pentenyl, 2,2-Dimethyl-3-methyl-3-butentyl, 1-Octenyl, 2-Octenyl, 3-Octenyl, 4-Octenyl, 2-Methyl-1-heptenyl, 2-Methyl-2-heptenyl, 2-Methyl-3-heptenyl, 2-Methyl-4-heptenyl, 2-Methyl-5-heptenyl, 2-Methyl-6-heptenyl, 3-Methyl-1-heptenyl, 3-Methyl-2-heptenyl, 3-Methyl-3-heptenyl, 3-Methyl-4-heptenyl, 3-Methyl-5-heptenyl, 3-Methyl-6-heptenyl, 4-Methyl-1-heptenyl, 4-Methyl-2-heptenyl, 4-Methyl-3-heptenyl, 2,2-Dimethyl-3-hexenyl, 2,2-Dimethyl-4-hexenyl, 2,2-Dimethyl-5-hexenyl, 2,3-Dimethyl-1-hexenyl, 2,3-Dimethyl-2-hexenyl, 2,3-Dimethyl-3-hexenyl, 2,3-Dimethyl-4-hexenyl, 2,3-Dimethyl-5-hexenyl, 2,4-Dimethyl-1-hexenyl, 2,4-Dimethyl-2-hexenyl, 2,4-Dimethyl-3-hexenyl, 2,4-Dimethyl-4-hexenyl, 2,4-Dimethyl-5-hexenyl, 3,3-Dimethyl-1-hexenyl, 3,3-Dimethyl-4-hexenyl, 3,3-Dimethyl-5-hexenyl, 2,2-Trimethyl-3-pentenyl, 2,2,3-Trimethyl-4-pentenyl, 2,3,3-Trimethyl-1-pentenyl, 2,3,3-Trimethyl-4-pentenyl, 2,3,4-Trimethyl-1-pentenyl und 2,3,4-Trimethyl-2-pentenyl;
- C₂-C₆-Alkinyl: z.B. Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
- C₂-C₈-Alkinyl: C₂-C₆-Alkinyl wie voranstehend genannt, sowie z.B. 1-Heptinyl, 2-Heptinyl, 3-Heptinyl, 2-Methyl-3-hexinyl, 2-Methyl-4-hexinyl, 2-Methyl-5-hexinyl, 3-Methyl-1-hexinyl, 3-Methyl-4-hexinyl, 3-Methyl-5-hexinyl, 2,2-Dimethyl-3-pentinyl, 2,2-Dimethyl-4-pentinyl, 2,3-Dimethyl-4-pentinyl, 3,3-Dimethyl-1-pentinyl, 1-Octinyl, 2-Octinyl, 3-Octinyl, 4-Octinyl, 2-Methyl-3-heptinyl, 2-Methyl-4-heptinyl, 2-Methyl-5-heptinyl, 2-Methyl-6-heptinyl, 3-Methyl-1-heptinyl, 3-Methyl-4-heptinyl, 3-Methyl-5-heptinyl, 3-Methyl-6-heptinyl, 4-Methyl-1-heptinyl, 4-Methyl-2-heptinyl, 2,2-Dimethyl-3-hexinyl, 2,2-Dimethyl-4-hexinyl, 2,2-Dimethyl-5-hexinyl, 2,3-Dimethyl-4-hexinyl, 2,3-Dimethyl-5-hexinyl, 2,4-Dimethyl-5-hexinyl, 3,3-Dimethyl-1-hexinyl, 3,3-Dimethyl-4-hexinyl, 3,3-Dimethyl-5-hexinyl, 2,2,3-Trimethyl-4-pentinyl und 2,3,3-Trimethyl-4-pentinyl;
- C₁-C₄-Halogenalkyl: einen C₁-C₄-Alkylrest wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl und Nonafluorbutyl;
- C₁-C₆-Halogenalkyl: C₁-C₄-Halogenalkyl wie voranstehend genannt, sowie z.B. 5-Fluorpentyl, 5-Chlorpentyl, 5-Brompentyl, 5-Iodpentyl, Undecafluorpentyl, 6-Fluorhexyl, 6-Chlorhexyl, 6-Bromhexyl, 6-Iodhexyl und Dodecafluorhexyl;
- C₁-C₈-Halogenalkyl: C₁-C₆-Halogenalkyl wie voranstehend genannt, sowie z.B. 7-Fluorheptyl, 7-Chlorheptyl, 7-Bromheptyl, 7-Iodheptyl, Perfluorheptyl, 8-Fluoroctyl, 8-Chloroctyl, 8-Bromoctyl, 8-Iodoctyl und Octadecafluoroctyl;
- C₂-C₆-Halogenalkenyl: ein C₂-C₆-Alkenylrest wie voranstehend genannt, der partiell oder vollständig durch Fluor, Chlor, Brom und/oder Iod substituiert ist, z.B. 2-Chlorvinyl, 2-Chlorallyl, 3-Chlorallyl, 2,3-Dichlorallyl, 3,3-Dichlorallyl, 2,3,3-Trichlorallyl, 2,3-Dichlor-2-butenyl, 2-Bromvinyl, 2-Bromallyl, 3-Bromallyl, 2,3-Dibromallyl, 3,3-Dibromallyl, 2,3,3-Tribromallyl und 2,3-Dibrom-2-butenyl;
- C₂-C₈-Halogenalkenyl: ein C₁-C₆-Halogenalkenylrest wie voranstehend genannt, sowie z.B. 2-Chlor-1-heptenyl, 3-Chlor-1-heptenyl, 2,3-Dichlor-1-heptenyl, 3,3-Dichlor-1-heptenyl, 2,3,3-Trichlor-1-heptenyl, 2-Brom-1-heptenyl, 3-Brom-1-heptenyl, 2,3-Dibrom-1-heptenyl, 3,3-Dibrom-1-heptenyl, 2,3,3-Tribrom-1-heptenyl, 2-Chlor-1-octenyl, 3-Chlor-1-octenyl, 2,3-Dichlor-1-octenyl, 3,3-Dichlor-1-octenyl, 2,3,3-Trichlor-1-octenyl, 2-Brom-1-octenyl, 3-Brom-1-octenyl, 2,3-Dibrom-1-octenyl, 3,3-Dibrom-1-octenyl und 2,3,3-Tribrom-1-octenyl;
- C₂-C₆-Halogenalkinyl: ein C₂-C₆-Alkinylrest, wie voranstehend genannt, der partiell oder vollständig durch Fluor, Chlor, Brom und/oder Iod substituiert ist, z.B. 1,1-Difluorprop-2-in-1-yl, 3-Iod-prop-2-in-1-yl, 4-Fluorbut-2-in-1-yl, 4-Chlorbut-2-in-1-yl, 1,1-Difluorbut-2-in-1-yl, 4-Iodbut-3-in-1-yl, 5-Fluorpent-3-in-1-yl, 5-Iodpent-4-in-1-yl, 6-Fluorhex-4-in-1-yl und 6-Iodhex-5-in-1-yl;
- C₂-C₈-Halogenalkinyl: ein C₁-C₆-Halogenalkinylrest wie voranstehend genannt, sowie z.B. 7-Fluorhept-5-in-1-yl, 7-Iodhept-6-in-1-yl, 8-Fluoroct-6-in-1-yl und 8-Iodoct-7-in-1-yl;
- C₁-C₄-Cyanoalkyl: z.B. Cyanomethyl, 1-Cyanoethyl, 1-Cyanopropyl, 2-Cyanopropyl, 3-Cyanopropyl, 1-Cyanoprop-2-yl, 2-Cyanoprop-2-yl, 1-Cyanbutyl, 2-Cyanobutyl, 3-Cyanobutyl, 4-Cyanobutyl, 1-Cyanobutyl-2-yl, 2-Cyanobut-2-yl, 1-Cyanobut-3-yl, 2-Cyanobut-3-yl, 1-Cyano-2-methyl-prop-3-yl, 2-Cyano-2-methyl-prop-3-yl, 3-Cyano-2-methyl-prop-3-yl oder 2-Cyanomethyl-prop-2-yl;
- C₁-C₆-Cyanoalkyl: C₁-C₄-Cyanoalkyl wie voranstehend genannt sowie z.B. 5-Cyanopentyl oder 6-Cyanohexyl;
- C₁-C₈-Cyanoalkyl: C₁-C₆-Cyanoalkyl wie voranstehend genannt sowie z.B. 7-Cyanoheptyl oder 8-Cyanooctyl;
- C₂-C₆-Cyanoalkenyl: ein C₂-C₆-Alkenylrest, wie vorstehend genannt, der durch Cyano substituiert ist, z.B. 2-Cyanovinyl, 2-Cyanoallyl, 3-Cyanoallyl, 2-Cyanobut-2-enyl oder 3-Cyanobut-2-enyl;
- C₂-C₈-Cyanoalkenyl: ein C₂-C₆-Cyanoalkenylrest wie vorstehend genannt, sowie z.B. 2-Cyano-1-heptenyl, 3-Cyano-1-heptenyl, 2-Cyano-1-octenyl oder 3-Cyano-1-octenyl;
- C₁-C₄-Alkoxy: sowie die Alkoxyteile von C₁-C₄-Alkoxy-C₁-C₄-alkyl, C₁-C₄-Alkoxy-C₁-C₄-alkyl, C₁-C₄-Alkoxycarbonyl, C₁-C₄-Alkoxycarbonyl-C₁-C₄-alkyl, C₁-C₄-Alkoxycarbonylamino, N-C₁-C₄-Alkoxycarbonyl-N-C₁-C₄-alkylamino, C₁-C₄-Alkoxycarbonyl(C₁-C₄)alkoxy und Phenyl-C₁-C₄-alkoxy, z.B. Methoxy, Ethoxy, Propoxy, 1-Methylethoxy, Butoxy, 1-Methylpropoxy, 2-Methylpropoxy und 1,1-Dimethylethoxy;
- C₁-C₆-Alkoxy: C₁-C₄-Alkoxy wie voranstehend genannt, sowie die Alkoxyteile von C₁-C₆-Alkoxy-C₁-C₆-alkyl, C₁-C₆-Alkoxy-C₁-C₆-alkoxy, C₁-C₆-Alkoxycarbonyl, C₁-C₆-Alkoxycarbonyl-C₁-C₆-alkyl C₁-C₆-Alkoxycarbonylamino, N-C₁-C₆-Alkoxycarbonyl-N-C₁-C₆-alkylamino, C₁-C₆-Alkoxycarbonyl (C₁-C₆)alkoxy und Phenyl-C₁-C₆-alkoxy, z.B. Pentoxy, 1-Methylbutoxy, 2-Methylbutoxy, 3-Methoxylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy;
- C₁-C₈-Alkoxy: C₁-C₆-Alkoxy wie voranstehend genannt, sowie die Alkoxyteile von C₁-C₈-Alkoxy-C₁-C₈-alkyl, C₁-C₈-Alkoxy-C₁-C₈-alkoxy, C₁-C₈-Alkoxycarbonyl, C₁-C₈-Alkoxycarbonyl-C₁-C₈-alkyl, C₁-C₈-Alkoxycarbonylamino, N-C₁-C₈-Alkoxycarbonyl-N-C₁-C₈-alkylamino und Phenyl-C₁-C₈-alkoxy, z.B. Heptoxy, 2-Methylhexoxy, 3-Methylhexoxy, 2,2-Dimethylpentoxy, 2,3-Dimethylpentoxy, 2,4-Dimethylpentoxy, 3,3-Dimethylpentoxy, 2,2-Dimethyl-3-methylbutoxy, Octoxy, 2-Methylheptoxy, 3-Methylheptoxy, 4-Methylhepoxy, 2,2-Diemthylhexoxy, 2,3-Dimethylhexoxy, 2,4-Dimethylhexoxy, 3,3-Dimethylhexoxy, 2,2-Dimethyl-3-methylpentoxy, 2-Methyl-3,3-dimethylpentoxy, 2,3,4-Trimethylpentoxy und 2,2,3,3-Tetramethylbutoxy;
- C₁-C₄-Halogenalkoxy: einen C₁-C₄-Alkoxyrest wie voranstehend genannt, der partiell oder vollständig durch Fluor, Chlor, Brom und/oder Iod substituiert ist, also z.B. Fluormethoxy, Difluormethoxy, Trifluormethoxy, Chlordifluormethoxy, Bromdifluormethoxy, 2-Fluorethoxy, 2-Chlorethoxy, 2-Brommethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2-Chlorpropoxy, 3-Chlorpropoxy, 2-Brompropoxy, 3-Brompropoxy, 2,2-difluorpropoxy, 2,3-Difluorpropoxy, 2,3-Dichlorpropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy und Nonafluorbutoxy;
- C₁-C₆-Halogenalkoxy: C₁-C₄-Halogenalkoxy wie voranstehend genannt, sowie z.B. 5-Fluorpentoxy, 5-Chlorpentoxy, 5-Brompentoxy, 5-Iodpentoxy, Undecafluorpentoxy, 6-Fluorhexoxy, 6-Chlorhexoxy, 6-Bromhexoxy, 6-Iodhexoxy und Dodecafluorhexoxy;
- C₁-C₈-Halogenalkoxy: C₁-C₆-Halogenalkoxy wie voranstehend genannt, sowie z.B. 7-Fluorheptoxy, 7-Chlorheptoxy, 7-Bromheptoxy, 7-Iodheptoxy, Perfluorheptoxy, 8-Fluoroctoxy, 8-Chloroctoxy, 8-Bromoctoxy, 8-Iodoctoxy und Octadecafluoroctoxy;
- C₁-C₆-Alkylamino sowie die Alkylamino-Teile von C₁-C₆-Alkylamino-C₁-C₆-alkyl, N-C₁-C₆-Alkylcarbonyl-N-C₁-C₆-alkylamino, C₁-C₆-Alkylaminocarbonyl und C₁-C₆-Alkylaminocarbonyl-C₁-C₆-alkyl, z.B. Methylamino, Ethylamino, Propylamino, 1-Methylethylamino, Butylamino, 1-Methylpropylamino, 2-Methylpropylamino, 1,1-Dimethylethylamino, Pentylamino, 1-Methylbutylamino, 2-Methylbutylamino, 3.Methylbutylamino, 2,2-Dimethylpropylamino, 1-Ethylpropylamino, Hexylamino, 1,1-Dimethylpropylamino, 1,2-Dimethylpropylamino, 1-Methylpentylamino, 2-Methylpentylamino, 3-Methylpentylamino, 4-Methylpentylamino, 1,1-Dimethylbutylamino, 1,2-Dimethylbutylamino, 1,3-Dimethylbutylamino, 2,2-Dimethylbutylamino, 2,3-Dimethylbutylamino, 3,3-Dimethylbutylamino, 1-Ethylbutylamino, 2-Ethylbutylamino, 1,1,2-Trimethylpropylamino, 1,2,2-Trimethylpropylamino, 1-Ethyl-1-methylpropylamino oder 1-Ethyl-2-methylpropylamino;
- C₁-C₈-Alkylamino: C₁-C₆-Alkylamino wie voranstehend genannt, sowie die Alkylamino-Teile von C₁-C₈-Alkylamino-C₁-C₈-alkyl, N-C₁-C₈-Alkylcarbonyl-N-C₁-C₈-alkylamino, N-C₁-C₈-Alkoxycarbonyl-N-C₁-C₈-alkylamino, C₁-C₈-Alkylaminocarbonyl und C₁-C₈-Alkylaminocarbonyl-C₁-C₈-alkyl: z.B. Heptylamino, 2-Methylhexylamino, 3-Methylhexylamino, 2,2-Dimethylpentylamino, 2,3-Dimethylpentylamino, 2,4-Dimethylpentylamino, 3,3-Dimethylpentylamino, 2,2-Dimethyl-3-methylbutylamino, Octylamino, 2-Methylheptylamino, 3-Methylheptylamino, 4-Methylheptylamino, 2,2-Diemthylhexylamino, 2,3-Dimethylhexylamino, 2,4-Dimethylhexylamino, 3,3-Dimethylhexylamino, 2,2-Dimethyl-3-methylpentylamino, 2-Methyl-3,3-dimethylpentylamino, 2,3,4-Trimethylpentylamino und 2,2,3,3-Tetramethylbutylamino;
- Di(C₁-C₄-alkyl)amino sowie die Dialkylamino-Teile von Di(C₁-C₄-alkyl)amino-C₁-C₄-alkyl, Di(C₁-C₄-alkyl)aminocarbonyl und Di(C₁-C₄-alkyl)aminocarbonyl-C₁-C₄-alkyl, z.B. N,N-Dimethylamino, N,N-Diethylamino, N,N-Dipropylamino, N,N-Di(1-methylethyl)amino, N,N-Dibutylamino, N,N-Di(1-methylpropyl)amino, N,N-Di(2-methylpropyl)amino, N,N-Di(1,1-dimethylethyl)amino, N-Ethyl-N-methylamino, N-Methyl-N-propylamino, N-Methyl-N-(1-methylethyl) amino, N-Butyl-N-methylamino, N-Methyl-N-(1-methylpropyl) amino, N-Methyl-N-(2-methylpropyl) amino, N-(1,1-Dimethylethyl)-N-methylamino, N-Ethyl-N-propylamino, N-Ethyl-N-(1-methylethyl)amino, N-Butyl-N-ethylamino, N-Ethyl-N-(1-methylpropyl)amino, N-Ethyl-N-(2-methyl- , propyl) amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, N-(1-Methylethyl)-N-(1-methylpropyl) amino, N-(1-Methylethyl)-N-(2-methylpropyl)amino, N-(1,1-Dimethylethyl)-N-(1-methylethyl)amino, N-Butyl-N-(1-methylpropyl)amino, N-Butyl-N-(2-methylpropyl)amino, N-Butyl-N-(1,1-dimethylethyl)amino, N-(1-Methylpropyl)-N-(2-methylpropyl)amino, N-(1,1-Dimethylethyl)-N-(1-methylpropyl)amino und N-(1,1-Dimethylethyl)-N-(2-methylpropyl)amino;
- Di(C₁-C₆-alkyl)amino: Di(C₁-C₄-alkyl)amino wie voranstehend genannt, sowie die Dialkylamino-Teile von Di(C₁-C₆-alkyl)amino-C₁-C₆-alkyl, Di(C₁-C₆-alkyl)aminocarbonyl und Di (C₁-C₆-alkyl) aminocarbonyl-C₁-C₆-alkyl: z.B. N,N-Dipentylamino, N,N-Dihexylamino, N-Methyl-N-pentylamino, N-Ethyl-N-pentylamino, N-Methyl-N-hexylamino und N-Ethyl-N-hexylamino;
- Di(C₁-C₈-alkyl)amino: Di(C₁-C₆-alkyl)amino wie voranstehend genannt, sowie die Dialkylamino-Teile von Di(C₁-C₈-alkyl)amino-C₁-C₈-alkyl, Di(C₁-C₈-alkyl)aminocarbonyl und Di(C₁-C₈-alkyl)aminocarbonyl-C₁-C₈-alkyl: z.B. N,N-Diheptylamino, N,N-Dioctylamino, N-Methyl-N-heptylamino, N-Ethyl-N-heptylamino, N-Methyl-N-octylamino und N-Ethyl-N-octylamino;
- C₁-C₄-Alkylthio sowie die Alkylthio-Teile von C₁-C₄-Alkylthio-C₁-C₄-alkyl, z.B. Methylthio, Ethylthio, Propylthio, 1-Methylethylthio, Butylthio, 1-Methylpropylthio, 2-Methylpropylthio und 1,1-Dimethylethylthio;
- C₁-C₆-Alkylthio: C₁-C₄-Alkylthio wie voranstehend genannt, sowie die Alkylthio-Teile von C₁-C₆-Alkylthio-C₁-C₆-alkyl, sowie z.B. Pentylthio, 1-Methylbutylthio, 2-Methylbutylthio, 3-Methylbutylthio, 2,2-Dimethylpropylthio, 1-Ethylpropylthio, Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio;
- C₁-C₈-Alkylthio: C₁-C₆-Alkylthio wie voranstehend genannt, sowie die Alkylthio-Teile von C₁-C₈-Alkylthio-C₁-C₈-alkyl, sowie z.B. Heptylthio, 2-Methylhexylthio, 3-Methylhexylthio, 2,2-Dimethylpentylthio, 2,3-Dimethylpentylthio, 2,4-Dimethylpentylthio, 3,3-Dimethylpentylthio, 2,2-Dimethyl-3-methylbutylthio, Octylthio, 2-Methylheptylthio, 3-Methylheptylthio 4-Methylheptylthio, 2,2-Diemthylhexylthio, 2,3-Dimethylhexylthio, 2,4-Dimethylhexylthio, 3,3-Dimethylhexylthio, 2,2,3-Trimethylpentylthio, 2,3,3-Trimethylpentylthio, 2,3,4-Trimethylpentylthio und 2,2,3,3-Tetramethylbutylthio;
- C₁-C₄-Halogenalkylthio: einen C₁-C₄-Alkylthiorest wie voranstehend genannt, der partiell oder vollständig durch Fluor, Chlor, Brom und/oder Iod substituiert ist, also z.B. Fluormethylthio, Difluormethylthio, Trifluormethylthio, Chlordifluormethylthio, Bromdifluormethylthio, 2-Fluorethylthio, 2-Chlorethylthio, 2-Bromethylthio, 2-Iodethylthio, 2,2-Difluorethylthio, 2,2,2-Trifluorethylthio, 2,2,2-Trichlorethylthio, 2-Chlor-2-fluorethylthio, 2-Chlor-2,2-difluorethylthio, 2,2-Dichlor-2-fluorethylthio, Pentafluorethylthio, 2-Fluorpropylthio, 3-Fluorpropylthio, 2-Chlorpropylthio, 3-Chlorpropylthio, 2-Brompropylthio, 3-Brompropylthio, 2,2-Difluorpropylthio, 2,3-Difluorpropylthio, 2,3-Dichlorpropylthio, 3,3,3-Trifluorpropylthio, 3,3,3-Trichlorpropylthio, 2,2,3,3,3-Pentafluorpropylthio, Heptafluorpropylthio, 1-(Fluormethyl)-2-fluorethylthio, 1-(Chlormethyl)-2- chlorethylthio, 1-(Brommethyl)-2-bromethylthio, 4-Fluorbutylthio, 4-Chlorbutylthio, 4-Brombutylthio und Nonafluorbutylthio;
- C₁-C₆-Halogenalkylthio: C₁-C₄-Halogenalkylthio wie voranstehend genannt, sowie z.B. 5-Fluorpentylthio, 5-Chlorpentylthio, 5-Brompentylthio, 5-Iodpentylthio, Undecafluorpentylthio, 6-Fluorhexylthio, 6-Chlorhexylthio, 6-Bromhexylthio, 6-Iodhexylthio und Dodecafluorhexylthio;
- C₁-C₈-Halogenalkylthio: C₁-C₆-Halogenalkylthio wie voranstehend genannt, sowie z.B. 7-Fluorheptylthio, 7-Chlorheptylthio, 7-Bromheptylthio, 7-Iodheptylthio, Perfluorheptylthio, 8-Fluoroctylthio, 8-Chloroctylthio, 8-Bromoctylthio, 8-Iodoctylthio und Octadecafluoroctylthio;
- C₁-C₆-Alkylsulfinyl (C₁-C₆-Alkyl-S(=O)-): z.B. Methylsulfinyl, Ethylsulfinyl, Propylsulfinyl, 1-Methylethylsulfinyl, Butylsulfinyl, 1-Methylpropylsulfinyl, 2-Methylpropylsulfinyl, 1,1-Dimethylethylsulfinyl, Pentylsulfinyl, 1-Methylbutylsulfinyl, 2-Methylbutylsulfinyl, 3-Methylbutylsulfinyl, 2,2-Dimethylpropylsulfinyl, 1-Ethylpropylsulfinyl, 1,1-Dimethylpropylsulfinyl, 1,2-Dimethylpropylsulfinyl, Hexylsulfinyl, 1-Methylpentylsulfinyl, 2-Methylpentylsulfinyl, 3-Methylpentylsulfinyl, 4-Methylpentylsulfinyl, 1,1-Dimethylbutylsulfinyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, 1,1,2-Trimethylpropylsulfinyl, 1,2,2-Trimethylpropylsulfinyl, 1-Ethyl-1-methylpropylsulfinyl und 1-Ethyl-2-methylpropylsulfinyl;
- C₁-C₈-Alkylsulfinyl (C₁-C₈-Alkyl-S(=O)-): C₁-C₆-Alkylsulfinyl wie voranstehend genannt sowie z.B. Heptylsulfinyl, 2-Methylhexylsulfinyl, 3-Methylhexylsulfinyl, 2,2-Dimethylpentylsulfinyl, 2,3-Dimethylpentylsulfinyl, 2,4-Dimethylpentylsulfinyl, 3,3-Dimethylpentylsulfinyl, 2,2-Dimethyl-3-methylbutylsulfinyl, Octylsulfinyl, 2-Methylheptylsulfinyl, 3-Methylheptylsulfinyl 4-Methylheptylsulfinyl, 2,2-Diemthylhexylsulfinyl, 2,3-Dimethylhexylsulfinyl, 2,4-Dimethylhexylsulfinyl, 3,3-Dimethylhexylsulfinyl, 2,2,3-Trimethylpentylsulfinyl, 2,3,3-Trimethylpentylsulfinyl, 2,3,4-Trimethylpentylsulfinyl und 2,2,3,3-Tetramethylbutylsulfinyl;
- C₁-C₆-Halogenalkylsulfinyl: C₁-C₆-Alkylsulfinylrest wie voranstehend genannt, der partiell oder vollständig durch Fluor, Chlor, Brom und/oder Iod substituiert ist, also z.B. Fluormethylsulfinyl, Difluormethylsulfinyl, Trifluormethylsulfinyl, Chlordifluormethylsulfinyl, Bromdifluormethylsulfinyl, 2-Fluorethylsulfinyl, 2-Chlorethylsulfinyl, 2-Bromethylsulfinyl, 2-Iodethylsulfinyl, 2,2-Difluorethylsulfinyl, 2,2,2-Trifluorethylsulfinyl, 2,2,2-Trichlorethylsulfinyl, 2-Chlor-2-fluorethylsulfinyl, 2-Chlor-2,2-difluorethylsulfinyl, 2,2-Dichlor-2-fluorethylsulfinyl, Pentafluorethylsulfinyl, 2-Fluorpropylsulfinyl, 3-Fluorpropylsulfinyl, 2-Chlorpropylsulfinyl, 3-Chlorpropylsulfinyl, 2-Brompropylsulfinyl, 3-Brompropylsulfinyl, 2,2-Difluorpropylsulfinyl, 2,3-Difluorpropylsulfinyl, 2,3-Dichlorpropylsulfinyl, 3,3,3-Trifluorpropylsulfinyl, 3,3,3-Trichlorpropylsulfinyl, 2,2,3,3,3-Pentafluorpropylsulfinyl, Heptafluorpropylsulfinyl, 1-(Fluormethyl)-2-fluorethylsulfinyl, 1-(Chlormethyl)-2-chlorethylsulfinyl, 1-(Brommethyl)-2-bromethylsulfinyl, 4-Fluorbutylsulfinyl, 4-Chlorbutylsulfinyl, 4-Brombutylsulfinyl, Nonafluorbutylsulfinyl, 5-Fluorpentylsulfinyl, 5-Chlorpentylsulfinyl, 5-Brompentylsulfinyl, 5-Iodpentylsulfinyl, Undecafluorpentylsulfinyl, 6-Fluorhexylsulfinyl, 6-Chlorhexylsulfinyl, 6-Bromhexylsulfinyl, 6-Iodhexylsulfinyl und Dodecafluorhexylsulfinyl;
- C₁-C₈-Halogenalkylsulfinyl: C₁-C₆-Halogenalkylsulfinyl wie voranstehend genannt, sowie z.B. 7-Fluorheptylsulfinyl, 7-Chlorheptylsulfinyl, 7-Bromheptylsulfinyl, 7-Iodheptylsulfinyl, Perfluorheptylsulfinyl, 8-Fluoroctylsulfinyl, 8-Chloroctylsulfinyl, 8-Bromoctylsulfinyl, 8-Iodoctylsulfinyl und Perfluoroctylsulfinyl;
- C₁-C₆-Alkylsulfonyl (C₁-C₆-Alkyl-S(=O)₂-) sowie die Alkylsulfonyl-Teile von C₁-C₆-Alkylsulfonyl-C₁-C₆-alkyl: z.B. Methylsulfonyl, Ethylsulfonyl, Propylsulfonyl, 1-Methylethylsulfonyl, Butylsulfonyl, 1-Methylpropylsulfonyl, 2-Methylpropylsulfonyl, 1,1-Dimethylethylsulfonyl, Pentylsulfonyl, 1-Methylbutylsulfonyl, 2-Methylbutylsulfonyl, 3-Methylbutylsulfonyl, 1,1-Dimethylpropylsulfonyl, 1,2-Dimethylpropylsulfonyl, 2,2-Dimethylpropylsulfonyl, 1-Ethylpropylsulfonyl, Hexylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl und 1-Ethyl-2-methylpropylsulfonyl;
- C₁-C₈-Alkylsulfonyl (C₁-C₈-Alkyl-S(=O)₂-): C₁-C₆-Alkylsulfonyl wie voranstehend genannt, sowie die Alkylsulfonyl-Teile von C₁-C₈-Alkylsulfonyl-C₁-C₈-alkyl: z.B. Heptylsulfonyl, 2-Methylhexylsulfonyl, 3-Methylhexylsulfonyl, 2,2-Dimethylpentylsulfonyl, 2,3-Dimethylpentylsulfonyl, 2,4-Dimethylpentylsulfonyl, 3,3-Dimethylpentylsulfonyl, 2,2-Dimethyl-3-methylbutylsulfonyl, Octylsulfonyl, 2-Methylheptylsulfonyl, 3-Methylheptylsulfonyl 4-Methylheptylsulfonyl, 2,2-Diemthylhexylsulfonyl, 2,3-Dimethylhexylsulfonyl, 2,4-Dimethylhexylsulfonyl, 3,3-Dimethylhexylsulfonyl, 2,2,3-Trimethylpentylsulfonyl, 2,3,3-Trimethylpentylsulfonyl, 2,3,4-Trimethylpentylsulfonyl und 2,2,3,3-Tetramethylbutylsulfonyl;
- C₁-C₆-Halogenalkylsulfonyl: einen C₁-C₆-Alkylsulfonylrest wie voranstehend genannt, der partiell oder vollständig durch Fluor, Chlor, Brom und/oder Iod substituiert ist, also z.B. Fluormethylsulfonyl, Difluormethylsulfonyl, Trifluormethylsulfonyl, Chlordifluormethylsulfonyl, Bromdifluormethylsulfonyl, 2-Fluorethylsulfonyl, 2-Chlorethylsulfonyl, 2-Bromethylsulfonyl, 2-Iodethylsulfonyl, 2,2-Difluorethylsulfonyl, 2,2,2-Trifluorethylsulfonyl, 2-Chlor-2-fluorethylsulfonyl, 2-Chlor-2,2-difluorethylsulfonyl, 2,2-Dichlor-2-fluorethylsulfonyl, 2,2,2-Trichlorethylsulfonyl, Pentafluorethylsulfonyl, 2-Fluorpropylsulfonyl, 3-Fluorpropylsulfonyl, 2-Chlorpropylsulfonyl, 3-Chlorpropylsulfonyl, 2-Brompropylsulfonyl, 3-Brompropylsulfonyl, 2,2-Difluorpropylsulfonyl, 2,3-Difluorpropylsulfonyl, 2,3-Dichlorpropylsulfonyl, 3,3,3-Trifluorpropylsulfonyl, 3,3,3-Trichlorpropylsulfonyl, 2,2,3,3,3-Pentafluorpropylsulfonyl, Heptafluorpropylsulfonyl, 1-(Fluormethyl)-2-fluorethylsulfonyl, 1-(Chlormethyl)-2-chlorethylsulfonyl, 1-(Brommethyl)-2-bromethylsulfonyl, 4-Fluorbutylsulfonyl, 4-Chlorbutylsulfonyl, 4-Brombutylsulfonyl, Nonafluorbutylsulfonyl, 5-Fluorpentylsulfonyl, 5-Chlorpentylsulfonyl, 5-Brompentylsulfonyl, 5-Iodpentylsulfonyl, 6-Fluorhexylsulfonyl, 6-Bromhexylsulfonyl, 6-Iodhexylsulfonyl und Dodecafluorhexylsulfonyl;
- C₁-C₈-Halogenalkylsulfonyl: C₁-C₆-Halogenalkylsulfonyl wie voranstehend genannt, sowie z.B. 7-Fluorheptylsulfonyl, 7-Chlorheptylsulfonyl, 7-Bromheptylsulfonyl, 7-Iodheptylsulfonyl, Perfluorheptylsulfonyl, 8-Fluoroctylsulfonyl, 8-Chloroctylsulfonyl, 8-Bromoctylsulfonyl, 8-Iodoctylsulfonyl und Octadecafluoroctylsulfonyl;
- Aryl: ein- bis dreikerniger aromatischer Carbocyclus mit 6 bis 14 Ringgliedern, wie z.B. Phenyl, Naphthyl, Anthracenyl und Phenanthrenyl;
- 5-gliedriges Heteroaryl mit ein bis vier Stickstoffatomen, oder mit ein bis drei Stickstoffatomen und einem Sauerstoff- oder Schwefelatom, oder mit einem Sauerstoff- oder Schwefelatom, steht für C-Atom verknüpfte aromatische 5-Ring Heterocyclen, welche neben Kohlenstoffatomen ein bis vier Stickstoffatome, oder ein bis drei Stickstoffatome und ein Schwefel- oder Sauerstoffatom, oder ein Sauerstoff- oder ein Schwefelatom als Ringglieder enthalten können,
   z.B. über ein C-Atom verknüpfte aromatische 5-Ring Heterocyclen mit einem Heteroatom, wie z.B. 2-Furyl, 3-Furyl, 2-Thienyl, 3-Thienyl, 2-Pyrrolyl, 3-Pyrrolyl;
   z.B. über ein C-Atom verknüpfte aromatische 5-Ring Heterocyclen mit zwei Heteroatomen, wie z.B. 3-Isoxazolyl, 4-Isoxazolyl, 5-Isoxazolyl, 3-Isothiazolyl, 4-Isothiazolyl, 5-Isothiazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 3-Pyrazolyl, 4-Pyrozolyl, 5-Pyrazolyl;
   z.B. über ein C-Atom verknüpfte aromatische 5-Ring Heterocyclen mit drei Heteroatomen, wie z.B. 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, Tetrazol-2-yl;
   z.B. über ein C-Atom verknüpfte aromatische 5-Ring Heterocyclen mit vier Heteroatomen wie z.B. Tetrazol-2-yl;
- 6-gliedriges Heteroaryl mit einem bis vier N-Atomen:
   z.B. über ein C-Atom verknüpfte aromatische 6-Ring Heterocyclen, welche neben Kohlenstoffatomen ein bis vier, vorzugsweise ein bis drei Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl, 1,2,4-Triazin-5-yl, 1,2,4-Triazin-6-yl und 1,2,4,5-Tetrazinyl;

In einer besonderen Ausführungsform haben die Variablen der Verbindungen der Formel I folgende Bedeutungen, wobei diese für sich allein betrachtet als auch in Kombination miteinander besondere Ausgestaltungen der Verbindungen der Formel I darstellen:

Bevorzugt sind die 3-heteroarylsubstituierten Isoxazoline der Formel I, in der
- X: 6-gliedriges Heteroaryl mit ein bis vier Stickstoffatomen, das partiell oder vollständig halogeniert ist und/oder ein bis drei Substituenten aus folgender Gruppe trägt:
Cyano, Nitro, Hydroxy, C₁-C₈-Alkyl, C₃-C₆-Cycloalkyl, C₃-C₆-Halogencycloalkyl, C₂-C₈-Alkenyl, C₂-C₈-Alkinyl, C₁-C₈-Halogenalkyl, C₂-C₆-Halogenalkenyl, C₂-C₆-Halogenalkinyl, C₁-C₈-Alkoxy, C₁-C₈-Halogenalkoxy, C₁-C₈-Alkoxy-C₁-C₈-alkyl, Amino, C₁-C₈-Alkylamino, Di(C₁-C₈-alkyl)amino, Amino-C₁-C₈-alkyl, C₁-C₈-Alkylamino-C₁-C₈-alkyl, Di(C₁-C₈-alkyl)amino-C₁-C₈-alkyl, C₁-C₈-Alkylthio, C₁-C₈-Halogenalkylthio, C₁-C₈-Alkylthio-C₁-C₈-alkyl, C₁-C₈-Alkylsulfinyl, C₁-C₈-Halogenalkylsulfinyl, C₁-C₈-Alkylsulfonyl, C₁-C₈-Halogenalkylsulfonyl, C₁-C₈-Alkylsulfonyl-C₁-C₈-alkyl, C₁-C₈-Alkoxycarbonyl, C₁-C₈-Alkoxycarbonyl-C₁-C₈-alkyl, Aminocarbonyl, C₁-C₈-Alkylaminocarbonyl, Di(C₁-C₈-alkyl)aminocarbonyl, Aminocarbonyl-C₁-C₈-alkyl, C₁-C₈-Alkylaminocarbonyl-C₁-C₈-alkyl, Di(C₁-C₈-alkyl)aminocarbonyl-C₁-C₈-alkyl, Carboxyl, Carboxy-C₁-C₈-alkyl, Phenyl, Phenoxy, Phenylthio, Phenylamino, Phenyl-C₁-C₈-alkyl, Phenyl-C₁-C₈-alkoxy, 3- bis 6-gliedriges Heterocyclyl und 5-oder 6-gliedriges Heteroaryl, wobei die acht letztgenannten Reste ihrerseits partiell oder vollständig halogeniert sein können und/oder ein bis drei Substituenten aus der Gruppe Nitro, Cyano, C₁-C₈-Alkyl, C₁-C₈-Alkoxy, C₁-C₈-Halogenalkyl, C₁-C₈-Halogenalkoxy, C₁-C₈-Alkylthio, C₁-C₈-Halogenalkylthio, C₁-C₈-Alkylsulfinyl, C₁-C₈-Halogenalkylsulfinyl, C₁-C₈-Alkylsulfonyl und C₁-C₈-Halogenalkylsulfonyl tragen können;
besonders bevorzugt 6-gliedriges Heteroaryl mit ein bis vier Stickstoffatomen, das partiell oder vollständig halogeniert ist und/oder ein bis drei Substituenten aus folgender Gruppe trägt:
Cyano, Nitro, Hydroxy, C₁-C₆-Alkyl, C₃-C₆-Cycloalkyl, C₃-C₆-Halogencycloalkyl, C₂-C₆-Alkenyl, C₂-C₆-Alkinyl, C₁-C₆-Halogenalkyl, C₂-C₆-Halogenalkenyl, C₂-C₆-Halogenalkinyl, C₁-C₆-Alkoxy, C₁-C₆-Halogenalkoxy, C₁-C₆-Alkoxy-C₁-C₆-alkyl, Amino, C₁-C₆-Alkylamino, Di(C₁-C₆-alkyl)amino, Amino-C₁-C₆-alkyl, C₁-C₆-Alkylamino-C₁-C₆-alkyl, Di (C₁-C₆-alkyl) amino-C₁-C₆-alkyl, C₁-C₆-Alkylthio, C₁-C₆-Halogenalkylthio, C₁-C₆-Alkylthio-C₁-C₆-alkyl, C₁-C₆-Alkylsulfinyl, C₁-C₆-Alkylsulfonyl, C₁-C₆-Alkylsulfonyl-C₁-C₆-alkyl, C₁-C₆-Alkoxycarbonyl, C₁-C₆-Alkoxycarbonyl-C₁-C₆-alkyl, Aminocarbonyl, C₁-C₆-Alkylaminocarbonyl, Di(C₁-C₆-alkyl)aminocarbonyl, Aminocarbonyl-C₁-C₆-alkyl, C₁-C₆-Alkylaminocarbonyl-C₁-C₆-alkyl, Di(C₁-C₆-alkyl)aminocarbonyl-C₁-C₆-alkyl, Carboxyl und Carboxy-C₁-C₆-alkyl;
insbesondere bevorzugt 6-gliedriges Heteroaryl mit ein bis vier Stickstoffatomen, das partiell oder vollständig halogeniert ist und/oder ein bis drei Substituenten aus folgender Gruppe trägt:
Cyano, Nitro, Hydroxy, C₁-C₆-Alkyl, C₃-C₆-Cycloalkyl, C₃-C₆-Halogencycloalkyl, C₁-C₆-Halogenalkyl, C₁-C₆-Alkoxy, C₁-C₆-Halogenalkoxy, C₁-C₆-Alkoxy-C₁-C₆-alkyl, Amino, C₁-C₆-Alkylamino, Di(C₁-C₆-alkyl)amino, C₁-C₆-Alkylthio, C₁-C₆-Halogenalkylthio, C₁-C₆-Alkylsulfinyl, C₁-C₆-Alkylsulfonyl, C₁-C₆-Alkoxycarbonyl, Aminocarbonyl, C₁-C₆-Alkylaminocarbonyl, Di(C₁-C₆-alkyl)aminocarbonyl, Carboxyl und Carboxy-C₁-C₆-alkyl;
außerordentlich bevorzugt 6-gliedriges Heteroaryl mit ein bis vier Stickstoffatomen, das partiell halogeniert ist und/oder ein bis zwei Substituenten aus folgender Gruppe trägt:
Cyano, Nitro, Hydroxy, C₁-C₆-Alkyl, C₃-C₆-Cycloalkyl, C₃-C₆-Halogencycloalkyl, C₁-C₆-Halogenalkyl, C₁-C₆-Alkoxy, C₁-C₆-Halogenalkoxy, C₁-C₆-Alkoxy-C₁-C₆-alkyl, Amino, C₁-C₆-Alkylamino, Di(C₁-C₆-alkyl)amino, C₁-C₆-Alkylthio, C₁-C₆-Halogenalkylthio, C₁-C₆-Alkylsulfinyl, C₁-C₆-Alkylsulfonyl, C₁-C₆-Alkoxycarbonyl, Aminocarbonyl, C₁-C₆-Alkylaminocarbonyl, Di(C₁-C₆-alkyl)aminocarbonyl, Carboxyl und Carboxy-C₁-C₆-alkyl;
sehr außerordentlich bevorzugt 6-gliedriges Heteroaryl mit ein bis vier Stickstoffatomen, das partiell halogeniert ist und/oder einen Substituenten aus folgender Gruppe trägt:
Cyano, Nitro, Hydroxy, C₁-C₆-Alkyl, C₃-C₆-Cycloalkyl, C₃-C₆-Halogencycloalkyl, C₁-C₆-Halogenalkyl, C₁-C₆-Alkoxy, C₁-C₆-Halogenalkoxy, C₁-C₆-Alkoxy-C₁-C₆-alkyl, Amino, C₁-C₆-Alkylamino, Di(C₁-C₆-alkyl)amino, C₁-C₆-Alkylthio, C₁-C₆-Halogenalkylthio, C₁-C₆-Alkylsulfinyl, C₁-C₆-Alkylsulfonyl, C₁-C₆-Alkoxycarbonyl, Aminocarbonyl, C₁-C₆-Alkylaminocarbonyl, Di(C₁-C₆-alkyl)aminocarbonyl, Carboxyl und Carboxy-C₁-C₆-alkyl;
bedeutet.

Ebenso bevorzugt sind die 3-heteroarylsubstituierten Isoxazoline der Formel I, in der
- X: 6-gliedriges Heteroaryl mit ein bis drei Stickstoffatomen, bevorzugt 6-gliedriges Heteroaryl mit ein bis zwei N-Atomen, insbesondere Pyridyl, Pyrimidyl oder Pyrazinyl, sehr bevorzugt Pyridyl oder Pyrimidyl;
ebenso sehr bevorzugt Pyridyl oder Pyrazinyl,
sehr bevorzugt Pyridyl; und
das 6-gliedrige Heteroaryl wie oben beschrieben substituiert ist;
außerordentlich bevorzugt 6-gliedriges Heteroaryl mit ein bis zwei Stickstoffatomen,
insbesondere Pyridyl oder Pyrazinyl,
sehr bevorzugt Pyridyl; und
das 6-gliedrige Heteroaryl wie oben beschrieben substituiert ist; und
das 6-gliedrige Heteroaryl ortho- oder meta zu einem Stickstoff mit dem Isoxazolingerüst verknüpft ist,
bedeutet.

Ebenso bevorzugt sind die 3-heteroarylsubstituierten Isoxazoline der Formel I, in der
- X: 6-gliedriges Heteroaryl mit ein bis zwei Stickstoffatomen, welches partiell oder vollständig halogeniert ist und/oder ein bis zwei Substituenten aus folgender Gruppe trägt:
Cyano, Nitro, Hydroxy, C₁-C₆-Alkyl, C₁-C₆-Halogenalkyl, C₂-C₆-Alkenyl, C₂-C₆-Alkinyl, C₁-C₆-Alkoxy, C₁-C₆-Halogenalkoxy, Amino, C₁-C₆-Alkylamino, Di(C₁-C₆-alkyl)amino, C₁-C₆-Alkylthio, C₁-C₆-Alkylsulfinyl und C₁-C₆-Alkylsulfonyl; bevorzugt Cyano, Nitro, Hydroxy, C₁-C₆-Alkyl, C₁-C₆-Halogenalkyl, C₁-C₆-Alkoxy, C₁-C₆-Halogenalkoxy, Amino, C₁-C₆-Alkylamino, Di(C₁-C₆-alkyl)amino, C₁-C₆-Alkylthio, C₁-C₆-Alkylsulfinyl und C₁-C₆-Alkylsulfonyl; und
in ortho- oder meta-Position zu einem Stickstoff des 6-gliedrigen Heteroaryls mit dem Isoxazolingerüst verknüpft ist;
bevorzugt 6-gliedriges Heteroaryl mit ein bis zwei Stickstoffatomen, welches partiell halogeniert ist und/oder ein bis zwei Substituenten in ortho-Position zu einem Stickstoffatom aus folgender Gruppe trägt:
Cyano, Nitro, Hydroxy, C₁-C₆-Alkyl, C₁-C₆-Halogenalkyl, C₂-C₆-Alkenyl, C₂-C₆-Alkinyl, C₁-C₆-Alkoxy, C₁-C₆-Halogenalkoxy, Amino, C₁-C₆-Alkylamiho, Di(C₁-C₆-alkyl)amino, C₁-C₆-Alkylthio und C₁-C₆-Alkylsulfinyl;
bevorzugt Cyano, Nitro, Hydroxy, C₁-C₆-Alkyl, C₁-C₆-Halogenalkyl, C₁-C₆-Alkoxy, C₁-C₆-Halogenalkoxy, Amino, C₁-C₆-Alkylamino, Di(C₁-C₆-alkyl)amino, C₁-C₆-Alkylthio und C₁-C₆-Alkylsulfinyl; und
in ortho- oder meta-Position zu einem Stickstoff des 6-gliedrigen Heteroaryls mit dem Isoxazolingerüst verknüpft ist;
insbesondere bevorzugt 6-gliedriges Heteroaryl mit ein bis zwei Stickstoffatomen, welches partiell halogeniert ist und/oder einen Substituenten in ortho-Position zu einem Stickstoffatom aus folgender Gruppe trägt:
Cyano, Nitro, C₁-C₆-Alkyl, C₁-C₆-Halogenalkyl, C₂-C₆-Alkenyl, C₂-C₆-Alkinyl, C₁-C₆-Alkoxy oder C₁-C₆-Halogenalkoxy und C₁-C₆-Alkylthio;
bevorzugt C₁-C₆-Alkyl, C₁-C₆-Halogenalkyl, C₁-C₆-Alkoxy und C₁-C₆-Halogenalkoxy; und
in ortho- oder meta-Position zu einem Stickstoff des 6-gliedrigen Heteroaryls mit dem Isoxazolingerüst verknüpft ist;
bedeutet.

Ebenso bevorzugt sind die 3-heteroarylsubstituierten Isoxazoline der Formel I, in der
- X: 6-gliedriges Heteroaryl mit ein bis zwei Stickstoffatomen, welches partiell oder vollständig halogeniert ist und/oder ein bis zwei Substituenten aus folgender Gruppe trägt:
Cyano, Nitro, C₁-C₆-Alkyl, C₁-C₆-Halogenalkyl, C₂-C₆-Alkenyl und C₂-C₆-Alkinyl; und
in ortho- oder meta-Position zu einem Stickstoff des 6-gliedrigen Heteroaryls mit dem Isoxazolingerüst verknüpft ist;
bevorzugt 6-gliedriges Heteroaryl mit ein bis zwei Stickstoffatomen, welches partiell halogeniert ist und/oder ein bis zwei Substituenten in ortho-Position zu einem Stickstoffatom aus folgender Gruppe trägt:
Cyano, Nitro, C₁-C₄-Alkyl, C₁-C₄-Halogenalkyl, C₂-C₄-Alkenyl und C₂-C₄-Alkinyl; und
in ortho- oder meta-Position zu einem Stickstoff des 6-gliedrigen Heteroaryls mit dem Isoxazolingerüst verknüpft ist;
insbesondere bevorzugt 6-gliedriges Heteroaryl mit ein bis zwei Stickstoffatomen, einen Substituenten in ortho-Position zu einem Stickstoffatom aus folgender Gruppe trägt: CN, NO₂, Halogen wie z.B. Fluor, Chlor, Brom, C₁-C₄-Alkyl wie z.B. Methyl, Ethyl, iso-Propyl, C₁-C₄-Halogenalkyl wie z.B. Trifluormethyl, C₂-C₄-Alkenyl wie z.B. Ethenyl, und C₂-C₄-Alkinyl wie z.B. Ethinyl; und
in ortho- oder meta-Position zu einem Stickstoff des 6-gliedrigen Heteroaryls mit dem Isoxazolingerüst verknüpft ist;
außerordentlich bevorzugt 6-gliedriges Heteroaryl mit ein bis zwei Stickstoffatomen, welches einen Substituenten in ortho-Position zu einem Stickstoff aus folgender Gruppe trägt: F, Cl, Br, CN, CH₃, C₂H₅, iC₃H₇, CF₃, Ethenyl und Ethinyl; und in ortho- oder meta-Position zu einem Stickstoff des 6-gliedrigen Heteroaryls mit dem Isoxazolingerüst verknüpft ist;
bedeutet.

Ebenso bevorzugt sind die 3-heteroarylsubstituierten Isoxazoline der Formel I, in der
- R¹ - R⁷: Wasserstoff, C₁-C₄-Alkyl, wie z.B. Methyl oder Ethyl, oder C₁-C₄-Halogenalkyl, wie z.B. Difluormethyl, Trifluormethyl oder Trichlormethyl;
besonders bevorzugt Wasserstoff, Methyl oder Trifluormethyl;
sehr bevorzugt Wasserstoff;
insbesondere bevorzugt stehen R¹, R², R⁴-R⁷ für Wasserstoff, Methyl oder Trifluormehtyl und R³ für Wasserstoff, Methyl, Ethyl oder Trifluormethyl;
insbesondere sehr bevorzugt stehen R¹, R², R⁴-R⁷ für Wasserstoff und R³ für Methyl.

Ebenso bevorzugt sind die 3-heteroarylsubstituierten Isoxazoline der Formel I, in der
- Y: Aryl, das partiell oder vollständig halogeniert sein kann und/oder ein bis drei Substituenten aus der Gruppe Cyano, Nitro, C₁-C₆-Alkyl, C₂-C₆-Alkenyl, C₂-C₆-Alkinyl, C₁-C₆-Halogenalkyl, C₁-C₆-Alkoxy, C₁-C₆-Halogenalkoxy, C₁-C₆-Alkoxycarbonyl-C₁-C₆-alkoxy, C₁-C₆-Alkylsulfonyl, Phenyl, Phenoxy und Phenylcarbonyl tragen kann; oder Benzo[1,4]dioxanyl, Benzo[1,3]dioxolanyl, 2,3-Dihydrobenzofuranyl oder Benzinidazolyl; oder
5- bis 6-gliedriges Heteroaryl mit einem bis vier Stickstoff-, Sauerstoff- und/oder Schwefelatomen, das partiell oder vollständig halogeniert sein kann und/oder ein bis drei Substituenten aus der Gruppe C₁-C₆-Alkyl, C₁-C₆-Halogenalkyl, C₁-C₆-Alkoxy, C₁-C₆-Alkoxy-C₁-C₄-alkyl, C₁-C₆-Alkoxycarbonyl tragen kann;
besonders bevorzugt Aryl, das partiell halogeniert sein kann und/oder ein bis zwei Substituenten aus der Gruppe Cyano, C₁-C₆-Alkyl, C₁-C₆-Halogenalkyl und C₁-C₆-Alkylsulfonyl tragen kann; oder
Benzo[1,4]dioxanyl, Benzo[1,3]dioxolanyl, 2,3-Dihydrobenzofuranyl oder Benzinidazolyl; oder
5- bis 6-gliedriges Heteroaryl mit einem bis vier Stickstoff-, Sauerstoff- und/oder Schwefelatomen, das partiell halogeniert sein kann und/oder ein bis zwei Substituenten aus der Gruppe C₁-C₆-Alkyl, C₁-C₆-Alkoxy-C₁-C₄-alkyl und C₁-C₆-Alkoxycarbonyl tragen kann;
insbesondere bevorzugt Aryl, das partiell halogeniert sein kann und/oder ein bis zwei Substituenten aus der Gruppe Cyano, C₁-C₄-Alkyl, C₁-C₄-Halogenalkyl und C₁-C₄-Alkylsulfonyl tragen kann; oder
Benzo[1,4]dioxanyl, Benzo[1,3]dioxolanyl, 2,3-Dihydrobenzofuranyl oder Benzimidazolyl; oder
5- bis 6-gliedriges Heteroaryl aus der Gruppe Pyrazolyl, Thiazolyl, Isoxozolyl, Thiadiazolyl und Pyridyl, das partiell halogeniert sein kann und/oder ein bis zwei Substituenten aus der Gruppe C₁-C₆-Alkyl, C₁-C₆-Alkoxy-C₁-C₄-alkyl und C₁-C₆-Alkoxycarbonyl tragen kann;
bedeutet.

Ebenso bevorzugt sind die 3-heteroarylsubstituierten Isoxazoline der Formel I, in der
- Y: Aryl, das partiell oder vollständig halogeniert sein kann und/oder ein bis drei Substituenten aus der Gruppe Cyano, Nitro, C₁-C₆-Alkyl oder C₂-C₆-Alkenyl, C₂-C₆-Alkinyl, C₁-C₆-Halogenalkyl, C₁-C₆-Alkoxy, C₁-C₆-Halogenalkoxy, C₁-C₆-Alkoxycarbonyl-C₁-C₄-alkoxy, C₁-C₆-Alkylsulfonyl, Phenyl, Phenoxy und Phenylcarbonyl; bevorzugt Cyano, Nitro, C₁-C₆-Alkyl, C₂-C₆-Alkenyl, C₂-C₆-Alkinyl, C₁-C₆-Halogenalkyl oder C₁-C₆-Alkoxy tragen kann;
besonders bevorzugt Aryl, das partiell halogeniert sein kann und/oder ein bis zwei Substituenten aus der Gruppe Cyano, C₁-C₆-Alkyl, C₁-C₆-Halogenalkyl und C₁-C₆-Alkylsulfonyl;
bevorzugt Cyano, C₁-C₆-Alkyl oder C₁-C₆-Halogenalkyl tragen kann;
insbesondere bevorzugt Aryl, das partiell halogeniert sein kann und/oder ein bis zwei Substituenten aus der Gruppe Cyano, C₁-C₄-Alkyl, C₁-C₄-Halogenalkyl und C₁-C₄-Alkylsulfonyl;
bevorzugt Cyano tragen kann;
bedeutet.

Ebenso bevorzugt sind die 3-heteroarylsubstituierten Isoxazoline der Formel I, in der
- Y: Aryl, das partiell oder vollständig halogeniert sein kann und/oder ein bis zwei Substituenten aus der Gruppe Cyano, C₁-C₆-Alkyl, C₁-C₆-Halogenalkyl, C₁-C₆-Alkoxy und C₁-C₆-Alkylsulfonyl;
bevorzugt Cyano, C₁-C₄-Alkyl und C₁-C₄-Halogenalkyl tragen kann;
besonders bevorzugt Aryl, das in einer oder beiden ortho-Positionen zur Verknüpfungsstelle halogeniert ist und/oder einen Substituenten aus der Gruppe Cyano, C₁-C₄-Alkyl, C₁-C₄-Halogenalkyl, C₁-C₄-Alkoxy und C₁-C₄-Alkoxysulfonyl;
bevorzugt Cyano, C₁-C₄-Alkyl und C₁-C₄-Halogenalkyl tragen kann;
insbesondere besonders bevorzugt Aryl, das in einer oder beiden ortho-Positionen zur Verknüpfungsstelle halogeniert ist;
bedeutet.

Ebenso bevorzugt sind die 3-heteroarylsubstituierten Isoxazoline der Formel I, in der
- Y: Phenyl, das partiell oder vollständig halogeniert sein kann und/oder ein bis drei Substituenten aus der Gruppe Cyano, Nitro, C₁-C₆-Alkyl, C₂-C₆-Alkenyl, C₂-C₆-Alkinyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkoxycarbonyl, C₁-C₆-Alkoxycarbonyl-C₁-C₄-alkoxy, C₁-C₆-Alkylsulfonyl, Phenyl Phenoxy und Phenylcarbonyl;
bevorzugt Cyano, Nitro, C₁-C₆-Alkyl, C₂-C₆-Alkenyl, C₂-C₆-Alkinyl, C₁-C₆-Halogenalkyl, C₁-C₆-Alkoxy, C₁-C₆-Halogenalkoxy, C₁-C₆-Alkylthio und C₁-C₆-Halogenalkylthio tragen kann;
besonders bevorzugt Phenyl, das partiell oder vollständig halogeniert sein kann und/oder ein bis drei Substituenten aus der Gruppe Cyano, Nitro, C₁-C₆-Alkyl, C₂-C₆-Alkenyl, C₂-C₆-Alkinyl, C₁-C₆-Halogenalkyl, C₁-C₆-Alkoxy, C₁-C₆-Alkoxycarbonyl;
bevorzugt Cyano, Nitro, C₁-C₆-Alkyl, C₂-C₆-Alkenyl, C₂-C₆-Alkinyl, C₁-C₆-Halogenalkyl und C₁-C₆-Alkoxy tragen kann;
insbesondere besonders bevorzugt Phenyl, das partiell halogeniert sein kann und/oder ein bis zwei Substituenten aus der Gruppe Cyano, C₁-C₆-Alkyl, C₁-C₆-Halogenalkyl und C₁-C₆-Alkylsulfonyl;
bevorzugt Cyano, C₁-C₆-Alkyl und C₁-C₆-Halogenalkyl tragen kann;
bedeutet.

Ebenso bevorzugt sind die 3-heteroarylsubstituierten Isoxazoline der Formel I, in der
- Y: Phenyl, das partiell oder vollständig halogeniert sein kann und/oder ein bis zwei Substituenten aus der Gruppe Cyano, C₁-C₄-Alkyl und C₁-C₄-Halogenalkyl tragen kann;
besonders bevorzugt Phenyl, das in 2- und/oder 6-Position zur Verknüpfungsstelle halogeniert ist und/oder einen Substituenten aus der Gruppe Cyano, C₁-C₄-Alkyl und C₁-C₄-Halogenalkyl tragen kann;
insbesondere besonders bevorzugt Phenyl, das in 2- und/oder 6-Position zur Verknüpfungsstelle halogeniert ist;
bedeutet.

Ebenso bevorzugt sind die 3-heteroarylsubstituierten Isoxazoline der Formel I, in der
- Y: Naphtyl, das partiell oder vollständig halogeniert sein kann und/oder ein bis drei Substituenten aus der Gruppe Cyano, Nitro, C₁-C₆-Alkyl, C₂-C₆-Alkenyl, C₂-C₆-Alkinyl, C₁-C₆-Halogenalkyl, C₁-C₆-Alkoxy, C₁-C₆-Halogenalkoxy, C₁-C₆-Alkylthio und C₁-C₆-Halogenalkylthio tragen kann;
besonders bevorzugt Naphthyl, das partiell oder vollständig halogeniert sein kann und/oder ein bis drei Substituenten aus der Gruppe Cyano, Nitro, C₁-C₆-Alkyl, C₂-C₆-Alkenyl, C₂-C₆-Alkinyl, C₁-C₆-Halogenalkyl und C₁-C₆-Alkoxy tragen kann;
insbesondere besonders bevorzugt Naphthyl, das partiell halogeniert sein kann und/oder ein bis zwei Substituenten aus der Gruppe Cyano, C₁-C₆-Alkyl und C₁-C₆-Halogenalkyl tragen kann;
bedeutet.

Ebenso bevorzugt sind die-3-heteroarylsubstituierten Isoxazoline der Formel I, in der
- Y: Naphthyl, das partiell oder vollständig halogeniert sein kann und/oder ein bis zwei Substituenten aus der Gruppe Cyano, C₁-C₄-Alkyl und C₁-C₄-Halogenalkyl tragen kann;
besonders bevorzugt Naphthyl, das in einer oder beiden ortho-Positionen zur Verknüpfungsstelle halogeniert ist und/oder einen Substituenten aus der Gruppe Cyano, C₁-C₄-Alkyl und C₁-C₄-Halogenalkyl tragen kann;
insbesondere besonders bevorzugt Naphthyl, das in einer oder beiden ortho-Positionen zur Verknüpfungsstelle halogeniert ist;
bedeutet.

Ebenso bevorzugt sind die 3-heteroarylsubstituierten Isoxazoline der Formel I, in der
- Y: 5-gliedriges Heteroaryl mit ein bis vier Stickstoffatomen, oder mit ein bis 3 Stickstoffatomen und einen Sauerstoff- oder Schwefelatom, oder einem Sauerstoff oder Schwefelatom; oder
6-gliedriges Heteroaryl mit ein bis 4 Stickstoffatomen;
wobei die Heterocyclen partiell oder vollständig halogeniert sein können und/oder ein bis drei Substituenten aus der Gruppe Cyano, C₁-C₆-Alkyl, C₁-C₆-Halogenalkyl, C₁-C₆-Alkoxy, C₁-C₆-Alkoxy-C₁-C₄-alkyl, C₁-C₆-Alkoxycarbonyl und C₁-C₆-Alkylsulfonyl tragen können;
besonders bevorzugt 5-gliedriges Heteroaryl mit ein bis vier Stickstoffatomen, oder mit ein bis 3 Stickstoffatomen und einen Sauerstoff- oder Schwefelatom, oder einem Sauerstoff- oder Schwefelatom; oder
6-gliedriges Heteroaryl mit ein bis 4 Stickstoffatomen; wobei die Heterocyclen partiell oder vollständig halogeniert sein können und/oder ein bis drei Substituenten aus der Gruppe Cyano, C₁-C₆-Alkyl, C₁-C₆-Halogenalkyl,
C₁-C₆-Alkoxy- C₁-C₄-alkyl und C₁-C₆-Alkoxycarbonyl tragen können;
insbesondere besonders bevorzugt 5- oder 6-gliedriges Heteroaryl aus der Gruppe Pyrozolyl, Thiozolyl, Isoxazolyl, Thiadiazolyl und Pyridyl, wobei das 5- oder 6-gliedrige Heteroaryl partiell halogeniert sein kann und/oder ein bis zwei Substituenten aus der Gruppe Cyano, C₁-C₄-Alkyl, C₁-C₄-Halogenalkyl, C₁-C₄-Alkoxy-C₁-C₄-alkyl und C₁-C₄-Alkoxycarbonyl tragen kann;
bedeutet.

Ebenso bevorzugt sind die 3-heteroarylsubstituierten Isoxazoline der Formel I, in der
- X: 6-gliedriges Heteroaryl mit ein bis drei Stickstoffatomen,
bevorzugt 6-gliedriges Heteroaryl mit einem bis zwei Stickstoffatomen,
insbesondere Pyridyl, Pyrimidyl und Pyrazinyl,
sehr bevorzugt Pyridyl und Pyrimidyl;
wobei das 6-gliedrige Heteroaryl wie oben beschrieben substituiert ist;
- R¹-R⁷: Wasserstoff, C₁-C₄-Alkyl, wie z. B. Methyl oder Ethyl, oder C₁-C₄-Halogenalkyl, wie z. B. Difluormethyl, Trifluormethyl oder Trichlormethyl;
besonders bevorzugt Wasserstoff, Methyl oder Trifluormethyl;
sehr bevorzugt Wasserstoff;
insbesondere bevorzugt stehen R¹, R², R⁴-R⁷-für Wasserstoff, Methyl oder Trifluormethyl und R³ für Wasserstoff, Methyl, Ethyl oder Trifluormethyl; insbesondere sehr bevorzugt stehen R¹, R², R⁴-R⁷ für Wasserstoff und R³ für Methyl; und
- Y: Aryl, das partiell oder vollständig halogeniert sein kann und/oder ein bis zwei Substituenten aus der Gruppe Cyano, C₁-C₄-Alkyl und C₁-C₄-Halogenalkyl tragen kann;
besonders bevorzugt Aryl, das in einer oder beiden ortho-Positionen zur Verknüpfungsstelle halogeniert ist und/oder einen Substituenten aus der Gruppe Cyano, C₁-C₄-Alkyl und C₁-C₄-Halogenalkyl tragen kann;
insbesondere besonders bevorzugt Aryl, das in einer oder beiden ortho-Positionen zur Verknüpfungsstelle halogeniert ist;
bedeutet.

Außerordentlich bevorzugt sind die Verbindungen der Formel I.a (entspricht Formel I mit R¹, R², R⁴ - R⁷ = Wasserstoff, R³ = Methyl), insbesondere die Verbindungen der Formel I.a.1 bis I.a.234 der Tabelle 1, wobei die Definitionen der Variablen R¹ bis R⁷, X und Y nicht nur in Kombination miteinander sondern auch jeweils für sich allein betrachtet für die erfindungsgemäßen Verbindungen eine besondere Rolle spielen.

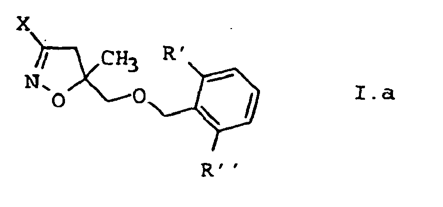

**Tabelle 1**

| Nr. | X | R' | R" |
|---|---|---|---|
| I.a.1 | 4-Fluorpyrid-3-yl | H | H |
| I.a.2 | 4-Fluorpyrid-3-yl | F | H |
| I.a.3 | 4-Fluorpyrid-3-yl | F | F |
| I.a.4 | 3-Chlorpyrid-2-yl | H | H |
| I.a.5 | 3-Chlorpyrid-2-yl | F | H |
| I.a.6 | 3-Chlorpyrid-2-yl | F | F |
| I.a.7 | 4-Chlorpyrid-3-yl | H | H |
| I.a.8 | 4-Chlorpyrid-3-yl | F | H |
| I.a.9 | 4-Chlorpyrid-3-yl | F | F |
| I.a.10 | 4-Brompyrid-3-yl | H | H |
| I.a.11 | 4-Brompyrid-3-yl | F | H |
| I.a.12 | 4-Brompyrid-3-yl | F | F |
| I.a.13 | 2-Methylpyrid-3-yl | H | H |
| I.a.14 | 2-Methylpyrid-3-yl | F | H |
| I.a.15 | 2-Methylpyrid-3-yl | F | F |
| I.a.16 | 4-Methylpyrid-3-yl | H | H |
| I.a.17 | 4-Methylpyrid-3-yl | F | H |
| I.a.18 | 4-Methylpyrid-3-yl | F | F |
| I.a.19 | 2-Trifluormethylpyrid-3-yl | H | H |
| I.a.20 | 2-Trifluormethylpyrid-3-yl | F | H |
| I.a.21 | 2-Trifluormethylpyrid-3-yl | F | F |
| I.a.22 | 2-Methoxypyrid-3-yl | H | H |
| I.a.23 | 2-Methoxypyrid-3-yl | F | H |
| I.a.24 | 2-Methoxypyrid-3-yl | F | F |
| I.a.25 | 2,6-Difluorpyrid-3-yl | H | H |
| I.a.26 | 2,6-Difluorpyrid-3-yl | F | H |
| I.a.27 | 2,6-Difluorpyrid-3-yl | F | F |
| I.a.28 | 2,3-Dichlorpyrid-4-yl | H | H |
| I.a.29 | 2,3-Dichlorpyrid-4-yl | F | H |
| I.a.30 | 2,3-Dichlorpyrid-4-yl | F | F |
| I.a.31 | 2,4-Dichlorpyrid-3-yl | H | H |
| I.a.32 | 2,4-Dichlorpyrid-3-yl | F | H |
| I.a.33 | 2,4-Dichlorpyrid-3-yl | F | F |
| I.a.34 | 2,6-Dichlorpyrid-3-yl | H | H |
| I.a.35 | 2,6-Dichlorpyrid-3-yl | F | H |
| I.a.36 | 2,6-Dichlorpyrid-3-yl | F | F |
| I.a.37 | 2,6-Dibrompyrid-3-yl | H | H |
| I.a.38 | 2,6-Dibrompyrid-3-yl | F | H |
| I.a.39 | 2,6-Dibrompyrid-3-yl | F | F |
| I.a.40 | 2,4-Dimethylpyrid-3-yl | H | H |
| I.a.41 | 2,4-Dimethylpyrid-3-yl | F | H |
| I.a.42 | 2,4-Dimethylpyrid-3-yl | F | F |
| I.a.43 | 2,6-Dimethylpyrid-3-yl | H | H |
| I.a.44 | 2,6-Dimethylpyrid-3-yl | F | H |
| I.a.45 | 2,6-Dimethylpyrid-3-yl | F | F |
| I.a.46 | 2-Fluor-6-methylpyrid-3-yl | H | H |
| I.a.47 | 2-Fluor-6-methylpyrid-3-yl | F | H |
| I.a.48 | 2-Fluor-6-methylpyrid-3-yl | F | F |
| I.a.49 | 2-Chlor-6-methylpyrid-3-yl | H | H |
| I.a.50 | 2-Chlor-6-methylpyrid-3-yl | F | H |
| I.a.51 | 2-Chlor-6-methylpyrid-3-yl | F | F |
| I.a.52 | 3-Chlor-6-methylpyrid-2-yl | H | H |
| I.a.53 | 3-Chlor-6-methylpyrid-2-yl | F | H |
| I.a.54 | 3-Chlor-6-methylpyrid-2-yl | F | F |
| I.a.55 | 3-Chlor-5-methylpyrid-4-yl | H | H |
| I.a.56 | 3-Chlor-5-methylpyrid-4-yl | F | H |
| I.a.57 | 3-Chlor-5-methylpyrid-4-yl | F | F |
| I.a.58 | 2-Brom-6-methylpyrid-3-yl | H | H |
| I.a.59 | 2-Brom-6-methylpyrid-3-yl | F | H |
| I.a.60 | 2-Brom-6-methylpyrid-3-yl | F | F |
| I.a.61 | 2-Methyl-3-chlorpyrid-4-yl | H | H |
| I.a.62 | 2-Methyl-3-chlorpyrid-4-yl | F | H |
| I.a.63 | 2-Methyl-3-chlorpyrid-4-yl | F | F |
| I.a.64 | 2-Methyl-6-chlorpyrid-3-yl | H | H |
| I.a.65 | 2-Methyl-6-chlorpyrid-3-yl | F | H |
| I.a.66 | 2-Methyl-6-chlorpyrid-3-yl | F | F |
| I.a.67 | 2,4,6-Trichlorpyrid-3-yl | H | H |
| I.a.68 | 2,4,6-Trichlorpyrid-3-yl | F | H |
| I.a.69 | 2,4,6-Trichlorpyrid-3-yl | F | F |
| I.a.70 | 2,4,6-Trimethylpyrid-3yl | H | H |
| I.a.71 | 2,4,6-Trimethylpyrid-3yl | F | H |
| I.a.72 | 2,4,6-Trimethylpyrid-3yl | F | F |
| I.a.73 | 2-Fluor-4,6-dimethylpyrid-3-yl | H | H |
| I.a.74 | 2-Fluor-4,6-dimethylpyrid-3-yl | F | H |
| I.a.75 | 2-Fluor-4,6-dimethylpyrid-3-yl | F | F |
| I.a.76 | 2-Chlor-4,6-dimethylpyrid-3-yl | H | H |
| I.a.77 | 2-Chlor-4,6-dimethylpyrid-3-yl | F | H |
| I.a.78 | 2-Chlor-4,6-dimethylpyrid-3-yl | F | F |
| I.a.79 | 5-Chlor-2,3-dimethylpyrid-4-yl | H | H |
| I.a.80 | 5-Chlor-2,3-dimethylpyrid-4-yl | F | H |
| I.a.81 | 5-Chlor-2,3-dimethylpyrid-4-yl | F | F |
| I.a.82 | 3,5-Dichlor-2-methylpyrid-4-yl | H | H |
| I.a.83 | 3,5-Dichlor-2-methylpyrid-4-yl | F | H |
| I.a.84 | 3,5-Dichlor-2-methylpyrid-4-yl | F | F |
| I.a.85 | 2-Brom-4,6-dimethylpyrid-3-yl | H | H |
| I.a.86 | 2-Brom-4,6-dimethylpyrid-3-yl | F | H |
| I.a.87 | 2-Brom-4,6-dimethylpyrid-3-yl | F | F |
| I.a.88 | 4-Chlorpyrimidin-5-yl | H | H |
| I.a.89 | 4-Chlorpyrimidin-5-yl | F | H |
| I.a.90 | 4-Chlorpyrimidin-5-yl | F | F |
| I.a.91 | 4,6-Dichlorpyrimidin-5-yl | H | H |
| I.a.92 | 4,6-Dichlorpyrimidin-5-yl | F | H |
| I.a.93 | 4,6-Dichlorpyrimidin-5-yl | F | F |
| I.a.94 | 4-Methylpyrimidin-5-yl | H | H |
| I.a.95 | 4-Methylpyrimidin-5-yl | F | H |
| I.a.96 | 4-Methylpyrimidin-5-yl | F | F |
| I.a.97 | 4,6-Dimethylpyrimidin-5-yl | H | H |
| I.a.98 | 4,6-Dimethylpyrimidin-5-yl | F | H |
| I.a.99 | 4,6-Dimethylpyrimidin-5-yl | F | F |
| I.a.100 | 2-Chlorpyrazin-3-yl | H | H |
| I.a.101 | 2-Chlorpyrazin-3-yl | F | H |
| I.a.102 | 2-Chlorpyrazin-3-yl | F | F |
| I.a.103 | 2-Methylpyrazin-3-yl | H | H |
| I.a.104 | 2-Methylpyraz üz-3-yl | F | H |
| I.a.105 | 2-Methylpyrazin-3-yl | F | F |
| I.a.106 | 3-Chlorpyridazin-4-yl | H | H |
| I.a.107 | 3-Chlorpyridazin-4-yl | F | H |
| I.a.108 | 3-Chlorpyridazin-4-yl | F | F |
| I.a.109 | 3-Methylpyridazin-4-yl | H | H |
| I.a.110 | 3-Methylpyridazin-4-yl | F | H |
| I.a.111 | 3-Methylpyridazin-4-yl | F | F |
| I.a. 112 | 4-Cyanopyrid-3-yl | F | F |
| I.a. 113 | 4-Cyanopyrid-3-yl | H | H |
| I.a. 114 | 4-Cyanopyrid-3-yl | F | F |
| I.a. 115 | 4-Trifluormethylpyrid-3-yl | F | F |
| I.a. 116 | 4-Trifluormethylpyrid-3-yl | H | H |
| I.a. 117 | 4-Trifluormethylpyrid-3-yl | F | H |
| I.a. 118 | 4-Methoxypyrid-3-yl | F | F |
| I.a. 119 | 4-Methoxypyrid-3-yl | H | H |
| I.a. 120 | 4-Methoxypyrid-3-yl | F | H |
| I.a. 121 | 2-Fluoridpyrid-3-yl | F | F |
| I.a. 122 | 2-Fluoridpyrid-3-yl | H | H |
| I.a. 123 | 2-Fluoridpyrid-3-yl | F | H |
| I.a. 124 | 2-Chlorpyrid-3-yl | F | F |
| I.a. 125 | 2-Chlorpyrid-3-yl | H | H |
| I.a. 126 | 2-Chlorpyrid-3-yl | F | H |
| I.a. 127 | 2-Brompyrid-3-yl | F | F |
| I.a. 128 | 2-Brompyrid-3-yl | H | H |
| I.a. 129 | 2-Brompyrid-3-yl | F | H |
| I.a. 130 | 2-Cyanopyrid-3-yl | F | F |
| I.a. 131 | 2-Cyanopyrid-3-yl | H | H |
| I.a. 132 | 2-Cyanopyrid-3-yl | F | H |
| I.a. 133 | 2-Fluorpyrazin-3-yl | F | F |
| I.a. 134 | 2-Fluorpyrazin-3-yl | H | H |
| I.a. 135 | 2-Fluorpyrazin-3-yl | F | H |
| I.a. 136 | 2-Brompyrazin-3-yl | F | F |
| I.a. 137 | 2-Brompyrazin-3-yl | H | H |
| I.a. 138 | 2-Brompyrazin-3-yl | F | H |
| I.a. 139 | 2-Cyanopyrazin-3-yl | F | F |
| I.a. 140 | 2-Cyanopyrazin-3-yl | H | H |
| I.a. 141 | 2-Cyanopyrazin-3-yl | F | H |
| I.a. 142 | 2-Trifluormethylpyrazin-3-yl | F | F |
| I.a. 143 | 2-Trifluormethylpyrazin-3-yl | H | H |
| I.a. 144 | 2-Trifluormethylpyrazin-3-yl | F | H |
| I.a. 145 | 2-Methoxypyrazin-3-yl | F | F |
| I.a. 146 | 2-Methoxypyrazin-3-yl | H | H |
| I.a. 147 | 2-Methoxypyrazin-3-yl | F | H |

Ebenso außerordentlich bevorzugt sind die Verbindungen der Formel I.b, insbesondere die Verbindungen der Formel I.b.1 bis I.b. 147, die sich von den entsprechenden Verbindungen der Formel I.a.1 bis I.a. 147dadurch unterscheiden, daß R³ für Wasserstoff steht.

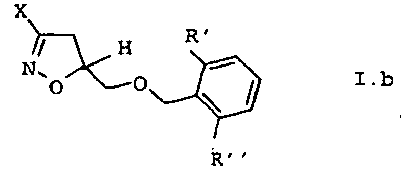

Ebenso außerordentlich bevorzugt sind die Verbindungen der Formel I.c, insbesondere die Verbindungen der Formel I.c.1 bis I.c.147, die sich von den entsprechenden Verbindungen der Formel I.a.1 bis I.a. 147 dadurch unterscheiden, daß R³ für Ethyl steht.

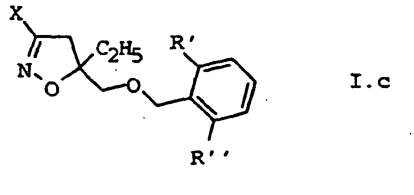

Ebenso außerordentlich bevorzugt sind die Verbindungen der Formel I.d, insbesondere die Verbindungen der Formel I.d.1 bis I.d. 147, die sich von den entsprechenden Verbindungen der Formel I.a.1 bis I.a. 147 dadurch unterscheiden, daß Y für entsprechend substituiert Naphth-2-yl steht.

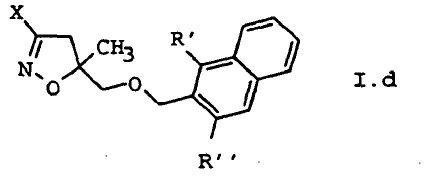

Ebenso außerordentlich bevorzugt sind die Verbindungen der Formel I.e, insbesondere die Verbindungen der Formel I.e.1 bis I.e. 147, die sich von den entsprechenden Verbindungen der Formel I.a.1 bis I.a. 147dadurch unterscheiden, daß R³ für Wasserstoff und Y für entsprechend substituiert Naphth-2-yl steht.

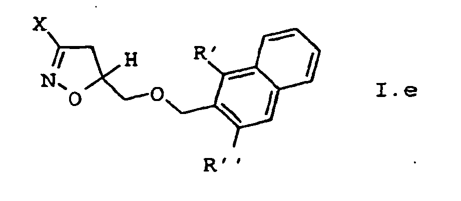

Ebenfalls außerordentlich bevorzugt sind die Verbindungen der Formel I.f (entspricht Formel I mit X = 4-Fluorpyrid-3-yl und R¹, R², R⁴ - R⁷ = Wasserstoff), insbesondere die Verbindungen der Formel I.f.1 bis I.f.228 der Tabelle 1A, wobei die Definitionen der Variablen R¹ bis R⁷, X und Y nicht nur in Kombination miteinander sondern auch jeweils für sich allein betrachtet für die erfindungsgemäßen Verbindungen eine besondere Rolle spielen.

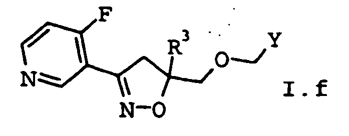

**Tabelle 1A**

| Nr. | R³ | Y |
|---|---|---|
| I.f.1 | H | 3-Fluorphenyl |
| I.f.2 | CH₃ | 3-Fluorphenyl |
| I.f.3 | C₂H₅ | 3-Fluorphenyl |
| I.f.4 | H | 4-Fluorphenyl |
| I.f.5 | CH₃ | 4-Fluorphenyl |
| I.f.6 | C₂H₅ | 4-Fluorphenyl |
| I.f.7 | H | 2-Chlorphenyl |
| I.f.8 | CH₃ | 2-Chlorphenyl |
| I.f.9 | C₂H₅ | 2-Chlorphenyl |
| I.f.10 | H | 3-Chlorphenyl |
| I.f.11 | CH₃ | 3-Chlorphenyl |
| I.f.12 | C₂H₅ | 3-Chlorphenyl |
| I.f.13 | H | 4-Chlorphenyl |
| I.f.14 | CH₃ | 4-Chlorphenyl |
| I.f.15 | C₂H₅ | 4-Chlorphenyl |
| I.f.16 | H | 2-Bromphenyl |
| I.f.17 | CH₃ | 2-Bromphenyl |
| I.f.18 | C₂H₅ | 2-Bromphenyl |
| I.f.19 | H | 3-Bromphenyl |
| I.f.20 | CH₃ | 3-Bromphenyl |
| I.f.21 | C₂H₅ | 3-Bromphenyl |
| I.f.22 | H | 4-Bromphenyl |
| I.f.23 | CH₃ | 4-Bromphenyl |
| I.f.24 | C₂H₅ | 4-Bromphenyl |
| I.f.25 | H | 2-Iodphenyl |
| I.f.26 | CH₃ | 2-Iodphenyl |
| I.f.27 | C₂H₅ | 2-Iodphenyl |
| I.f.28 | H | 2,4-Difluorphenyl |
| I.f.29 | CH₃ | 2,4-Difluorphenyl |
| I.f.30 | C₂H₅ | 2,4-Difluorphenyl |
| I.f.31 | H | 3,5-Difluorphenyl |
| I.f.32 | CH₃ | 3,5-Difluorphenyl |
| I.f.33 | C₂H₅ | 3,5-Difluorphenyl |
| I.f.34 | H | 2,6-Dichlorphenyl |
| I.f.35 | CH₃ | 2,6-Dichlorphenyl |
| I.f.36 | C₂H₅ | 2,6-Dichlorphenyl |
| I.f.37 | H | 2,4-Dichlorphenyl |
| I.f.38 | CH₃ | 2,4-Dichlorphenyl |
| I.f.39 | C₂H₅ | 2,4-Dichlorphenyl |
| I.f.40 | H | 3,4-Dichlorphenyl |
| I.f.41 | CH₃ | 3,4-Dichlorphenyl |
| I.f.42 | C₂H₅ | 3,4-Dichlorphenyl |
| I.f.43 | H | 2,4,6-Trichlorphenyl |
| I.f.44 | CH₃ | 2,4,6-Trichlorphenyl |
| I.f.45 | C₂H₅ | 2,4,6-Trichlorphenyl |
| I.f.46 | H | 2,3,5-Trichlorphenyl |
| I.f.47 | CH₃ | 2,3,5-Trichlorphenyl |
| I.f.48 | C₂H₅ | 2,3,5-Trichlorphenyl |
| I.f.49 | H | 2,3,4,5,6-Pentafluorphenyl |
| I.f.50 | CH₃ | 2,3,4,5,6-Pentafluorphenyl |
| I.f.51 | C₂H₅ | 2,3,4,5,6-Pentafluorphenyl |
| I.f.52 | H | 2-Nitrophenyl |
| I.f.53 | CH₃ | 2-Nitrophenyl |
| I.f.54 | C₂H₅ | 2-Nitrophenyl |
| I.f.55 | H | 3-Nitrophenyl |
| I.f.56 | CH₃ | 3-Nitrophenyl |
| I.f.57 | C₂H₅ | 3-Nitrophenyl |
| I.f.58 | H | 4-Nitrophenyl |
| I.f.59 | CH₃ | 4-Nitrophenyl |
| I.f.60 | C₂H₅ | 4-Nitrophenyl |
| I.f.61 | H | 2-Cyanophenyl |
| I.f.62 | CH₃ | 2-Cyanophenyl |
| I.f.63 | C₂H₅ | 2-Cyanophenyl |
| I.f.64 | H | 3-Cyanophenyl |
| I.f.65 | CH₃ | 3-Cyanophenyl |
| I.f.66 | C₂H₅ | 3-Cyanophenyl |
| I.f.67 | H | 4-Cyanophenyl |
| I.f.68 | CH₃ | 4-Cyanophenyl |
| I.f.69 | C₂H₅ | 4-Cyanophenyl |
| I.f.70 | H | 2-Methylphenyl |
| I.f.71 | CH₃ | 2-Methylphenyl |
| I.f.72 | C₂H₅ | 2-Methylphenyl |
| I.f.73 | H | 3-Methylphenyl |
| I.f.74 | CH₃ | 3-Methylphenyl |
| I.f.75 | C₂H₅ | 3-Methylphenyl |
| I.f.76 | H | 4-Methylphenyl |
| I.f.77 | CH₃ | 4-Methylphenyl |
| I.f.78 | C₂H₅ | 4-Methylphenyl |
| I.f.79 | H | 4-Ethylphenyl |
| I.f.80 | CH₃ | 4-Ethylphenyl |
| I.f.81 | C₂H₅ | 4-Ethylphenyl |
| I.f.82 | H | 2,5-Dimethylphenyl |
| I.f.83 | CH₃ | 2,5-Dimethylphenyl |
| I.f.84 | C₂H₅ | 2,5-Dimethylphenyl |
| I.f.85 | H | 3,4-Dimethylphenyl |
| I.f.86 | CH₃ | 3,4-Dimethylphenyl |
| I.f.87 | C₂H₅ | 3,4-Dimethylphenyl |
| I.f.88 | H | 2,4-Dimethylphenyl |
| I.f.89 | CH₃ | 2,4-Dimethylphenyl |
| I.f.90 | C₂H₅ | 2,4-Dimethylphenyl |
| I.f.91 | H | 2,4,6-Trimethylphenyl |
| I.f.92 | CH₃ | 2,4,6-Trimethylphenyl |
| I.f.93 | C₂H₅ | 2,4,6-Trimethylphenyl |
| I.f.94 | H | 2-Trifluormethylphenyl |
| I.f.95 | CH₃ | 2-Trifluormethylphenyl |
| I.f.96 | C₂H₅ | 2-Trifluormethylphenyl |
| I.f.97 | H | 3-Trifluormethylphenyl |
| I.f.98 | CH₃ | 3-Trifluormethylphenyl |
| I.f.99 | C₂H₅ | 3-Trifluormethylphenyl |
| I.f.100 | H | 4-Trifluormethylphenyl |
| I.f.101 | CH₃ | 4-Trifluormethylphenyl |
| I.f.102 | C₂H₅ | 4-Trifluormethylphenyl |
| I.f.103 | H | 2-Methoxyphenyl |
| I.f.104 | CH₃ | 2-Methoxyphenyl |
| I.f.105 | C₂H₅ | 2-Methoxyphenyl |
| I.f.106 | H | 3-Methoxyphenyl |
| I.f.107 | CH₃ | 3-Methoxyphenyl |
| I.f.108 | C₂H₅ | 3-Methoxyphenyl |
| I.f.109 | H | 4-Methoxyphenyl |
| I.f.110 | CH₃ | 4-Methoxyphenyl |
| I-f-111 | C₂H₅ | 4-Methoxyphenyl |
| I.f.112 | H | 3,4,5-Trimethoxyphenyl |
| I.f.113 | CH₃ | 3,4,5-Trimethoxyphenyl |
| I.f.114 | C₂H₅ | 3,4,5-Trimethoxyphenyl |
| I.f.115 | H | 3-Trifluormethoxyphenyl |
| I.f.116 | CH₃ | 3-Trifluormethoxyphenyl |
| I.f.117 | C₂H₅ | 3-Trifluormethoxyphenyl |
| I.f.118 | H | 2-Difluormethoxyphenyl |
| I.f.119 | CH₃ | 2-Difluormethoxyphenyl |
| I.f.120 | C₂H₅ | 2-Difluormethoxyphenyl |
| I.f.121 | H | 2-(Ethoxycarbonylmethoxy) phenyl |
| I.f.122 | CH₃ | 2-(Ethoxycarbonylmethoxy)phenyl |
| I.f.123 | C₂H₅ | 2-(Ethoxycarbonylmethoxy)phenyl |
| I.f.124 | H | 4-(Phenyl)phenyl |
| I.f.125 | CH₃ | 4-(Phenyl) phenyl |
| I.f.126 | C₂H₅ | 4-(Phenyl)phenyl |
| I.f.127 | H | 3-Phenoxyphenyl |
| I.f.128 | CH₃ | 3-Phenoxyphenyl |
| I.f.129 | C₂H₅ | 3-Phenoxyphenyl |
| I.f.130 | H | 2-Methyl-3-(phenyl)phenyl |
| I.f.131 | CH₃ | 2-Methyl-3-(phenyl)phenyl |
| I.f.132 | C₂H₅ | 2-Methyl-3-(phenyl)phenyl |
| I.f.133 | H | 3-Phenoxyphenyl |
| I.f.134 | CH₃ | 3-Phenoxyphenyl |
| I.f.135 | C₂H₅ | 3-Phenoxyphenyl |
| I.f.136 | H | 3-(Phenylcarbonyl)phenyl |
| I.f.137 | CH₃ | 3-(Phenylcarbonyl)phenyl |
| I.f.138 | C₂H₅ | 3-(Phenylcarbonyl)phenyl |
| I.f.139 | H | 4-(Methoxycarbonyl)phenyl |
| I.f.140 | CH₃ | 4-(Methoxycarbonyl)phenyl |
| I.f.141 | C₂H₅ | 4-(Methoxycarbonyl)phenyl |
| I.f.142 | H | 4-(Isopropoxycarbonyl)phenyl |
| I.f.143 | CH₃ | 4-(Isopropoxycarbonyl)phenyl |
| I.f.144 | C₂H₅ | 4-(Isopropoxycarbonyl)phenyl |
| I.f.145 | H | 2-Chlor-6-nitrophenyl |
| I.f.146 | CH₃ | 2-Chlor-6-nitrophenyl |
| I.f.147 | C₂H₅ | 2-Chlor-6-nitrophenyl |
| I.f.148 | H | 3-Chlor-4-methoxyphenyl |
| I.f.149 | CH₃ | 3-Chlor-4-methoxyphenyl |
| I.f.150 | C₂H₅ | 3-Chlor-4-methoxyphenyl |
| I.f.151 | H | 3-Chlor-4-ethoxyphenyl |
| I.f.152 | CH₃ | 3-Chlor-4-ethoxyphenyl |
| I.f.153 | C₂H₅ | 3-Chlor-4-ethoxyphenyl |
| I.f.154 | H | 2-Ethyl-5-nitrophenyl |
| I.f.155 | CH₃ | 2-Ethyl-5-nitrophenyl |
| I.f.156 | C₂H₅ | 2-Ethyl-5-nitrophenyl |
| I.f.157 | H | 2,3-Dichlor-4-methoxyphenyl |
| I.f.158 | CH₃ | 2,3-Dichlor-4-methoxyphenyl |
| I.f.159 | C₂H₅ | 2,3-Dichlor-4-methoxyphenyl |
| I.f.160 | H | 2,3-Dichlor-4-isopropoxyphenyl |
| I.f.161 | CH₃ | 2,3-Dichlor-4-isopropoxyphenyl |
| I.f.162 | C₂H₅ | 2,3-Dichlor-4-isopropoxyphenyl |
| I.f.163 | H | 2,4-Dichlor-6-nitrophenyl |
| I.f.164 | CH₃ | 2,4-Dichlor-6-nitrophenyl |
| I.f.165 | C₂H₅ | 2,4-Dichlor-6-nitrophenyl |
| I.f.166 | H | 2-Chlor-3-methyl-4-(methylsulfonyl)phenyl |
| I.f.167 | CH₃ | 2-Chlor-3-methyl-4-(methylsulfonyl)phenyl |
| I.f.168 | C₂H₅ | 2-Chlor-3-methyl-4-(methylsulfonyl)phenyl |
| I.f.169 | H | Benzo[1,4]dioxan-6-yl |
| I.f.170 | CH₃ | Benzo[1,4]dioxan-6-yl |
| I.f.171 | C₂H₅ | Benzo[1,4]dioxan-6-yl |
| I.f.172 | H | Benzo[1,3]dioxolan-5-yl |
| I.f.173 | CH₃ | Benzo[1,3]dioxolan-5-yl |
| I.f.174 | C₂H₅ | Benzo[1,3]dioxolan-5-yl |
| I.f.175 | H | 2,3-Dihydrobenzofuran-2-yl |
| I.f.176 | CH₃ | 2,3-Dihydrobenzofuran-2-yl |
| I.f.177 | C₂H₅ | 2,3-Dihydrobenzofuran-2-yl |
| I.f.178 | H | Benzimidazol-2-yl |
| I.f.179 | CH₃ | Benzimidazol-2-yl |
| I.f.180 | C₂H₅ | Benzimidazol-2-yl |
| I.f.181 | H | Naphth-1-yl |
| I.f.182 | CH₃ | Naphth-1-yl |
| I.f.183 | C₂H₅ | Naphth-1-yl |
| I.f.184 | H | 2-Methylnapht-1-yl |
| I.f.185 | CH₃ | 2-Methylnapht-1-yl |
| I.f.186 | C₂H₅ | 2-Methylnapht-1-yl |
| I.f.187 | H | Anthrancen-9-yl |
| I.f.188 | CH₃ | Anthrancen-9-yl |
| I.f.189 | C₂H₅ | Anthrancen-9-yl |
| I.f.190 | H | 5-Chlor-l-methylpyrazol-4-yl |
| I.f.191 | CH₃ | 5-Chlor-1-methylpyrazol-4-yl |
| I.f.192 | C₂H₅ | 5-Chlor-1-methylpyrazol-4-yl |
| I.f.193 | H | 5-Chlor-1-methyl-3-trifluormethylpyrazol-4-yl |
| I.f.194 | CH₃ | 5-Chlor-1-methyl-3-trifluormethylpyrazol-4-yl |
| I.f.195 | C₂H₅ | 5-Chlor-1-methyl-3-trifluormethylpyrazol-4-yl |
| I.f.196 | H | Thiazol-4-yl |
| I.f.197 | CH₃ | Thiazol-4-yl |
| I.f.198 | C₂H₅ | Thiazol-4-yl |
| I.f.199 | H | 2-Chlorthiazol-5-yl |
| I.f.200 | CH₃ | 2-Chlorthiazol-5-yl |
| I.f.201 | C₂H₅ | 2-Chlorthiazol-5-yl |
| I.f.202 | H | 3-Ethylisoxazol-5-yl |
| I.f.203 | CH₃ | 3-Ethylisoxazol-5-yl |
| I.f.204 | C₂H₅ | 3-Ethylisoxazol-5-yl |
| I.f.205 | H | 3-nPropylisoxazol-5-yl |
| I.f.206 | CH₃ | 3-nPropylisoxazol-5-yl |
| I.f.207 | C₂H₅ | 3-nPropylisoxazol-5-yl |
| I.f.208 | H | 3-(Methoxymethyl)isoxazol-5-yl |
| I.f.209 | CH₃ | 3-(Methoxymethyl)isoxazol-5-yl |
| I.f.210 | C₂H₅ | 3-(Methoxymethyl)isoxazol-5-yl |
| I.f.211 | H | 5-Methoxymethyl-1,3,4-thiadiazol-2-yl |
| I.f.212 | CH₃ | 5-Methoxymethyl-1,3,4-thiadiazol-2-yl |
| I.f.213 | C₂H₅ | 5-Methoxymethyl-1,3,4-thiadiazol-2-yl |
| I.f.214 | H | Pyrid-2-yl |
| I.f.215 | CH₃ | Pyrid-2-yl |
| I.f.216 | C₂H₅ | Pyrid-2-yl |
| I.f.217 | H | Pyrid-3-yl |
| I.f.218 | CH₃ | Pyrid-3-yl |
| I.f.219 | C₂H₅ | Pyrid-3-yl |
| I.f.220 | H | Pyrid-4-yl |
| I.f.221 | CH₃ | Pyrid-4-yl |
| I.f.222 | C₂H₅ | Pyrid-4-yl |
| I.f.223 | H | 2-Chlorpyrid-5-yl |
| I.f.224 | CH₃ | 2-Chlorpyrid-5-yl |
| I.f.225 | C₂H₅ | 2-Chlorpyrid-5-yl |
| I.f.226 | H | 5-Ethyl-3-(ethoxycarbonyl)pyrid-2-yl |
| I.f.227 | CH₃ | 5-Ethyl-3-(ethoxycarbonyl)pyrid-2-yl |
| I.f.228 | C₂H₅ | 5-Ethyl-3-(ethoxycarbonyl)pyrid-2-yl |

Ebenso außerordentlich bevorzugt sind die Verbindungen der Formel I.g, insbesondere die Verbindungen der Formel I.g.1 bis I.g.228, die sich von den entsprechenden Verbindungen der Formel I.f.1 bis I.f.228 dadurch unterscheiden, daß X für 4-Chlorpyrid-3-yl steht.

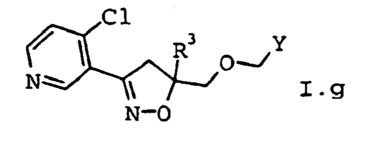

Ebenso außerordentlich bevorzugt sind die Verbindungen der Formel I.h, insbesondere die Verbindungen der Formel I.h.1 bis I.h.228, die sich von den entsprechenden Verbindungen der Formel I.f.1 bis I.f.228 dadurch unterscheiden, daß X für 4-Brompyrid-3-yl steht.

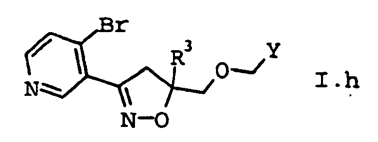

Ebenso außerordentlich bevorzugt sind die Verbindungen der Formel I.i, insbesondere die Verbindungen der Formel I.i.1 bis I.i.228, die sich von den entsprechenden Verbindungen der Formel I.f.1 bis I.f.228 dadurch unterscheiden, daß X für 4-Cyanopyrid-3-yl steht.

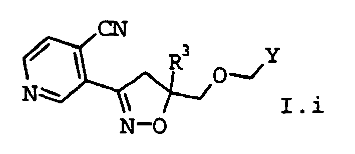

Ebenso außerordentlich bevorzugt sind die Verbindungen der Formel I.k, insbesondere die Verbindungen der Formel I.k.1 bis I.k.228, die sich von den entsprechenden Verbindungen der Formel I.f.1 bis I.f.228 dadurch unterscheiden, daß X für 4-Methylpyrid-3-yl steht.

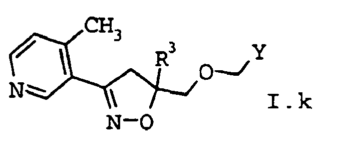

Ebenso außerordentlich bevorzugt sind die Verbindungen der Formel I.1, insbesondere die Verbindungen der Formel 1.1.1 bis 1.1.228, die sich von den entsprechenden Verbindungen der Formel I.f.1 bis I.f.228 dadurch unterscheiden, daß X für 4-Trifluormethylpyrid-3-yl steht.

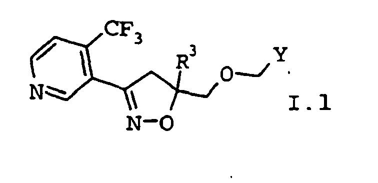

Ebenso außerordentlich bevorzugt sind die Verbindungen der Formel I.m, insbesondere die Verbindungen der Formel I.m.1 bis I.m.228, die sich von den entsprechenden Verbindungen der Formel I.f.1 bis I.f.228 dadurch unterscheiden, daß X für 4-Methoxypyrid-3-yl steht.

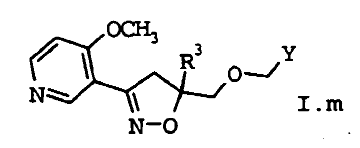

Ebenso außerordentlich bevorzugt sind die Verbindungen der Formel I.n, insbesondere die Verbindungen der Formel I.n.1 bis I.n.228, die sich von den entsprechenden Verbindungen der Formel I.f.1 bis I.f.228 dadurch unterscheiden, daß X für 2-Fluorpyrid-3-yl steht.

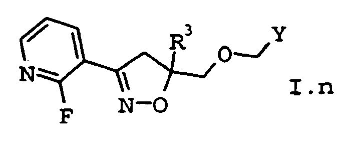

Ebenso außerordentlich bevorzugt sind die Verbindungen der Formel I.o, insbesondere die Verbindungen der Formel I.o.1 bis I.o.228, die sich von den entsprechenden Verbindungen der Formel 1.f.1 bis I.f.228 dadurch unterscheiden, daß X für 2-Chlorpyrid-3-yl steht.

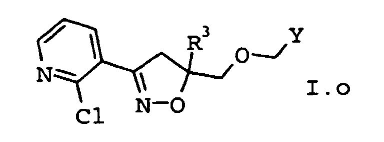

Ebenso außerordentlich bevorzugt sind die Verbindungen der Formel I.p, insbesondere die Verbindungen der Formel I.p.1 bis I.p.228, die sich von den entsprechenden Verbindungen der Formel I.f.1 bis I.f.228 dadurch unterscheiden, daß X für 2-Brompyrid-3-yl steht.

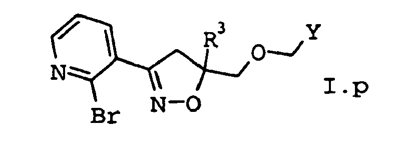

Ebenso außerordentlich bevorzugt sind die Verbindungen der Formel I.q, insbesondere die Verbindungen der Formel I.q.1 bis I.q.228, die sich von den entsprechenden Verbindungen der Formel I.f.1 bis I.f.228 dadurch unterscheiden, daß X für 2-Cyanopyrid-3-yl steht.

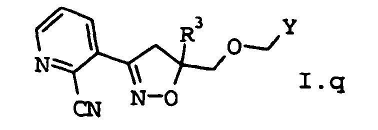

Ebenso außerordentlich bevorzugt sind die Verbindungen der Formel I.r, insbesondere die Verbindungen der Formel I.r.1 bis I.r.228, die sich von den entsprechenden Verbindungen der Formel I.f.1 bis I.f.228 dadurch unterscheiden, daß X für 2-Methylpyrid-3-yl steht.

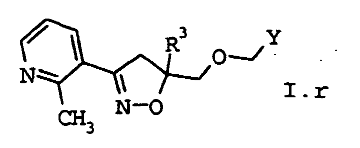

Ebenso außerordentlich bevorzugt sind die Verbindungen der Formel I.s, insbesondere die Verbindungen der Formel 1.s.1 bis I.s.228, die sich von den entsprechenden Verbindungen der Formel I.f.1 bis I.f.228 dadurch unterscheiden, daß X für 2-Trifluormethylpyrid-3-yl steht.

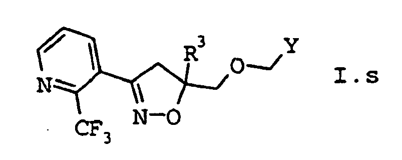

Ebenso außerordentlich bevorzugt sind die Verbindungen der Formel I.t, insbesondere die Verbindungen der Formel I.t.1 bis I.t.228, die sich von den entsprechenden Verbindungen der Formel I.f.1 bis I.f.228 dadurch unterscheiden, daß X für 2-Methoxypyrid-3-yl steht.

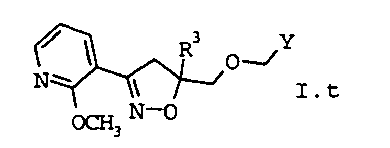

Ebenso außerordentlich bevorzugt sind die Verbindungen der Formel I.u, insbesondere die Verbindungen der Formel I.u.1 bis I.u.228, die sich von den entsprechenden Verbindungen der Formel I.f.1 bis I.f.228 dadurch unterscheiden, daß X für 2,4-Dichlorpyrid-3-yl steht.

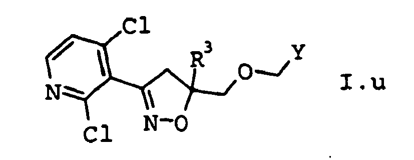

Ebenso außerordentlich bevorzugt sind die Verbindungen der Formel I.v, insbesondere die Verbindungen der Formel I.v.1 bis I.v.228, die sich von den entsprechenden Verbindungen der Formel I.f.1 bis I.f.228 dadurch unterscheiden, daß X für 2,4-Dimethylpyrid-3-yl steht.

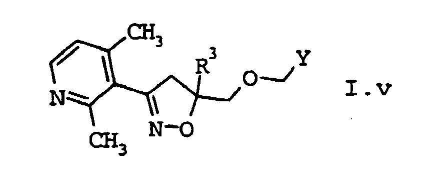

Ebenso außerordentlich bevorzugt sind die Verbindungen der Formel I.w, insbesondere die Verbindungen der Formel I.w.1 bis I.w.228, die sich von den entsprechenden Verbindungen der Formel I.f.1 bis I.f.228 dadurch unterscheiden, daß X für 2,6-Difluorpyrid-3-yl steht.

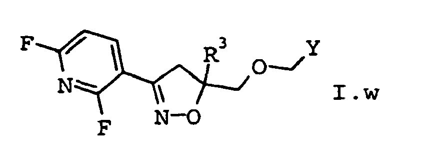

Ebenso außerordentlich bevorzugt sind die Verbindungen der Formel I.x, insbesondere die Verbindungen der Formel I.x.1 bis I.x.228, die sich von den entsprechenden Verbindungen der Formel I.f.1 bis I.f.228 dadurch unterscheiden, daß X für 2,6-Dichlorpyrid-3-yl steht.

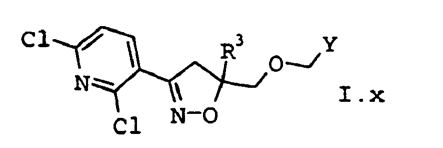

Ebenso außerordentlich bevorzugt sind die Verbindungen der Formel I.y, insbesondere die Verbindungen der Formel I.y.1 bis I.y.228, die sich von den entsprechenden Verbindungen der Formel I.f.1 bis I.f.228 dadurch unterscheiden, daß X für 2,6-Dibrompyrid-3-yl steht.

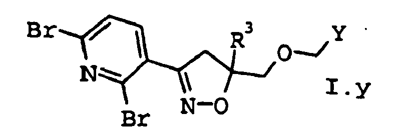

Ebenso außerordentlich bevorzugt sind die Verbindungen der Formel I.z, insbesondere die Verbindungen der Formel I.z.1 bis I.z.228, die sich von den entsprechenden Verbindungen der Formel I.f.1 bis I.f.228 dadurch unterscheiden, daß X für 2,6-Dimethylpyrid-3-yl steht.

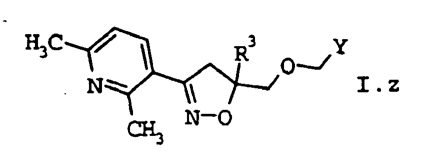

Ebenso außerordentlich bevorzugt sind die Verbindungen der Formel I.aa, insbesondere die Verbindungen der Formel I.aa.1 bis I.aa.228, die sich von den entsprechenden Verbindungen der Formel I.f.1 bis I.f.228 dadurch unterscheiden, daß X für 2-Chlor-6-Methylpyrid-3-yl steht.

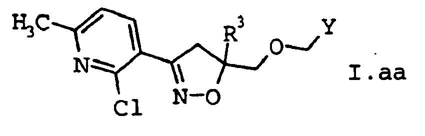

Ebenso außerordentlich bevorzugt sind die Verbindungen der Formel I.ab, insbesondere die Verbindungen der Formel I.ab.1 bis I.ab.228, die sich von den entsprechenden Verbindungen der Formel I.f.1 bis I.f.228 dadurch unterscheiden, daß X für 6-Chlor-2-Methylpyrid-3-yl steht.

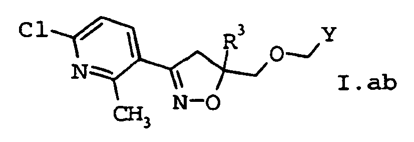

Ebenso außerordentlich bevorzugt sind die Verbindungen der Formel I.ac, insbesondere die Verbindungen der Formel I.ac.1 bis I.ac.228, die sich von den entsprechenden Verbindungen der Formel I.f.1 bis I.f.228 dadurch unterscheiden, daß X für 2,4,6-Trichlorpyrid-3-yl steht.

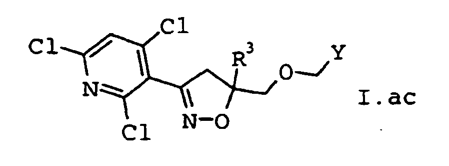

Ebenso außerordentlich bevorzugt sind die Verbindungen der Formel I.ad, insbesondere die Verbindungen der Formel I.ad.1 bis I.ad.228, die sich von den entsprechenden Verbindungen der Formel I.f.1 bis I.f.228 dadurch unterscheiden, daß X für 2,4-Difluor-6-methylpyrid-3-yl steht.

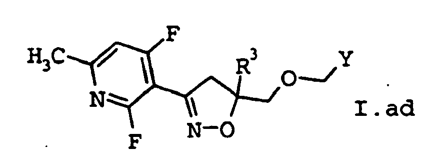

Ebenso außerordentlich bevorzugt sind die Verbindungen der Formel I.ae, insbesondere die Verbindungen der Formel I.ae.1 bis I.ae.228, die sich von den entsprechenden Verbindurigen der Formel I.f.1 bis I.f.228 dadurch unterscheiden, daß X für 2,4,6-Trimethylpyrid-3-yl steht.

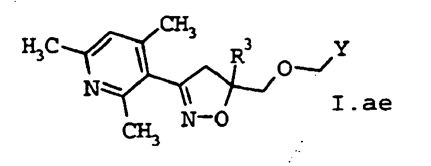

Ebenso außerordentlich bevorzugt sind die Verbindungen der Formel I.af, insbesondere die Verbindungen der Formel I.af.1 bis I.af.228, die sich von den entsprechenden Verbindungen der Formel I.f.1 bis I.f.228 dadurch unterscheiden, daß X für 4,6-Dimethyl-2-fluor-pyrid-3-yl steht.

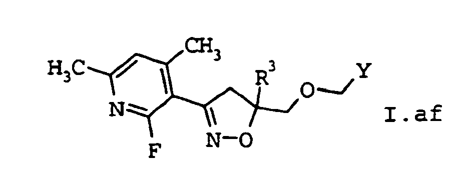

Ebenso außerordentlich bevorzugt sind die Verbindungen der Formel I.ag, insbesondere die Verbindungen der Formel I.ag.1 bis I.ag.228, die sich von den entsprechenden Verbindungen der Formel I.f.1 bis I.f.228 dadurch unterscheiden, daß X für 2-Chlor-4,6-dimethylpyrid-3-yl steht.

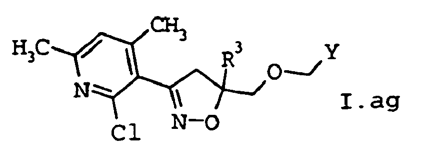

Ebenso außerordentlich bevorzugt sind die Verbindungen der Formel I.ah, insbesondere die Verbindungen der Formel I.ah.1 bis I.ah.228, die sich von den entsprechenden Verbindungen der Formel I.f.1 bis I.f.228 dadurch unterscheiden, daß X für 2-Brom-4-Fluor-6-methylpyrid-3-yl steht.

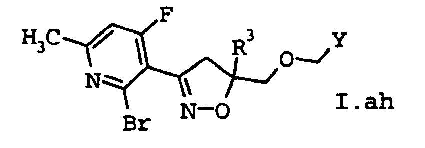

Ebenso außerordentlich bevorzugt sind die Verbindungen der Formel I.ai, insbesondere die Verbindungen der Formel I.ai.1 bis I.ai.228, die sich von den entsprechenden Verbindungen der Formel I.f.1 bis I.f.228 dadurch unterscheiden, daß X für 3-Chlorpyrid-2-yl steht.

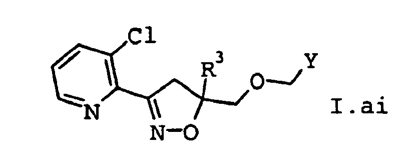

Ebenso außerordentlich bevorzugt sind die Verbindungen der Formel I.ak, insbesondere die Verbindungen der Formel I.ak.1 bis I.ak.228, die sich von den entsprechenden Verbindungen der Formel I.f.1 bis I.f.228 dadurch unterscheiden, daß X für 3-Chlor-6-methylpyrid-2-yl steht.

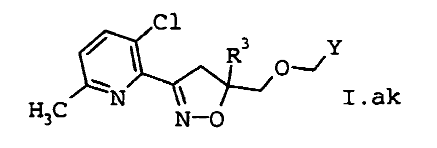

Ebenso außerordentlich bevorzugt sind die Verbindungen der Formel I.al, insbesondere die Verbindungen der Formel I.al.1 bis I.al.228, die sich von den entsprechenden Verbindungen der Formel I.f.1 bis I.f.228 dadurch unterscheiden, daß X für 3,5-Dichlorpyrid-4-yl steht.

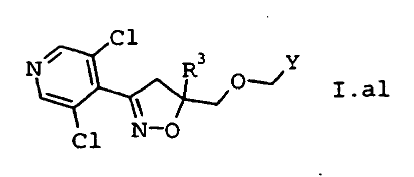

Ebenso außerordentlich bevorzugt sind die Verbindungen der Formel I.am, insbesondere die Verbindungen der Formel I.am.1 bis I.am.228, die sich von den entsprechenden Verbindungen der Formel I.f.1 bis I.f.228 dadurch unterscheiden, daß X für 2,3-Dichlorpyrid-4-yl steht.

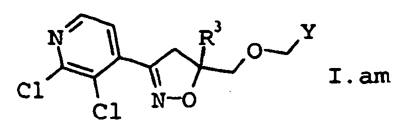

Ebenso außerordentlich bevorzugt sind die Verbindungen der Formel I.an, insbesondere die Verbindungen der Formel I.an.1 bis I.an.228, die sich von den entsprechenden Verbindungen der Formel I.f.1 bis I.f.228 dadurch unterscheiden, daß X für 3-Chlor-2-methylpyrid-4-yl steht.

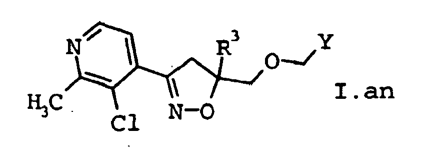

Ebenso außerordentlich bevorzugt sind die Verbindungen der Formel I.ao, insbesondere die Verbindungen der Formel I.ao.1 bis I.ao.228, die sich von den entsprechenden Verbindungen der Formel I.f.1 bis I.f.228 dadurch unterscheiden, daß X für 3,5-Dichlor-2-methylpyrid-4-yl steht.

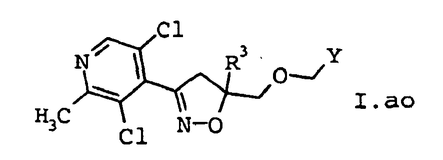

Ebenso außerordentlich bevorzugt sind die Verbindungen der Formel I.ap, insbesondere die Verbindungen der Formel I.ap.1 bis I.ap.228, die sich von den entsprechenden Verbindungen der Formel I.f.1 bis I.f.228 dadurch unterscheiden, daß X für 5-Chlor-2,3-dimethylpyrid-4-yl steht.

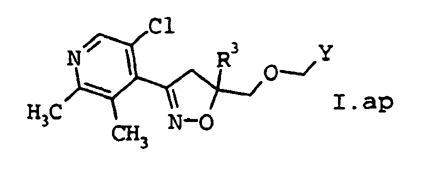

Ebenso außerordentlich bevorzugt sind die Verbindungen der Formel I.aq, insbesondere die Verbindungen der Formel I.aq.1 bis I.aq.228, die sich von den entsprechenden Verbindungen der Formel I.f.1 bis I.f.228 dadurch unterscheiden, daß X für 2-Fluorpyrazin-3-yl steht.

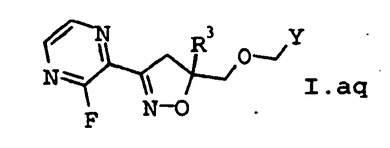

Ebenso außerordentlich bevorzugt sind die Verbindungen der Formel I.ar, insbesondere die Verbindungen der Formel-I.ar.1 bis I.ar.228, die sich von den entsprechenden Verbindungen der Formel I.f.1 bis I.f.228 dadurch unterscheiden, daß X für 2-Chlorpyrazin-3-yl steht.

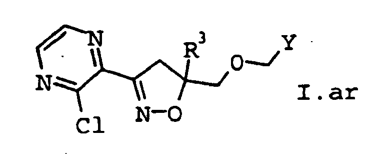

Ebenso außerordentlich bevorzugt sind die Verbindungen der Formel I.as, insbesondere die Verbindungen der Formel I.as.1 bis I.as.228, die sich von den entsprechenden Verbindungen der Formel I.f.1 bis I.f.228 dadurch unterscheiden, daß X für 2-Brompyrazin-3-yl steht.

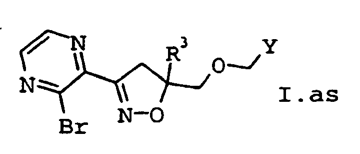

Ebenso außerordentlich bevorzugt sind die Verbindungen der Formel I.at, insbesondere die Verbindungen der Formel I.at.1 bis I.at.228, die sich von den entsprechenden Verbindungen der Formel I.f.1 bis I.f.228 dadurch unterscheiden, daß X für 2-Cyanopyrazin-3-yl steht.

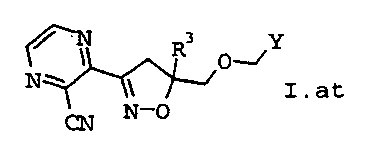

Ebenso außerordentlich bevorzugt sind die Verbindungen der Formel I.au, insbesondere die Verbindungen der Formel I.au.1 bis I.au.228, die sich von den entsprechenden Verbindungen der Formel I.f.1 bis I.f.228 dadurch unterscheiden, daß X für 2-Methylpyrazin-3-yl steht.

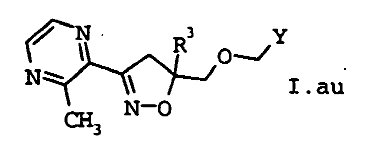

Ebenso außerordentlich bevorzugt sind die Verbindungen der Formel I.av, insbesondere die Verbindungen der Formel I.av.1 bis I.av.228, die sich von den entsprechenden Verbindungen der Formel I.f.1 bis I.f.228 dadurch unterscheiden, daß X für 2-Trifluormethylpyrazin-3-yl steht.

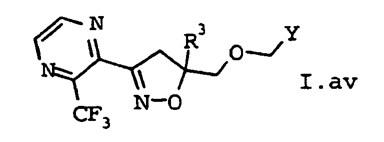

Ebenso außerordentlich bevorzugt sind die Verbindungen der Formel I.aw, insbesondere die Verbindungen der Formel I.aw.1 bis I.aw.228, die sich von den entsprechenden Verbindungen der Formel I.f.1 bis I.f.228 dadurch unterscheiden, daß X für 2-Methoxy-pyrazin-3-yl steht.

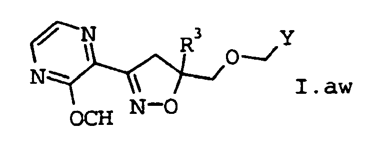

Die 3-heteroarylsubstituierten Isoxazoline der Formel I sind auf verschiedene Art und Weise erhältlich, beispielsweise nach folgenden Verfahren:

### Verfahren A

Ein Aldoxim der Formel VII wird über das entsprechende Hydroxamsäurehalogenid der Formel VI zu einem Nitriloxid der Formel V umgesetzt. Das Nitriloxid der Formel V reagiert mit einem Allylalkohol der Formel IV zu einem 5-Hydroxymethylisoxazolin der Formel III. Dieses wird anschließend mit einem Arylmethylderivat der Formel II zum 3-heteroarylsubstituierten Isoxazolin der Formel I verethert:

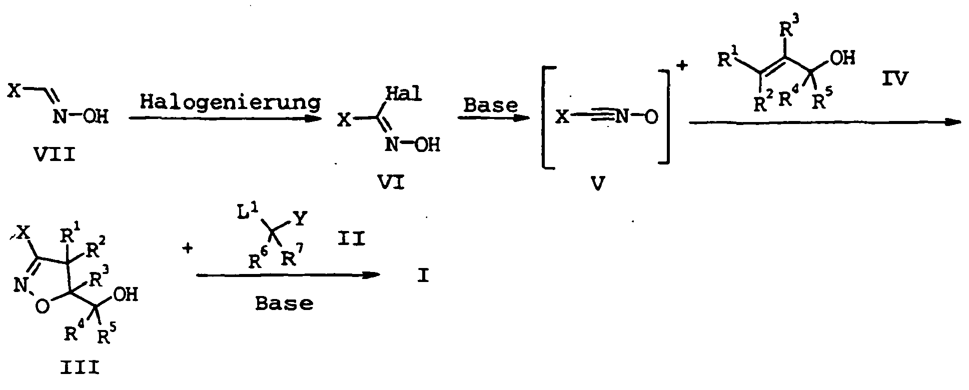

L¹ in Formel II steht für eine nucleophil verdrängbare Abgangsgruppe wie Halogen (z.B. Chlor, Brom oder Iod), Aryl-, C₁-C₆-Alkylsulfonyl, C₁-C₆-Halogenalkylsulfonyl, C₁-C₆-Alkylsulfonyloxy oder C₁-C₆-Halogenalkylsulfonyloxy, wie z.B. Toluoylsufonyl oder Trifluormethylsulfonyloxy, oder eine andere äquivalente Abgangsgruppe.

Hal in Formel VI steht für Halogen, bevorzugt Chlor oder Brom, sehr bevorzugt Brom.

Die Umsetzung des 5-Hydroxymethylisoxazolins der Formel III mit einem Arylmethylderivat der Formel II in Gegenwart einer Base zum 3-heteroarylsubstituierten Isoxazolin der Formel I erfolgt üblicherweise bei Temperaturen von 0°C bis 120°C, vorzugsweise 10°C bis 80°C, in einem inerten organischen Lösungsmittel in Gegenwart einer Base [vgl. EP-0 334 120].

Geeignete aprotische Lösungsmittel sind Ether wie Diethylether, Diisopropylether, tert.-Butylmethylether, Dioxan, Anisol und Tetrahydrofuran, sowie Dimethylsulfoxid, Dimethylformamid und Dimethylacetamid, besonders bevorzugt Diethylether, tert.-Butylmethylether, Dioxan, und Tetrahydrofuran.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetallhydride wie Lithiumhydrid, Natriumhydrid, Kaliumhydrid und Calciumhydrid, Alkalimetallamide wie Lithiumamid, Natriumamid und Kaliumamid, Alkalimetall- und Erdalkalimetallcarbonate wie Lithiumcarbonat,Natrimcarbonat, Kaliumcarbonat und Calciumcarbonat außerdem organische Basen, z.B. tertiäre Amine wie Trimethylamin, Triethylamin, Diisopropylethylamin und N-Methylpiperidin, Pyridin, substituierte Pyridine wie Collidin, Lutidin und 4-Dimethylaminopyridin sowie bicyclische Amine in Betracht.

Die Basen werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt. Es kann vorteilhaft sein, II in einem Überschuß bezogen auf III einzusetzen.

Die Aufarbeitung und Isolierung der Produkte kann in an sich bekannter Weise erfolgen.

Die für die Herstellung der 3-heteroarylsubstituierten Isoxazoline der Formel I benötigten Arylmethylderivate der Formel II sind in der Literatur bekannt [vgl. Organikum, 1979, S 213.f., 413f.] oder können gemäß der zitierten Literatur hergestellt werden.

Die für die Herstellung der 5-Hydroxymethylisoxozoline der Formel III benötigten Nitriloxide der Formel V können gemäß der Literatur in situ aus den entsprechenden Aldoximen der Formel VII über die Stufe des Hydroxamsäurehalogenids der Formel VI hergestellt werden [vgl. Houben-Weyl, Methoden der organischen Chemie, Bd. E 5, 1985, S. 1591 ff. und dort zitierte Literatur] oder sind gemäß der zitierten Literatur bekannt.

In der Regel wird das Aldoxim der Formel VII zusammen mit dem Allylalkohol der Formel IV vorgelegt, mit einem Halogenierungsmittel ggf. unter Basenzugabe entsteht das entsprechende Hydroxomsäurechlorid der Formel VI, welches durch weitere Basenzugabe zum Nitriloxid der Formel V umgesetzt wird. Das so generierte Nitriloxid der Formel V reagiert dann mit dem am Anfang mit der Reaktionsmischung vorgelegten Allylalkohol der Formel IV zu dem 5-Hydroxymethylisoxozolin der Formel III.

Des weiteren kann auch das Aldoxim der Formel VIII zunächst zum Hydroxomsäurechlorid der Formel VI halogeniert werden, anschließend werden der Allylalkohol der Formel IV und Base zugegeben. Das so generierte Nitriloxid der Formel V reagiert dann mit dem Allylalkohol der Formel IV zu dem 5-Hydroxymethylisoxozolin der Formel III.

Die Umsetzung des Nitriloxids der Formel V mit einem Allylalkohol der Formel IV erfolgt üblicherweise bei Temperaturen von -30°C bis 80°C, vorzugsweise -10°C bis 20°C, in einem inerten organischen Lösungsmittel [vgl. Houben-Weyl, Methoden der organischen Chemie, Bd. E 5, 1985, 1607 ff. und dort zitierte Literatur].

Geeignete Lösungsmittel sind aliphatische Kohlenwasserstoffe wie Pentan, Hexan, Cyclohexan und Gemische von C₅-C₈-Alkanen, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Dimethylsulfoxid, Dimethylformamid und Dimethylacetamid.

Es können auch Gemische der genannten Lösungsmittel verwendet werden.

Die Edukte werden im allgemeinen in äquimolaren Mengen miteinander umgesetzt. Es kann vorteilhaft sein, IV in einem Überschuß bezogen auf V einzusetzen.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Die entsprechenden Aldoxime der Formel VII sind in der Literatur bekannt [vgl. Houben-Weyl, Methoden der organischen Chemie, Bd. 10/4, 1968, S. 55 ff.] oder können gemäß der zitierten Literatur aus den entsprechenden Aldehyden hergestellt werden.

Die für die Herstellung der 5-Hydroxymethylisoxazoline III benötigten Allylalkohole der Formel IV sind in der Literatur bekannt [vgl. EP-514 987; Houben-Weyl, Methoden der organischen Chemie, Bd. 6/la, 1979, S. 55 ff. und darin zitierte Literatur] oder können gemäß der zitierten Literatur hergestellt werden.

### Verfahren B

Ein Nitriloxid der Formel V reagiert mit einem Allylderivat der Formel VIII zu einem Methylisoxazolinderivat der Formel IX. Dieses wird anschließend mit einem Arylmethylalkohol der Formel X zum 3-heteroarylsubstituierten Isoxazolin der Formel I verethert:

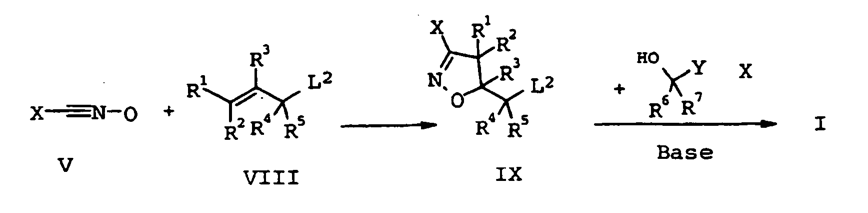

L² in den Formeln VIII und IX steht für eine nucleophil verdrängbare Abgangsgruppe wie Halogen (z.B. Chlor, Brom oder Iod), C₁-C₆-Alkylsulfonyloxy oder C₁-C₆-Halogenalkylsulfonyloxy, wie z.B. Trifluormethylsulfonyloxy, oder eine andere äquivalente Abgangsgruppe.

Die Umsetzung des Methylisozolinderivats der Formel IX mit einem Arylmethylalkohol der Formel X erfolgt analog der Umsetzung des 5-hydroxymethylisoxozolins der Formel III mit einem Arylmethylderivat der Formel II in Verfahren A.

Die für die Herstellung der 3-heteroarylsubstituierten Isoxozoline der Formel I benötigen Arylmethylalkohole der Formel X sind dem Fachmann bekannt oder können durch literaturbekannte Reaktionen hergestellt werden.

Die Herstellung der Methylisoxozolinderivate der Formel IX aus Nitriloxiden der Formel V und Allylderivaten der Formel VIII erfolgt analog zur Herstellung der 5-Hydroxymethylisoxozoline der Formel III in Verfahren A.

Des Weiteren besteht die Möglichkeit, die Methylisoxazolinderivate der Formel IX aus den 5-Hydroxymethylisoxozolinen der Formel III herzustellen:

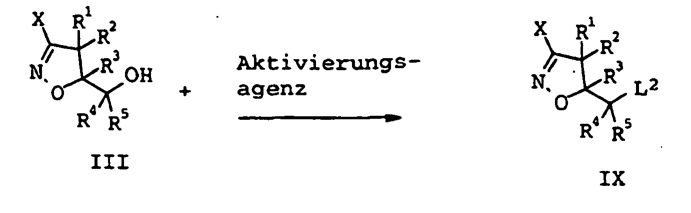

Die Umsetzung der -Hydroxymethylisoxazoline zu Methylisoxazolinderivaten der Formel IX erfolgt üblicherweise bei Temperaturen von -20°C bis zur Rückflußtemperatur des entsprechenden Reaktionsgemischen in einem inerten Lösungsmittel.

Geeignete Lösungsmittel sind aliphatische Kohlenwasserstoffe wie Penton, Hexon, Cyclohexon und Gemische von C₅-C₈-Alkonen, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxon, Anisol und Tetrahydrofuran. Es können auch Gemische der genannten Lösungsmittel verwendet werden.

Als Aktivierungsagentien eignen sich z. B. Halogenierungsmittel wie Thionylchorid, Phosgen, Diphosgen, Triphosgen, Oxalylchlorid, Thionylbromid, Oxalylbromid, oder z. B. Alkylsulfonsäurechloride, wie z. B. Methylsulfonsäurechlorid, oder Halogenalkylsulfonsäurechloride, wie z.B. Trifluormethylsulfonsäurechlorid.

Die Edukte werden im allgemeinen in äquimolaren Mengen miteinander umgesetzt. Es kann vorteilhaft sein, das Aktivierungsagenz im Überschuß einzusetzen.

Die Allylderivate der Formel VIII sind dem Fachmann bekannt oder können z. B. aus den Allylalkoholen der Formel IV analog der Synthese von III zu IX hergestellt werden.

### Verfahren C

Ein Allylalkohol der Formel IV wird mit einem Arylmethylderivat der Formel II zu einem Arylmethylallylether der Formel XI umgesetzt. Dieser reagiert anschließend mit einem Nitriloxid der Formel V zu den 3-heteroarylsubstituierten Isoxazolinen der Formel I:

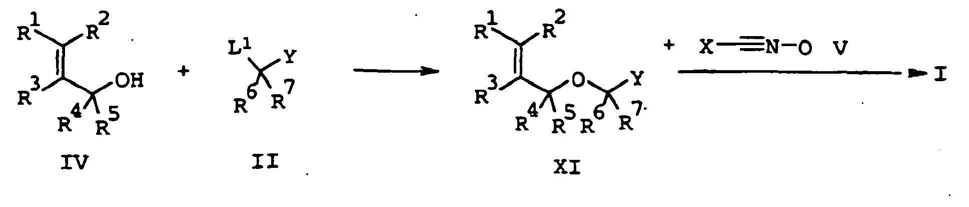

**L¹** in Formel II steht für eine nucleophil verdrängbare Abgangsgruppe wie Halogen (z.B. Chlor, Brom oder Iod), Aryl-, C₁-C₆-Alkylsulfonyl, Arylsulfonyl (z.B. Toluolylsulfonyl), C₁-C₆-Halogenalkylsulfonyl, C₁-C₆-Alkylsulfonyloxy oder C₁-C₆-Halogenalkylsulfonyloxy wie (z.B. Trifluormethylsulfonyloxy), oder eine andere äqivatente Abgangsgruppe.

Die Umsetzung der Arylmethylallylether der Formel XI mit einem Nitriloxid der Formel V zu den 3-heteroarylsubstituierten Isoxazolinen der Formel I erfolgt analog zur Umsetzung des Nitriloxids der Formel V mit einem Allylalkohol der Formel IV in Verfahren A.

Die Edukte werden im allgemeinen in äquimolaren Mengen miteinander umgesetzt. Es kann vorteilhaft sein, XI in einem Überschuß bezogen auf V einzusetzen.

Die Aufarbeitung und Isolierung der Produkte kann in an sich bekannter Weise erfolgen.

Die für die Herstellung der 3-heteroarylsubstituierten Isoxazoline der Formel I benötigten Nitriloxide der Formel V sind analog Verfahren A erhältlich.

Die Umsetzung des Allylalkohols der Formel IV mit einem Arylmethylderivat der Formel II erfolgt analog zur Umsetzung des 5-Hydroxymethylisoxazolins der Formel III mit einem Arylmethylderivat der Formel II in Verfahren A.

Die Edukte werden im allgemeinen in äquimolaren Mengen miteinander umgesetzt. Es kann vorteilhaft sein, II in einem Überschuß bezogen auf IV einzusetzen.

Die Aufarbeitung und Isolierung der Produkte kann in an sich bekannter Weise erfolgen.

Die für die Herstellung der Arylmethylallylether der Formel XI benötigten Arylmethylderivate der Formel II und Allylalkohole der Formel IV sind gemäß Verfahren A erhältlich.

Des Weiteren besteht die Möglichkeit, den Arylmethylallylether der Formel XI durch Umsetzung des entsprechenden Allylderivats der Formel VIII mit einem Arylmethylalkohol der Formel X (vgl. Verfahren B) herzustellen.

Die Umsetzung erfolgt analog zur Umsetzung des Allylalkohols der Formel IV mit einme Arylmethylderivat der Formel II bzw. analog zur Umsetzung des 5-Hydroxymethylisoxazolins der Formel III mit einem Arylmethylderivat der Formel II in Verfahren A.

Die benötigten Allylderivate der Formel VIII und Arylmethylalkohole der Formel X sind gemäß Verfahren B erhältlich.

### 5-Hydroxymethylisoxazoline der Formel III

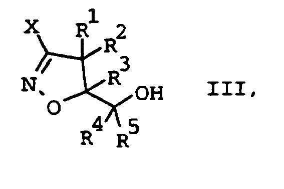

wobei X, R¹, R², R³, R⁴ und R⁵ die unter Anspruch 1 genannten Bedeutungen haben, sind ebenfalls ein Gegenstand der vorliegenden Erfindung.

Die besonders bevorzugten Ausführungsformen der Zwischenprodukte in Bezug auf die Variablen entsprechen denen der Reste X, R¹, R², R³, R⁴ und R⁵ der Formel I.

Besonders bevorzugt werden 5-Hydroxymethylisoxazoline der Formel III, in der
- X: 6-gliedriges Heteroaryl mit ein bis drei Stickstoffatomen;
bevorzugt 6-gliedriges Heteroaryl mit einem bis zwei Stickstoffatomen;
insbesondere Pyridyl, Pyridmidyl und Pyrazinyl;
sehr bevorzugt Pyridyl und Pyrimidyl;
ebenso bevorzugt Pyridyl und Pyrozinyl;
wobei das 6-gliedrige Heteroaryl jeweils wie oben beschrieben substituiert ist; und
- R¹-R⁵: Wasserstoff, C₁-C₄-Alkyl, wie z. B. Methyl oder Ethyl, oder C₁-C₄-Haloenalkyl, wie z. B. Difluormethyl, Trifluormethyl oder Trichlormethyl;
besonders bevorzugt Wasserstoff, Methyl oder Trifluormethyl;
sehr bevorzugt Wasserstoff;
insbesondere bevorzugt stehen R¹, R², R⁴-R⁵ für Wasserstoff, Methyl oder Trifluormethyl und R³ für Wasserstoff, Methyl, Ethyl oder Trifluormethyl;
insbesondere sehr bevorzugt stehen R¹, R², R⁴-R⁵ für Wasserstoff und R³ für Methyl;
bedeuten.

### Methylisoxazoline der Formel IX

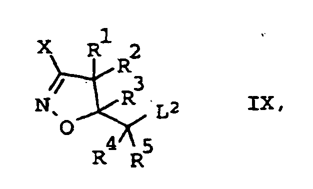

wobei X und R¹-R⁵ die unter Anspruch 1 genannten Bedeutungen haben und L² für eine nucleophil verdrängbare Abgangsgruppe wie Halogen (z.B. Chlor, Brom und Iod), C₁-C₆-Alkylsulfonyloxy oder C₁-C₆-Halogenalkylsulfonyloxy (z.B. Trifluormethylsulfonyloxy) , oder eine andere äquvivalente Abgangsgruppe steht, sind ebenfalls Gegenstand der vorliegenden Erfindung.

Die besonders bevorzugten Ausführungsformen der Zwischenprodukte in Bezug auf die Variablen entprechen denen der Reste X und R¹-R⁵ der Formel I.

Besonders bevorzugt werden Methylisoxazoline der Formel IX, in der
- X: 6-gliedriges Heteroaryl mit ein bis drei Stickstoffatomen;
insbesondere 6-gliedriges Heteroaryl mit einem bis zwei Stickstoffatomen;
sehr bevorzugt Pyridyl;
wobei das 6-gliedrige Heteroaryl wie oben beschrieben substituiert ist; und
- R¹-R⁵: Wasserstoff, C₁-C₄-Alkyl, wie z. B. Methyl oder Ethyl, oder C₁-C₄-Halogenalkyl, wie z. B. Difluormethyl, Trifluormethyl oder Trichlormethyl;
besonders bevorzugt Wasserstoff, Methyl oder Trifluormethyl;
sehr bevorzugt Wasserstoff;
insbesondere bevorzugt stehen R¹, R², R⁴-R⁵ für Wasserstoff, Methyl oder Trifluormethyl und R³ für Wasserstoff, Methyl, Ethyl oder Trifluormethyl;
insbesondere sehr bevorzugt stehen R¹, R², R⁴-R⁵ für Wasserstoff und R³ für Methyl; und
- L²: eine nucleophil verdrängbare Abgangsgruppe;
bevorzugt Halogen, C₁-C₆-Alkylsulfonyloxy oder C₁-C₆-Halogenalkylsulfonyloxy;
besonders bevorzugt Chlor, Brom, Iod oder Trifluormethylsulfonyloxy;
bedeuten.

### 3-(2-Chlorpyrid-3-yl)-4,5-dihydro-5-hydroxymethyl-5-methylisoxazol

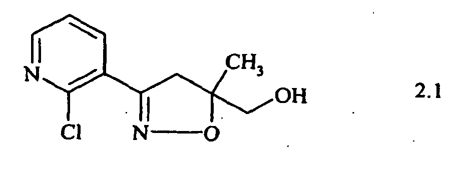

5.0 g (0.03 mol) 2-Chlorpyrid-3-yl-carboxaldehydoxim wurden in Dimethylformamid vorgelegt, mit einer Spatelspitze N-Chlorsuccinimid versetzt und auf 40-45°C erwärmt. Anschließend wurden 4.26 g (0.03 mol) N-Chlorsuccinimid portionsweise zugegeben, so dass die Reaktionstemperatur 50°C nicht überstieg. Nach 1 h Rühren bei 45-50°C wurde der Ansatz hydrolysiert, mit Diethylether extrahiert und die organische Phase getrocknet. Zu der erhaltenen Lösung wurden 2.07 g (0.03 mol) 2-Methyl-2-propenol gegeben und unter Eiskühlung 5.37 g (0.04 mol) Triethylamin zugetropft. Nach 1 h ließ man die Reaktionslösung auf Raumtemperatur erwärmen, filtrierte den Niederschlag ab und entfernte das Lösungsmittel. Man erhielt 2.8 g (38.7 % der Theorie) der Titelverbindung (Schmp. 71°C).

In den Tabellen 2 bis 5 sind neben der voranstehenden Verbindung noch weitere 5-Hydroxymethylisoxazoline der Formel III und 3-heteroarylsubstituierte Isoxazoline der Formel I aufgeführt, die in analoger Weise nach den voranstehend beschrieben Verfahren hergestellt wurden oder herstellbar sind. 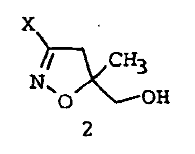 (=III mit R¹, R², R⁴, R⁵ = Wasserstoff, R³ = Methyl)

**Tabelle 2**

| Nr. | X | ausgewählte ¹H-NMR-Daten [DMSO] bzw. Schmp. |
|---|---|---|
| 2.1 | 2-Chlorpyrid-3-yl | 71°C |
| 2.2 | 6-Methylpyrid-2-yl | |

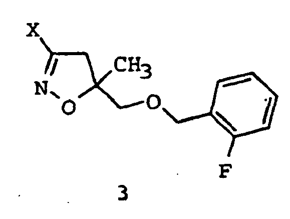

(= I mit R¹, R², R⁴-R⁷ = Wasserstoff,
R³ = Methyl,
Y = 2-Fluorphenyl)

**Tabelle 3**

| Nr. | X | ausgewählte ¹H-NMR-Daten [cm⁻¹]DMSO] bzw. Schmp.bzw. MS [^{m}/e] bzw. ausgewählte IR-Daten [cm⁻¹] |
|---|---|---|
| 3.1 | 2-Chlorpyrid-3-yl | 3.31 (dd, 2H), 3.59 (dd, 2H) |
| 3.2 | 6-Methylpyrid-2-yl | 3.27 (dd, 2H), 3.53 (dd, 2H) |
| 3.3 | 4-Chlorpyrid-3-yl | 3.35 (dd, 2H), 3.59 (dd, 2H) |
| 3.4 | 2-Chlorid-3-yl-N-oxid | IR [cm⁻¹] 1259 N-Oxid |
| 3.5 | 2,4-Dichlorpyrid-3-yl | 3.15 (dd, 2H), 3.60 (dd, 2H) |
| 3.6 | 2,6-Dichlorpyrid-3-yl | 3.32 (dd, 2H), 3.56 (dd, 2H) |
| 3.7 | 2-Brompyrid-3-yl | 3.28 (dd, 2H), 3.58 (dd, 2H) |
| 3.8 | 2-Methylpyrid-3-yl | 3.28 (dd, 2H), 3.56 (dd, 2H) |
| 3.9 | 2-Trifluormethylpyrid-3-yl | 3.26 (dd, 2H), 3.58 (dd, 2H) |
| 3.10 | 4-Trifluormethylpyrid-3-yl | 3.3 (dd, 2H), 3.55 (dd, 2H) |
| 3.11 | 2-Methoxypyridin-3-yl | 3.27 (dd, 2H), 3.54 (dd, 2H) |
| 3.12 | 6-Methoxypyridin-3-yl | 3.25 (dd, 2H), 3.53 (dd, 2H) |
| 3.13 | 2-Chlor-4-Isopropylpyrid-3-yl | 3.3 (dd, 2H), 3.55 (dd, 2H) |
| 3.14 | 2-Chlor-6-methylpyrid-3-yl | 3.35 (dd, 2H), 3.54 (dd, 2H) |
| 3.15 | 2-Chlor-4,6-dimethylpyrid-3-yl | 3.12 (dd, 2H), 3.57 (dd, 2H) |
| 3.16 | 2,6-Dichlor-4-methylpyridin-3-yl | 3.18 (dd, 2H), 3.59 (dd, 2H) |
| 3.17 | 3-Chlorpyrid-4-yl | 3.32 (dd, 2H), 3.62 (dd, 2H) |
| 3.18 | 3-Brompyrid-4-yl | 3.38 (dd, 2H), 3.58 (dd, 2H) |
| 3.19 | 2-Methylpyrid-4-yl | 3.24 (dd, 2H), 3.58 (dd, 2H) |
| 3.20 | 3-Trifluormethylpyrid-4-yl | 3.25 (dd, 2H), 3.57 (dd, 2H) |
| 3.21 | 2-Trifluormethyl-5-chlorpyrid-4-yl | 3.43 (dd, 2H), 3.59 (dd, 2H) |
| 3.22 | 2-Cyclopropylpyrimidin-4-yl | 3.25 (dd, 2H), 3.58 (dd, 2H) |
| 3.23 | 2-Phenyl-4-methylpyrimidin-6-yl | 3.35 (dd, 2H), 3.60 (dd, 2H) |
| 3.24 | 3-Chlorpyrazin-2-yl | 3.4 (dd, 2H), 3.68 (dd, 2H) |
| 3.25 | 1,4-Dimethyl-2-Chlor-imidazol-2-yl | 3.31 (dd, 2H), 3.57 (dd, 2H) |
| 3.26 | "2-Chlorpyrid-3-yl" Methylsulfonsäuresalz | 1492, 1456, 1351, 1194, 1058, 785, 772, 761, 536 |

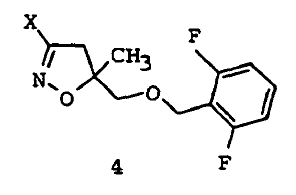

(=I mit R¹, R², R⁴-R⁷ = Wasserstoff,
R³ = Methyl,
Y = 2,6-Difluorphenyl)

**Tabelle 4**

| Nr. | X | ausgewählte ¹H-NMR-Daten [DMSO] bzw. Schmp.bzw. MS [m/e] |
|---|---|---|
| 4.1 | 2-Chlorpyrid-3-yl | 3.33 (dd, 2H), 3.53 (dd, 2H) |
| 4.2 | 6-Methylpyrid-2-yl | 70°C |
| 4. 3 | 3-Chlorpyrid-2-yl | Schmp. 102°C |
| 4. 4 | 3-Methylpyrid-2-yl | 3.34 (dd, 2H), 3.55 (dd, 2H) |
| 4. 5 | Trifluormethylpyrid-2-yl | Schmp. 95-96°C |
| 4. 6 | 2-Fluorpyrid-3-yl | 3.26 (dd, 2H), 3.53 (dd, 2H) |
| 4. 7 | 2-Fluorpyrid-3-yl | 3.32 (dd, 2H), 3.56 (dd, 2H) |
| 4. 8 | 2-Brompyrid-3-yl | 3.22 (dd, 2H), 3.55 (dd, 2H) |
| 4. 9 | 2,4-Dichlorpyrid-3-yl | 3.09 (dd, 2H), 3.59 (dd, 2H) |
| 4. 10 | 2,4-Dichlorpyrid-3-yl | 3.28 (dd, 2H), 3.56 (dd, 2H) |
| 4. 11 | 2-Chlorpyrid-3-yl-N-oxid | [M]⁺ 368 |
| 4. 12 | 2-Cyanopyrid-3-yl | 3.48 (dd, 2H), 3.61 (dd, 2H) |
| 4. 13 | 2-Hydroxypyrid-3-yl | 3.38 (dd, 2H), 3.52 (dd, 2H) |
| 4. 14 | 2-Nitropyrid-3-yl | 3.08 (dd, 2H), 3.60 (dd, 2H) |
| 4. 15 | 2-Methylpyrid-3-yl | Schmp. 85°C |
| 4. 16 | 4-Methylpyrid-3-yl | 3.30 (dd, 2H), 3.57 (dd, 2H) |
| 4. 17 | 2-Ethylpyrid-3-yl | 3.20 (dd, 2H), 3.58 (dd, 2H) |
| 4. 18 | 2-Trifluormethylpyrid-3-yl | 3.20 (dd, 2H), 3.51 (dd, 2H) |
| 4. 19 | 4-Trifluormethylpyrid-3-yl | 3.23 (dd, 2H), 3.53 (dd, 2H) |
| 4. 20 | 2-Methoxypyrid-3-yl | 3.24 (dd, 2H), 3.52 (dd, 2H) |
| 4. 21 | 2(Methylthio)pyrid-3-yl | Schmp. 97-98°C |
| 4. 22 | 2(Methylsulfonyl)pyrid-3-yl | IR: 1312 cm⁻¹ (SO₂) |
| 4. 23 | 2(Methylsulfonix)pyrid-3-yl | ¹³C-NMR: 87.27, 87.20 |
| 4. 24 | 2(Dimethylamino)pyrid-3-yl | [M+H]⁺ 362 |
| 4. 25 | 2(Phenylthio)pyrid-3-yl | 3.31 (dd, 2H), 3.58 (dd, 2H) |
| 4. 26 | 2-Chlor-6-methylpyrid-3-yl | 3.3 (dd, 2H), 3.55 (dd, 2H) |
| 4. 27 | 2-Chlor-4-isopropylpyrid-3-yl | 3.26 (dd, 2H), 3.54 (dd, 2H) |
| 4. 28 | 2-Chlor-4,6-dimethylpyrid-3-yl | 3.11 (dd, 2H), 3.58 (dd, 2H) |
| 4. 29 | 2-Methyl-4-chlorpyrid | 3.1 (dd, 2H), 3.54 (dd, 2H) |
| 4. 30 | 3-Chlorpyrid-4-yl | 3.34 (dd, 2H), 3.56 (dd, 2H) |
| 4. 31 | 3-Brompyrid-4-yl | 3.3 (dd, 2H), 3.55 (dd, 2H) |
| 4. 32 | 3-Trifluormethylpyrid-4-yl | 3.25 (dd, 2H), 3.55 (dd, 2H) |
| 4. 33 | 2-Trifluormethyl-5-chlorpyrid-4-yl | Schmp. 60°C |
| 4. 34 | 2-Methylpyrimidin-4-yl | Schmp. 89°C |
| 4. 35 | 2-Cyclopropylpyrimidin-4-yl | 3.26 (dd, 2H), 3.58 (dd, 2H) |
| 4.-36 | 3-Chlorpyrazin-2-yl | 3.3 (dd, 2H), 3.58 (dd, 2H) |
| 4.37 | 2-Ethenylpyrid-3-yl | 3.3 (dd, 2H), 3.54 (dd, 2H) |
| 4.38 | 2-Acetylpyrid-3-yl | [M+H]⁺ 361 |

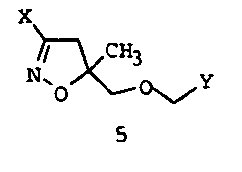

(I mit R¹, R², R⁴-R⁷ = Wasserstoff,
R³ = Methyl)

**Tabelle 5**

| Nr. | X | Y | ausgewählte ¹H-NMR-Daten bzw. MS |
|---|---|---|---|
| 5.1 | 2-Chlorpyrid-3-yl | Phenyl | 3.4 (dd, 2H), 3.56 (dd, 2H) |
| 5.2 | 2-Chlorpyrid-3-yl | 3-Fluorphenyl | 335 [M+H]⁺ |
| 5.3 | 2-Chlorpyrid-3-yl | 2-Chlorphenyl | 351 [M+H]⁺ |
| 5.4 | 2-Chlorpyrid-3-yl | 2,6-Dichlorphenyl | 385 [M+H]⁺ |
| 5.5 | 2-Chlorpyrid-3-yl | 2,3,6-Trichlorphenyl | 419 [M+H]⁺ |
| 5.6 | 2-Chlorpyrid-3-yl | 2-Chlor-6-fluorphenyl | 3.28 (dd, 2H), 3.56 (dd, 2H) |
| 5.7 | 2-Chlorpyrid-3-yl | 4-Methylphenyl | 331 [M+H]⁺ |
| 5.8 | 2-Chlorpyrid-3-yl | 2-Methylnophthyl-1-yl | 381 [M+H]⁺ |
| 5.9 | 2-Chlorpyrid-3-yl | 5-Chlor-1-methyl-3-trifluormethylpyrazol-4-yl | 423 [M+H]⁺ |

### Anwendung

Die 3-heteroarylsubstituierten Isoxazoline der Formel I und deren landwirtschaftlich brauchbaren Salze eignen sich - sowohl als Isomerengemische als auch in Form der reinen Isomeren - als Herbizide. Die Verbindungen der Formel I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen der Formel I bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa , Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus können die Verbindungen der Formel I auch in Kulturen, die durch Züchtung einschließlich gentechnischer Methoden gegen die Wirkung von Herbiziden tolerant sind, verwandt werden.

Die Verbindungen der Formel I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die herbiziden Mittel enthalten eine herbizid wirksame Menge mindestens einer Verbindung der Formel I oder eines landwirtschaftlich brauchbaren Salzes von I und für die Formulierung von Pflanzenschutzmitteln übliche Hilfsmittel.

Als inerte Hilfsstoffe kommen im Wesentlichen in Betracht: Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe (Adjuvantien) kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Verbindungen der Formel I in den anwendungsfertigen Zubereitungen können in weiten Bereichen variiert werden. Im allgemeinen enthalten die Formulierungen etwa von 0,001 bis 98 Gew.-%, vorzugsweise 0,01 bis 95 Gew.-%, mindestens eines Wirkstoffs. Die Wirkstoffe werden dabei in einer Reinheit von 90% bis 100%, vorzugsweise 95% bis 100% (nach NMR-Spektrum) eingesetzt.

Die folgenden Formulierungsbeispiele verdeutlichen die Herstellung solcher Zubereitungen:
I. 20 Gewichtsteile eines Wirkstoffs der Formel I werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Rizinusöl besteht. Durch Ausgießen und feines Verteilen der Lösung in 100000 Gewichtsteilen Wasser erhält man eine wäßrige Dispersion, die 0,02 Gew.-% des Wirkstoffs der Formel I enthält.
II. 20 Gewichtsteile eines Wirkstoffs der Formel I werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Rizinusöl besteht. Durch Eingießen und feines Verteilen der Lösung in 100000 Gewichtsteilen Wasser erhält man eine wäßrige Dispersion, die 0,02 Gew.-% des Wirkstoffs der Formel I enthält.
III. 20 Gewichtsteile eines Wirkstoffs der Formel I werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.-% des Wirkstoffs der Formel I enthält.
IV. 20 Gewichtsteile eines Wirkstoffs der Formel I werden mit 3 Gewichtsteilen des Natriumsalzes der Diisobutylnaphthalinsulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20000 Gewichtsteilen Wasser erhält man eine Spritzbrühe, die 0,1 Gew.-% des Wirkstoffs der Formel I enthält.
V. 3 Gewichtsteile eines Wirkstoffs der Formel I werden mit 97 Gewichtsteilen feinteiligem Kaolin vermischt. Man erhält auf diese Weise ein Stäubemittel, das 3 Gew.-% des Wirkstoffs der Formel I enthält.
VI. 20 Gewichtsteile eines Wirkstoffs der Formel I werden mit 2 Gewichtsteilen Calciumsalz der Dodecylbenzolsulfonsäure, 8 Gewichtsteilen Fettalkoholpolyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII. 1 Gewichtsteil eines Wirkstoffs der Formel I wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
VIII. 1 Gewichtsteil eines Wirkstoffs der Formel I wird in einer Mischung gelöst, die aus 80 Gewichtsteilen Cyclohexanon und 20 Gewichtsteilen Wettol^{R} EM 31 (= nichtionischer Emulgator auf der Basis von ethoxyliertem Rizinusöl) besteht. Man erhält ein stabiles Emulsionskonzentrat.

Die Applikation der Verbindungen der Formel I bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Verbindung der Formel I betragen je nach Bekämpfungsziel, Jahreszeit, Zielpflanzen und Wachstumsstadium 0,001 bis 3,0, vorzugsweise 0,01 bis 1,0 kg/ha aktive Substanz (a.S.).

Zur Verbreiterung des Wirkungsspektrums und zur Erzielung synergistischer Effekte können die 3 heteroarylsubstituierten Isoxazoline der Formel I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-(Hetaroyl/Aroyl)-1,3- cyclohexandione, Heteroaryl-Aryl-Ketone, Benzylisoxazolidinone, meta-CF₃-Phenylderivate, Carbamate, Chinolincarbonsäure und deren Derivate, Chloracetanilide, Cyclohexenonoximetherderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht.

Außerdem kann es von Nutzen sein, die Verbindungen der Formel I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Anwendungsbeispiele

Die herbizide Wirkung der 3-heteroarylsubstituierten Isoxazoline der Formel I ließ sich durch die folgenden Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.. Die Aufwandmenge für die Vorauflaufbehandlung betrug 3,0 kg/ha a.S. (aktive Substanz).

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 3,0 kg/ha a.S. (aktive Substanz).

Die Pflanzen wurden artenspezifisch bei Temperaturen von 10 bis 25°C bzw. 20 bis 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Abutilon theophrasti | Chinesischer Hanf | velvet leaf |
| Avena fatua | Flughafer | wild oat |
| Lolium multiflorum | Italienisches Raygras | italian ryegress |
| Setaria italica | Italienische Borstenhirse | foxtail millet |

Bei Aufwandmengen von 3,0 kg/ha zeigten die Verbindungen 3. 8, 3 . 9', 3.19, 3. 26, 4. 21 und 4. 30 im Vorauflauf eine sehr gute wirkung gegen die Schadpflanzen Solium multiforum und Setaria italica.

Die Wirkung von Verbindung 4.1 im Vorauflauf bei Aufwandmengen von 3.0 Kg/ha auf die Schadpflanzen Lolium multiflorum und Setaria italica ist sehr gut.

Bei Aufwandmengen von 3,0 kg/ha zeigte die Verbindung 4.1 im Nachauflauf eine sehr gute Wirkung gegen die unerwünschten Pflanzen Abutilon theophrasti, Avena fatua und Setaria italica.

## Patentansprüche

1. 3-Heteroarylsubstituierte Isoxazoline der Formel I 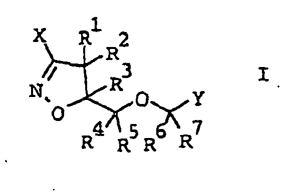 in der die Variablen die folgenden Bedeutungen haben:
X 6-gliedriges Heteroaryl mit ein bis vier Stickstoffatomen, das partiell oder vollständig halogeniert ist und/oder ein bis drei Substituenten aus folgender Gruppe trägt:
Cyano, Nitro, Hydroxy, C₁-C₈-Alkyl, C₃-C₆-Cycloalkyl, C₃-C₆-Halogencycloalkyl, C₂-C₈-Alkenyl, C₂-C₈-Alkinyl, C₁-C₈-Halogenalkyl, C₂-C₆-Halogenalkenyl, C₂-C₆-Halogenalkinyl, C₁-C₈-Cyanoalkyl, C₂-C₈-cyanoalkenyl, C₁-C₈-Alkoxy, C₁-C₈-Halogenalkoxy, C₁-C₈-Alkoxy-C₁-C₈-alkyl, C₁-C₈-Alkoxy-C₁-C₈-alkoxy, Amino, C₁-C₈-Alkylamino, Di(C₁-C₈-alkyl)amino, C₁-C₈-Alkylcarbonylamino, N-C₁-C₈-Alkylcarbonyl-N-C₁-C₈-alkylamino, C₁-C₈-Alkoxycarbonylamino, N-C₁-C₈-Alkoxycarbonyl-N-C₁-C₈-alkylamino, Amino-C₁-C₈-alkyl, C₁-C₈-Alkylamino-C₁-C₈-alkyl, Di(C₁-C₈-alkyl)amino-C₁-C₈-alkyl, C₁-C₈-Alkylthio, C₁-C₈-Halogenalkylthio, C₁-C₈-Alkylthio-C₁-C₈-alkyl, C₁-C₈-alkylsulfinyl, C₁-C₈-Halogenalkylsulfinyl, C₁-C₈-Alkylsulfonyl, C₁-C₈-Halogenalkylsulfonyl, C₁-C₈-Alkylsulfonyl-C₁-C₈-alkyl, C₁-C₈-Alkylcarbonyl, C₁-C₈-Alkoxycarbonyl, C₁-C₈-Alkoxycarbonyl-C₁-C₈-alkyl, Aminocarbonyl, C₁-C₈-Alkylaminocarbonyl, Di(C₁-C₈-alkyl)aminocarbonyl, Aminocarbonyl-C₁-C₈alkyl, C₁-C₈-Alkylaminocarbonyl-C₁-C₈-alkyl, Di(C₁-C₈-alkyl)aminocarbonyl-C₁-C₈-alkyl, Carboxyl, Carboxy-C₁-C₈-alkyl, Phenyl, Phenoxy, Phenylthio, Phenylamino, Phenyl-C₁-C₈-alkyl, Phenyl-C₁-C₈-alkoxy, 3- bis 6-gliedriges Heterocyclyl und 5- oder 6-gliedriges Heteroaryl, wobei die acht letztgenannten Reste ihrerseits partiell oder vollständig halogeniert sein können und/oder ein bis drei Substituenten aus der Gruppe Nitro, Cyano, C₁-C₈-Alkyl, C₁-C₈-Alkoxy, C₁-C₈-Halogenalkyl, C₁-C₈-Halogenalkoxy, C₁-C₈-Alkylthio, C₁-C₈-Halogenalkylthio, C₁-C₈-Alkylsulfinyl, C₁-C₈-Halogenalkylsulfinyl, C₁-C₈-Alkylsulfonyl und C₁-C₈-Halogenalkylsulfonyl tragen können; _
R¹ - R⁷ Wasserstoff, C₁-C₆-Alkyl oder C₁-C₆-Halogenalkyl;
Y Aryl, das partiell oder vollständig halogeniert sein kann und/oder ein bis drei Substituenten aus der Gruppe Cyano, Nitro, C₁-C₆-Alkyl, C₂-C₆-Alkenyl, C₂-C₆-Alkinyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkoxycarbonyl, C₁-C₆-Alkoxycarbonyl-C₁-C₄-alkoxy, C₁-C₆-Alkylsulfonyl, Phenyl, Phenoxy und Phenylcarbonyl, Benzo[1,4]dioxonyl, Benzo[1,3]dioxolanyl, 2,3-Dihydrobenzofuranyl oder Benzimidazol; oder
5 gliedriges Heteroaryl mit ein bis vier Stickstoffatomen, oder mit ein bis drei Stickstoffatomen und einem Sauerstoff- oder Schwefelatom, oder mit einem Sauerstoffoder Schwefelatom; oder
6-gliedriges Heteroaryl mit ein bis vier Stickstoffatomen;
wobei die Heterocyclen partiell oder vollständig halogeniert sein können und/oder ein bis drei Substituenten aus der Gruppe Cyano, C₁-C₆-Alkyl, C₁-C₆-Halogenalkyl, C₁-C₆-Alkoxy-C₁-C₄-alkyl, C₁-C₆-Alkoxycarbonyl und C₁-C₆-Alkylsulfonyl tragen können;
sowie deren landwirtschaftlich brauchbaren Salze.

2. 3-Heteroarylsubstituierte Isoxazoline der Formel I gemäß Anspruch 1, in der
X 6-gliedriges Heterorayl mit ein bis zwei Stickstoffatomen, welches partiell oder vollständig halogeniert ist und/oder ein bis zwei Substituenten in ortho-Position zu einem Stickstoffatom aus folgender Gruppe trägt: Cyano, Nitro, Hydroxy, C₁-C₆-Alkyl, C₁-C₆-Halogenalkyl , C₂-C₆-Alkenyl, C₂-C₆-Alkinyl, C₁-C₆-Alkoxy, C₁-C₆-Halogenalkoxy, Amino, C₁-C₆-Alkylamino, Di(C₁-C₆-alkyl)amino, C₁-C₆-Alkylthio und C₁-C₆-Alkylsulfinyl; und
welches ortho oder meta zu einem Stickstoff des 6-gliedrigen Heteroaryls mit dem Isoazolingerüst verknüpft ist,
bedeutet.

3. 3-Heteroarylsubstituierte Isoxazoline der Formel I gemäß Anspruch 1, in der
X Pyridyl, welches partiell oder vollständig halogeniert ist und/oder ein bis drei Substituenten aus folgender Gruppe trägt: Cyano, Nitro, Hydroxy, C₁-C₈-Alkyl, C₃-C₆-Cycloalkyl, C₃-C₆-Halogencycloalkyl, C₂-C₈-Alkenyl, C₂-C₈-Alkinyl, C₁-C₈-Halogenalkyl, C₂-C₆-Halogenalkenyl, C₂-C₆-Halogenalkinyl, C₁-C₈-Cyanoalkyl, C₂-C₈-Cyanoalkenyl, C₁-C₈-Alkoxy, C₁-C₈-Halogenalkoxy, C₁-C₈-Alkoxy- C₁-C₈-alkyl, C₁-C₈-Alkoxy-C₁-C₈-alkoxy, Amino, C₁-C₈-Alkylamino, Di(C₁-C₈-alkyl)amino, C₁-C₈-Alkylcarbonylamino, N-C₁-C₈-Alkylcarbonyl-N-C₁-C₈-alkylamino, C₁-C₈-Alkoxycarbonylamino, N-C₁-C₈-Alkoxycarbonyl-N-C₁-C₈-alkylamino, Amino-C₁-C₈-alkyl, C₁-C₈-Alkylamino-C₁-C₈-alkyl, Di(C₁-C₈-alkyl)amino-C₁-C₈-alkyl, C₁-C₈-Alkylthio, C₁-C₈-Halogenalkylthio, C₁-C₈-Alkylthio-C₁-C₈-alkyl, C₁-C₈-Alkylsulfinyl, C₁-C₈-Halogenalkylsulfinyl, C₁-C₈-Alkylsulfonyl, C₁-C₈-Halogenalkylsulfonyl, C₁-C₈-Alkylsulfonyl-C₁-C₈-alkyl, C₁-C₈-Alkylcarbonyl, C₁-C₈-Alkoxycarbonyl, C₁-C₈-Alkoxycarbonyl-C₁-C₈-alkyl, Aminocarbonyl, C₁-C₈-Alkylaminocarbonyl, Di(C₁-C₈-alkyl)aminocarbonyl, Aminocarbonyl-C₁-C₈-alkyl, C₁-C₈-Alkylaminocarbonyl-C₁-C₈-alkyl, Di(C₁-C₈-alkyl)aminocarbonyl-C₁-C₈-alkyl, Carboxyl, Carboxy-C₁-C₈-alkyl, Phenyl, Phenoxy, Phenylthio, Phenylamino, Phenyl-C₁-C₈-alkyl, Phenyl-C₁-C₈-alkoxy, 3- bis 6-gliedriges Heterocyclyl und 5- oder 6-gliedriges Heteroaryl, wobei die acht letztgenannten Reste ihrerseits partiell oder vollständig halogeniert sein können und/oder ein bis drei Substituenten aus der Gruppe Nitro, Cyano, C₁-C₈-Alkyl, C₁-C₈-Alkoxy, C₁-C₈-Halogenalkyl, C₁-C₈-Halogenalkoxy, C₁-C₈-Alkylthio, C₁-C₈-Halogenalkylthio, C₁-C₈-Alkylsulfinyl, C₁-C₈-Halogenalkylsulfinyl, C₁-C₈-Alkylsulfonyl und C₁-C₈-Halogenalkylsulfonyl tragen können;
bedeutet.

4. 3-Heteroarylsubstituierte Isoxazoline der Formel I gemäß Anspruch 1, in der
X Pyridyl, welches partiell oder vollständig halogeniert ist und/oder ein bis zwei Substituenten aus folgender Gruppe trägt: Cyano, Nitro, Hydroxy, C₁-C₈-Alkyl, C₃-C₆-Cycloalkyl, C₃-C₆-Halogencycloalkyl, C₂-C₈-Alkenyl, C₂-C₈-Alkinyl, C₁-C₈-Halogenalkyl, C₂-C₆-Halogenalkenyl, C₂-C₆-Halogenalkinyl, C₁-C₈-Cyanoalkyl, C₂-C₈-Cyanoalkenyl, C₁-C₈-Alkoxy, C₁-C₈-Halogenalkoxy, C₁-C₈-Alkoxy- C₁-C₈-alkyl, C₁-C₈-Alkoxy-C₁-C₈-alkoxy, Amino, C₁-C₈-Alkylamino, Di(C₁-C₈-alkyl)amino, C₁-C₈-Alkylcarbonylamino, N-C₁-C₈-Alkylcarbonyl-N-C₁-C₈-alkylamino, C₁-C₈-Alkoxycarbonylamino, N-C₁-C₈-Alkoxycarbonyl-N-C₁-C₈-alkylamino, Amino-C₁-C₈-alkyl, C₁-C₈-Alkylamino-C₁-C₈-alkyl, Di(C₁-C₈-alkyl) amino-C₁-C₈-alkyl, C₁-C₈-Alkylthio, C₁-C₈-Halogenalkylthio, C₁-C₈-Alkylthio-C₁-C₈-alkyl, C₁-C₈-Alkylsulfinyl, C₁-C₈-Halogenalkylsulfinyl, C₁-C₈-Alkylsulfonyl, C₁-C₈-Halogenalkylsulfonyl, C₁-C₈-Alkylsulfonyl-C₁-C₈-alkyl, C₁-C₈-Alkylcarbonyl, C₁-C₈-Alkoxycarbonyl, C₁-C₈-Alkoxycarbonyl-C₁-C₈-alkyl, Aminocarbonyl, C₁-C₈-Alkylaminocarbonyl, Di(C₁-C₈-alkyl)aminocarbonyl, Aminocarbonyl-C₁-C₈-alkyl, C₁-C₈-Alkylaminocarbonyl-C₁-C₈-alkyl, Di(C₁-C₈-alkyl)aminocarbonyl-C₁-C₈-alkyl, Carboxyl, Carboxy-C₁-C₈-alkyl, Phenyl, Phenoxy, Phenylthio, Phenylamino, Phenyl-C₁-C₈-alkyl, Phenyl-C₁-C₈-alkoxy, 3- bis 6-gliedriges Heterocyclyl und 5-oder 6-gliedriges Heteroaryl, wobei die acht letztgenannten Reste ihrerseits partiell oder vollständig halogeniert sein können und/oder ein bis drei Substituenten aus der Gruppe Nitro, Cyano, C₁-C₈-Alkyl, C₁-C₈-Alkoxy, C₁-C₈-Halogenalkyl, C₁-C₈-Halogenalkoxy, C₁-C₈-Alkylthio, C₁-C₈-Halogenalkylthio, C₁-C₈-Alkylsulfinyl, C₁-C₈-Halogenalkylsulfinyl, C₁-C₈-Alkylsulfonyl und C₁-C₈-Halogenalkylsulfonyl tragen können; und
welches ortho oder meta zu einem Stickstoff mit dem Isoazolingerüst verknüpft ist,
bedeutet.

5. 3-Heteroarylsubstituierte Isoxazoline der Formel I gemäß Anspruch 1, in der
Y Phenyl, das in 2- und/oder 6-Position zur Verknüpfungsstelle halogeniert ist und/oder einen Substituenten aus der Gruppe Cyano, C₁-C₄-Alkyl und C₁-C₄-Halogenalkyl tragen kann,
bedeutet.

6. Verfahren zur Herstellung von 3-heteroarylsubstituierten Isoxazolinen der Formel I gemäß Anspruch 1, **dadurch gekennzeichnet, daß** man
a) 5-Hydroxymechyloxazoline der Formel III 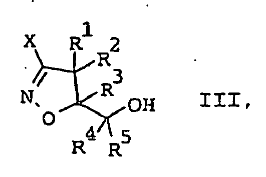 wobei X, R¹-R⁵ die unter Anspruch 1 genannten Bedeutungen haben,
mit Arylmethylderivaten der Formel II 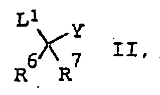 wobei R⁶ und R⁷ die unter Anspruch 1 genannten Bedeutungen haben und L¹ für eine nucleophil verdrängbare Abgangsgruppe steht, oder
b) Methylisoxazolinderivate der Formel IX 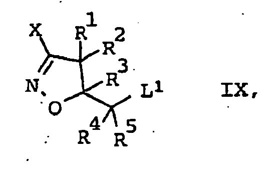 wobei X, R¹-R⁵ die unter Anspruch 1 genannten Bedeutung haben und L² für eine nucleophil verdrängbare Abgangsgruppe steht, mit Arylmethylalkoholen der Formel X 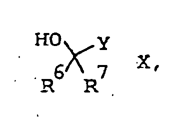 wobei R⁶ und R⁷ die unter Anspruch 1 genannten Bedeutung haben,
umsetzt.

7. Verfahren zur Herstellung von 3-heteroarylsubstituierten Isoxazolinen der Formel I gemäß Anspruch 1, **dadurch gekennzeichnet, daß** man Arylmethylallylether der Formel XI 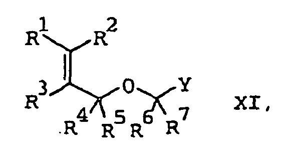 wobei Y, R¹-R⁷ die unter Anspruch 1 genannten Bedeutungen haben,
mit einem Nitriloxid der Formel V 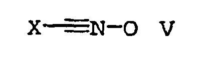 wobei X die unter Anspruch 1 genannten Bedeutungen hat, umsetzt.

8. 5-Hydroxymethyloxazoline der Formel III 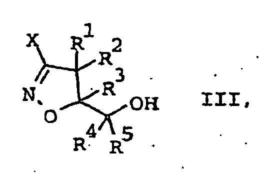 wobei X, R¹-R⁵ die unter Anspruch 1 genannten Bedeutungen haben.

9. Methylisoxazolinderivate der Formel IX 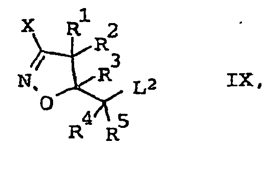 wobei X, R¹-R⁵ die unter Anspruch 1 genannten Bedeutungen haben und L² für Halogen, C₁-C₆-Alkylsulfonyl oder C₁-C₆-Halogenalkylsulfonyloxy steht. .

10. Mittel, enthaltend eine herbizid wirksame Menge mindestens eines 3-heteroarylsubstituierten Isoxazolins der Formel I oder eines landwirtschaftlich brauchbaren Salzes von I gemäß Anspruch 1 und für die Formulierung von Pflanzenschutzmitteln übliche Hilfsmittel.

11. Verfahren zur Herstellung von Mitteln gemäß Anspruch 10, **dadurch gekennzeichnet, daß** man eine herbizid wirksame Menge mindestens eines 3-heteroarylsubstituierten Isoxazolins der Formel I gemäß Anspruch 1 oder eines landwirtschaftlich brauchbaren Salzes von I und für die Formulierung von Pflanzenschutzmitteln übliche Hilfsmittel mischt.

12. Verfahren zur Bekämpfung von unerwünschtem Pflanzenwuchs, **dadurch gekennzeichnet, daß** man eine herbizid wirksame Menge mindestens eines 3-heteroarylsubstituierten Isoxazolins der Formel I gemäß Anspruch 1 oder eines landwirtschaftlich brauchbaren Salzes von I auf Pflanzen, deren Lebensraum und/oder auf Samen einwirken läßt.

13. Verwendung der 3-heteroarylsubstituierten Isoxazoline der Formel I gemäß Anspruch 1 und deren landwirtschaftlich brauchbaren Salze als Herbizide.

## Claims

1. A 3-heteroaryl-substituted isoxazoline of the formula I 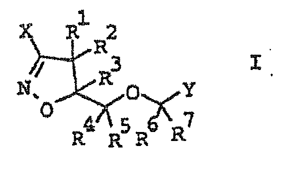 where:
X is 6-membered heteroaryl having one to four nitrogen atoms;
which is partially or fully halogenated and/or carries one to three substituents selected from the following group:
cyano, nitro, hydroxyl, C₁-C₈-alkyl, C₃-C₆-cycloalkyl, C₃-C₆-halocycloalkyl, C₂-C₈-alkenyl, C₂-C₈-alkynyl, C₁-C₈-haloalkyl, C₂-C₆-haloalkenyl, C₂-C₆-haloalkynyl, C₁-C₈-cyanoalkyl, C₂-C₈-cyanoalkenyl, C₁-C₈-alkoxy, C₁-C₈-haloalkoxy, C₁-C₈-alkoxy-C₁-C₈-alkyl, C₁-C₈-alkoxy-C₁-C₈-alkoxy, amino, C₁-C₈-alkylamino, di(C₁-C₈-alkyl) amino, C₁-C₈-alkylcarbonylamino, N-C₁-C₈-alkylcarbonyl-N-C₁-C₈-alkylamino, C₁-C₈-alkoxycarbonylamino, N-C₁-C₈-alkoxycarbonyl-N-C₁-C₈-alkylamino, amino-C₁-C₈-alkyl, C₁-C₈-alkylamino-C₁-C₈-alkyl, di (C₁-C₈-alkyl) amino-C₁-C₈-alkyl, C₁-C₈-alkylthio, C₁-C₈-haloalkylthio, C₁-C₈-alkylthio-C₁-C₈-alkyl, C₁-C₈-alkylsulfinyl, C₁-C₈-haloalkylsulfinyl, C₁-C₈-alkylsulfonyl, C₁-C₈-haloalkylsulfonyl, C₁-C₈-alkylsulfonyl-C₁-C₈-alkyl, C₁-C₈-alkylcarbonyl, C₁-C₈-alkoxycarbonyl, C₁-C₈-alkoxycarbonyl-C₁-C₈-alkyl, aminocarbonyl, C₁-C₈-alkylaminocarbonyl, di (C₁-C₈-alkyl)aminocarbonyl, aminocarbonyl-C₁-C₈-alkyl, C₁-C₈-alkylaminocarbonyl-C₁-C₈-alkyl, di (C₁-C₈-alkyl)aminocarbonyl-C₁-C₈-alkyl, carboxyl, carboxy-C₁-C₈-alkyl, phenyl, phenoxy, phenylthio, phenylamino, phenyl-C₁-C₈-alkyl, phenyl-C₁-C₈-alkoxy, 3- to 6-membered heterocyclyl and 5- or 6-membered heteroaryl, where the eight last-mentioned radicals for their part may be partially or fully halogenated and/or may carry one to three substituents selected from the group consisting of nitro, cyano, C₁-C₈-alkyl , C₁-C₈-alkoxy, C₁-C₈-haloalkyl, C₁-C₈-haloalkoxy, C₁-C₈-alkylthio, C₁-C₈-haloalkylthio, C₁-C₈-alkylsulfinyl, C₁-C₈-haloalkylsulfinyl, C₁-C₈-alkylsulfonyl and C₁-C₈-haloalkylsulfonyl;
R¹ - R⁷ are hydrogen, C₁-C₆-alkyl or C₁-C₆-haloalkyl ;
Y is aryl which may be partially or fully halogenated and/or may carry one to three substituents selected from the group consisting of cyano, nitro, C₁-C₆-alkyl, C₂-C₆-alkenyl, C₂-C₆-alkynyl, C₁-C₆-haloalkyl, C₁-C₆-alkoxy, C₁-C₆-haloalkoxy, C₁-C₆-alkylthio, C₁-C₆-haloalkylthio, C₁-C₆-alkoxycarbonyl, C₁-C₆-alkoxycarbonyl-C₁-C₄-alkoxy, C₁-C₆-alkylsulfonyl, phenyl, phenoxy and phenylcarbonyl, benzo[1,4]dioxonyl, benzo[1,3]di.oxolanyl, 2,3-dihydrobenzofuranyl or benzimidazole; or
5-membered heteroaryl having one to four nitrogen atoms or having one to three nitrogen atoms and one oxygen or sulfur atom or having one oxygen or sulfur atom; or
6-membered heteroaryl having one to four nitrogen atoms;
where the heterocycles may be partially or fully halogenated and/or may carry one to three substituents from the group consisting of cyano, C₁-C₆-alkyl, C₁-C₆-haloalkyl, C₁-C₆-alkoxy-C₁-C₄-alkyl, C₁-C₆-alkoxycarbonyl and C₁-C₆-alkylsulfonyl;
and its agriculturally useful salts.

2. A 3-heteroaryl-substituted isoxazoline of the formula I according to claim 1, in which
X is 6-membered heteroaryl containing one or two nitrogen atoms which is partially or fully halogenated and/or carries, in a position ortho to a nitrogen atom, one or three substituents selected from the following group: cyano, nitro, hydroxyl, C₁-C₆-alkyl, C₁-C₆-haloalkyl, C₂-C₆-alkenyl, C₂-C₆-alkynyl, C₁-C₆-alkoxy, C₁-C₆-haloalkoxy, amino, C₁-C₆-alkylamino, di(C₁-C₆-alkyl) amino, C₁-C₆-alkylthio and C₁-C₆-alkylsulfinyl; and
which is attached to the isoxazoline skeleton ortho or meta to a nitrogen of the 6-membered heteroaryl.

3. A 3-heteroaryl-substituted isoxazoline of the formula I according to claim 1, in which
X is pyridyl which is partially or fully halogenated and/or carries one to two substituents selected from the following group: cyano, nitro, hydroxyl, C₁-C₈-alkyl, C₃-C₆-cycloalkyl, C₃-C₆-halocycloalkyl, C₂-C₈-alkenyl, C₂-C₈-alkynyl, C₁-C₈-haloalkyl, C₂-C₆-haloalkenyl, C₂-C₆-haloalkynyl, C₁-C₈-cyanoalkyl, C₂-C₈-cyanoalkenyl, C₁-C₈-alkoxy, C₁-C₈-haloalkoxy, C₁-C₈-alkoxy-C₁-C₈-alkyl, C₁-C₈-alkoxy-C₁-C₈-alkoxy, amino, C₁-C₈-alkylamino, di(C₁-C₈-alkyl)amino, C₁-C₈-alkylcarbonylamino, N-C₁-C₈-alkylcarbonyl-N-C₁-C₈-alkylamino, C₁-C₈-alkoxycarbonylamino, N-C₁-C₈-alkoxycarbonyl-N-C₁-C₈-alkylamino, amino-C₁-C₈-alkyl, C₁-C₈-alkylamino-C₁-C₈-alkyl, di(C₁-C₈-alkyl) amino-C₁-C₈-alkyl, C₁-C₈-alkylthio,C₁-C₈-haloalkylthio, C₁-C₈-alkylthio-C₁-C₈-alkyl, C₁-C₈-alkylsulfinyl, C₁-C₈-haloalkylsulfinyl, C₁-C₈-alkylsulfonyl, C₁-C₈-haloalkylsulfonyl, C₁-C₈-alkylsulfonyl-C₁-C₈-alkyl, C₁-C₈-alkylcarbonyl, C₁-C₈-alkoxycarbonyl, C₁-C₈-alkoxycarbonyl-C₁-C₈-alkyl, aminocarbonyl, C₁-C₈-alkylaminocarbonyl, di(C₁-C₈-alkyl)aminocarbonyl, aminocarbonyl-C₁-C₈-alkyl, C₁-C₈-alkylaminocarbonyl-C₁-C₈-alkyl, di(C₁-C₈-alkyl)aminocarbonyl-C₁-C₈-alkyl, carboxyl, carboxy-C₁-C₈-alkyl, phenyl, phenoxy, phenylthio, phenylamino, phenyl-C₁-C₈-alkyl, phenyl-C₁-C₈-alkoxy, 3- to 6-membered heterocyclyl and 5- or 6-membered heteroaryl, where the eight last-mentioned radicals for their part may be partially or fully halogenated and/or may carry one to three substituents selected from the group consisting of nitro, cyano, C₁-C₈-alkyl, C₁-C₈-alkoxy, C₁-C₈-haloalkyl, C₁-C₈-haloalkoxy, C₁-C₈-alkylthio, C₁-C₈-haloalkylthio, C₁-C₈-alkylsulfinyl, C₁-C₈-haloalkylsulfinyl, C₁-C₈-alkylsulfonyl, and C₁-C₈-haloalkylsulfonyl.

4. A 3-heteroaryl-substituted isoxazoline of the formula I according to claim 1, in which
X is pyridyl which is partially or fully halogenated and/or carries one to two substituents selected from the following group: cyano, nitro, hydroxyl, C₁-C₈-alkyl, C₃-C₆-cycloalkyl, C₃-C₆--halocycloalkyl, C₂-C₈-alkenyl, C₂-C₈-alkynyl, C₁-C₈-haloalkyl, C₂-C₆-haloalkenyl, C₂-C₆-haloalkynyl, C₁-C₈-cyanoalkyl, C₂-C₈-cyanoalkenyl, C₁-C₈-alkoxy, C₁-C₈-haloalkoxy, C₁-C₈-alkoxy-C₁-C₈-alkyl, C₁-C₈-alkoxy-C₁-C₈-alkoxy, amino, C₁-C₈-alkylamino, di(C₁-C₈-alkyl)amino, C₁-C₈-alkylcarbonylamino, N-C₁-C₈-alkylcarbonyl-N-C₁-C₈-alkylamino, C₁-C₈-alkoxycarbonylamino, N-C₁-C₈-alkoxycarbonyl-N-C₁-C₈-alkylamino, amino-C₁-C₈-alkyl, C₁-C₈-alkylamino-C₁-C₈-alkyl, di (C₁-C₈-alkyl) amino-C₁-C₈-alkyl , C₁-C₈-alkylthio, C₁-C₈-haloalkylthio, C₁-C₈-alkylthio-C₁-C₈-alkyl, C₁-C₈-alkylsulfinyl, C₁-C₈-haloalkylsulfinyl, C₁-C₈-alkylsulfonyl, C₁-C₈-haloalkylsulfonyl, C₁-C₈-alkylsulfonyl-C₁-C₈-alkyl, C₁-C₈-alkylcarbonyl, C₁-C₈-alkoxycarbonyl, C₁-C₈-alkoxycarbonyl-C₁-C₈-alkyl, aminocarbonyl, C₁-C₈-alkylaminocarbonyl, di (C₁-C₈-alkyl) aminocarbonyl, aminocarbonyl-C₁-C₈-alkyl , C₁-C₈-alkylaminocarbonyl-C₁-Cₐ-alkyl, di(C₁-C₈-alkyl)aminocarbonyl-C₁-C₈-alkyl, carboxyl, carboxy-C₁-C₈-alkyl, phenyl, phenoxy, phenylthio, phenylamino, phenyl-C₁-C₈-alkyl, phenyl-C₁-C₈-alkoxy, 3- to 6-membered heterocyclyl and 5- or 6-membered heteroaryl, where the eight last-mentioned radicals for their part may be partially or fully halogenated and/or may carry one to three substituents selected from the group consisting of nitro, cyano, C₁-C₈-alkyl, C₁-C₈-alkoxy, C₁-C₈-haloalkyl, C₁-C₈-haloalkoxy, C₁-C₈-alkylthio, C₁-C₈-haloalkylthio, C₁-C₈-alkylsulfinyl, C₁-C₈-haloalkylsulfinyl, C₁-C₈-alkylsulfonyl, and C₁-C₈-haloalkylsulfonyl; and
which is attached to the isoxazoline skeleton ortho or meta to a nitrogen.

5. A 3-heteroaryl-substituted isoxazoline of the formula I according to claim 1, in which
Y is phenyl which is halogentated in the 2 and/or 6 position to the point of attachment and/or may carry a substituent selected from the group consisting of cyano, C₁-C₄-alkyl and C₁-C₄-haloalkyl.

6. A process for preparing a 3-heteroaryl-substituted isoxazoline of the formula I according to claim 1, which comprises reacting
a) a 5-hydroxymethyloxazoline of the formula III 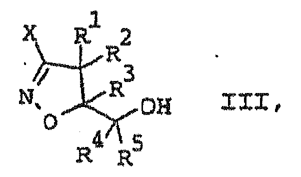 where X and R¹-R⁵ are as defined in claim 1,
with an arylmethyl derivative of the formula II 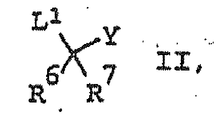 where R⁶ and R⁷ are as defined in claim 1 and L¹ is a nucleophilically displaceable leaving group, or
b) a methylisoxazoline derivative of the formula IX 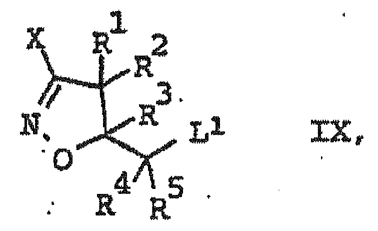
where X and R¹-R⁵ are as defined in claim 1 and L² is a nucleophilically displaceable leaving group,
with an arylmethyl alcohol of the formula X 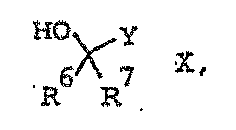 where R⁶ and R⁷ are as defined in claim 1.

7. A process for preparing a 3-heteroaryl-substituted isoxazoline of the formula I according to claim 1, which comprises reacting an arylmethyl allyl ether of the formula XI 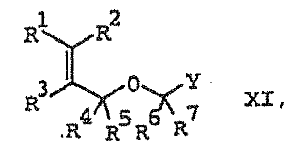 where Y and R¹-R⁷ are as defined in claim 1
with a nitrile oxide of the formula V 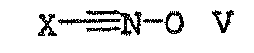 where X is as defined in claim 1.

8. A 5-hydroxymethyloxazoline of the formula III 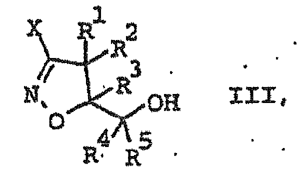 where X and R¹-R⁵ are as defined in claim 1.

9. A methylisoxazoline derivative of the formula IX 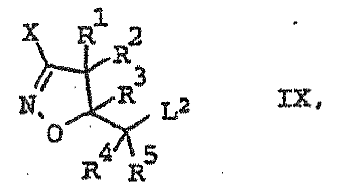 where X and R¹-R⁵ are as defined in claim 1 and L² is halogen, C₁-C₆-alkylsulfonyl or C₁-C₆-haloalkylsulfonyloxy.

10. A composition, comprising a herbicidally effective amount of at least one 3-heteroaryl-substituted isoxazoline of the formula I or an agriculturally useful salt of I according to claim 1 and auxiliaries customary for formulating crop protection agents.

11. A process for preparing a composition according to claim 10, which comprises mixing a herbicidally effective amount of at least one 3-heteroaryl-substituted isoxazoline of the formula I according to claim 1 or an agriculturally useful salt of I and auxiliaries customary for formulating crop protection agents.

12. A method for controlling unwanted vegetation, which comprises allowing a herbicidally effective amount of at least one 3-heteroaryl-substituted isoxazoline of the formula I according to claim 1 or an agriculturally useful salt of I to act on plants, their habitat and/or on seeds.

13. The use of the 3-heteroaryl-substituted isoxazolines of the formula I according to claim 1 and their agriculturally useful salts as herbicides.

## Revendications

1. Isoxazolines à substitution 3-hétéroaryle de formule I 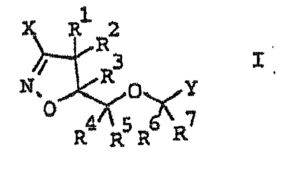 dans laquelle les variables ont les significations suivantes :
X représente un groupe hétéroaryle à 6 chaînons, comportant de un à quatre atomes d'azote, qui est partiellement ou totalement halogéné et/ou porte un à trois substituants choisis dans l'ensemble suivant :
cyano, nitro, hydroxy, alkyle en C₁-C₈, cycloalkyle en C₃-C₆, halogénocycloalkyle en C₃-C₆, alcényle en C₂-C₈, alcynyle en C₂-C₈, halogénoalkyle en C₁-C₈, halogénoalcényle en C₂-C₆, halogénoalcynyle en C₂-C₆, cyanoalkyle(C₁-C₈), cyanoalcényle(C₂-C₈), alcoxy en C₁-C₈, halogénoalcoxy en C₁-C₈, alcoxy(C₁-C₈)-alkyle(C₁-C₈), alcoxy(C₁-C₈)-alcoxy(C₁-C₈), amino, alkyl(C₁-C₈)amino, dialkyl(C₁-C₈)amino, alkyl(C₁-C₈-carbonylamino, N-alkyl(C₁-C₈)carbonyl-N-alkyl(C₁-C₈)amino, alcoxy(C₁-C₈)carbonylamino, N-alcoxy(C₁-C₈)carbonyl-N-alkyl(C₁-C₈)amino, amino-alkyle (C₁-C₈) , alkyl(C₁-C₈) amino-alkyle (C₁-C₈) , dialkyl(C₁-C₈)amino-alkyle (C₁-C₈), alkyl(C₁-C₈)thio, halogénoalkyl (C₁-C₈) thio, alkyl (C₁-C₈) thio-alkyle (C₁-C₈) , alkyl(C₁-C₈)sulfinyle, halogénoalkyl (C₁-C₈) sulfinyle, alkyl(C₁-C₈)sulfonyle, halogénoalkyl (C₁-C₈) sulfonyle, alkyl(C₁-C₈)sulfonyl-alkyle(C₁-C₈), alkyl(C₁-C₈)carbonyle, alcoxy (C₁-C₈) carbonyle, alcoxy (C₁-C₈) carbonyl-alkyle (C₁-C₈), aminocarbonyle, alkyl(C₁-C₈)aminocarbonyle, dialkyl(C₁-C₈)aminocarbonyle, aminocarbonylalkyle(C₁-C₈), alkyl(C₁-C₈)aminocarbonylalkyle (C₁-C₈), dialkyl (C₁-C₈) aminocarbonylalkyle(C₁-C₈), carboxy, carboxy-alkyle (C₁-C₈), phényle, phénoxy, phénylthio, phénylamino, phényl-alkyle (C₁-C₈), phényl-alcoxy(C₁-C₈), hétérocyclyle à 3-6 chaînons et hétéroaryle à 5 ou 6 chaînons, les huit radicaux nommés en dernier pouvant pour leur part être partiellement ou totalement halogénés et/ou pouvant porter un à trois substituants choisis dans l'ensemble constitué par les groupes nitro, cyano, alkyle en C₁-C₈, alcoxy en C₁-C₈, halogénoalkyle en C₁-C₈, halogénoalcoxy en C₁-C₈, alkyl(C₁-C₈)thio, halogénoalkyl(C₁-C₈)thio, alkyl(C₁-C₈)sulfinyle, halogénoalkyl(C₁-C₈)sulfinyle, alkyl (C₁-C₈) sulfonyle et halogénoalkyl(C₁-C₈)sulfonyle ;
R¹ - R⁷ représentent un atome d'hydrogène, un groupe alkyle en C₁-C₆ ou halogénoalkyle en C₁-C₆ ;
Y représente un groupe aryle qui peut être partiellement ou totalement halogéné et/ou peut porter un à trois substituants choisis dans l'ensemble constitué par les groupes cyano, nitro, alkyle en C₁-C₆, alcényle en C₂-C₆, alcynyle en C₂-C₆, halogénoalkyle en C₁-C₆, alcoxy en C₁-C₆, halogénoalcoxy en C₁-C₆, alkyl(C₁-C₆)thio, halogénoalkyl(C₁-C₆)thio, alcoxy(C₁-C₆) carbonyle, alcoxy(C₁-C₆)carbonyl-alcoxy(C₁-C₄), alkyl(C₁-C₆)sulfonyle, phényle, phénoxy et phénylcarbonyle, benzo[1,4]dioxonyle, benzo[1,3]dioxolanyle, 2,3-dihydrobenzofurannyle ou benzimidazole ; ou
hétéroaryle à 5 chaînons, comportant un à quatre atomes d'azote ou comportant un à trois atomes d'azote et un atome d'oxygène ou de soufre, ou comportant un atome d'oxygène ou de soufre ; ou
hétéroaryle à 6 chaînons, ayant de un à quatre atomes d'azote ;
les hétérocycles pouvant être partiellement ou totalement halogénés et/ou pouvant porter un à trois substituants choisis dans l'ensemble constitué par les groupes cyano, alkyle en C₁-C₆, halogénoalkyle en C₁-C₆, alcoxy (C₁-C₆) -alkyle (C₁-C₄) , alcoxy (C₁-C₆)carbonyle et alkyl(C₁-C₆)-sulfonyle ;
ainsi que leurs sels utilisables en agriculture.

2. Isoxazolines à substitution 3-hétéroaryle de formule I selon la revendication 1, dans lesquelles
X représente un groupe hétéroaryle à 6 chaînons, comportant un ou deux atomes d'azote, qui est partiellement ou totalement halogéné et/ou porte un ou deux substituants en position ortho par rapport à un atome d'azote, choisis dans l'ensemble suivant : cyano, nitro, hydroxy, alkyle en C₁-C₆, halogénoalkyle en C₁-C₆, alcényle en C₂-C₆, alcynyle en C₂-C₆, alcoxy en C₁-C₆, halogénoalcoxy en C₁-C₆, amino, alkyl(C₁-C₆)amino, dialkyl(C₁-C₆)amino, alkyl(C₁-C₆)thio et alkyl(C₁-C₆)sulfinyle ; et qui est lié au squelette isoxazoline en position ortho ou méta par rapport à un atome d'azote du groupe hétéroaryle à 6 chaînons.

3. Isoxazolines à substitution 3-hétéroaryle de formule I selon la revendication 1, dans lesquelles
X représente un groupe pyridyle qui est partiellement ou totalement halogéné et/ou porte un à trois substituants choisis dans l'ensemble suivant : cyano, nitro, hydroxy, alkyle en C₁-C₈, cycloalkyle en C₃-C₆, halogénocycloalkyle en C₃-C₆, alcényle en C₂-C₈, alcynyle en C₂-C₈, halogénoalkyle en C₁-C₈, halogénoalcényle en C₂-C₆, halogénoalcynyle en C₂-C₆, cyanoalkyle(C₁-C₈), cyanoalcényle(C₂-C₈), alcoxy en C₁-C₈, halogénoalcoxy en C₁-C₈, alcoxy(C₁-C₈)alkyle (C₁-C₈) , alcoxy (C₁-C₈) -alcoxy (C₁-C₈) , amino, alkyl(C₁-C₈)amino, dialkyl(C₁-C₈)amino, alkyl(C₁-C₈)-carbonylamino, N-alkyl(C₁-C₈)carbonyl-N-alkyl (C₁-C₈) amino, alcoxy(C₁-C₈)carbonylamino, N-alcoxy(C₁-C₈)carbonyl-N-alkyl(C₁-C₈)amino, amino-alkyle (C₁-C₈) , alkyl(C₁-C₈)amino-alkyle(C₁-C₈), dialkyl(C₁-C₈)amino-alkyle(C₁-C₈), alkyl (C₁-C₈) thio, halogénoalkyl (C₁-C₈) thio, alkyl(C₁-C₈)thio-alkyle(C₁-C₈), alkyl (C₁-C₈) sulfinyle, halogénoalkyl (C₁-C₈)sulfinyle, alkyl(C₁-C₈)-sulfonyle, halogénoalkyl(C₁-C₈)sulfonyle, alkyl (C₁-C₈)sulfonyl-alkyle(C₁-C₈), alkyl(C₁-C₈)carbonyle, alcoxy(C₁-C₈) carbonyle, alcoxy(C₁-C₈)carbonylalkyle (C₁-C₈) , aminocarbonyle, alkyl (C₁-C₈)aminocarbonyle, dialkyl(C₁-C₈)aminocarbonyle, aminocarbonyl-alkyle (C₁-C₈) , alkyl(C₁-C₈)aminocarbonyl-alkyle(C₁-C₈), dialkyl(C₁-C₈)aminocarbonyl-alkyle(C₁-C₈), carboxy, carboxyalkyle(C₁-C₈), phényle, phénoxy, phénylthio, phénylamino, phényl-alkyle(C₁-C₈), phénylalcoxy(C₁-C₈), hétérocyclyle à 3-6 chaînons et hétéroaryle à 5 ou 6 chaînons, les huit radicaux nommés en dernier pouvant pour leur part être partiellement ou totalement halogénés et/ou pouvant porter un à trois substituants choisis dans l'ensemble constitué par les groupes nitro, cyano, alkyle en C₁-C₈, alcoxy en C₁-C₈, halogénoalkyle en C₁-C₈, halogénoalcoxy en C₁-C₈, alkyl(C₁-C₈)thio, halogénoalkyl(C₁-C₈)thio, alkyl (C₁-C₈) sulfinyle, halogénoalkyl (C₁-C₈) sulfinyle, alkyl(C₁-C₈)sulfonyle et halogénoalkyl(C₁-C₈)sulfonyle.

4. Isoxazolines à substitution 3-hétéroaryle de formule I selon la revendication 1, dans lesquelles
X représente un groupe pyridyle qui est partiellement ou totalement halogéné et/ou porte un à trois substituants choisis dans l'ensemble suivant : cyano, nitro, hydroxy, alkyle en C₁-C₈, cycloalkyle en C₃-C₆, halogénocycloalkyle en C₃-C₆, alcényle en C₂-C₈, alcynyle en C₂-C₈, halogénoalkyle en C₁-C₈, halogénoalcényle en C₂-C₆, halogénoalcynyle en C₂-C₆, cyanoalkyle(C₁-C₈), cyanoalcényle(C₂-C₈), alcoxy en C₁-C₈, halogénoalcoxy en C₁-C₈, alcoxy (C₁-C₈)alkyle (C₁-C₈) , alcoxy(C₁-C₈)-alcoxy(C₁-C₈), amino, alkyl(C₁-C₈)amino, dialkyl(C₁-C₈)amino, alkyl(C₁-C₈)-carbonylamino, N-alkyl (C₁-C₈)carbonyl-N-alkyl(C₁-C₈)amino, alcoxy (C₁-C₈)carbonylamino, N-alcoxy(C₁-C₈)carbonyl-N-alkyl(C₁-C₈)amino, amino-alkyle(C₁-C₈), alkyl(C₁-C₈) amino-alkyle(C₁-C₈), dialkyl(C₁-C₈) amino-alkyle(C₁-C₈), alkyl(C₁-C₈)thio, halogénoalkyl(C₁-C₈)thio, alkyl(C₁-C₈) thio-alkyle (C₁-C₈) , alkyl(C₁-C₈)sulfinyle, halogénoalkyl(C₁-C₈)sulfinyle, alkyl (C₁-C₈) -sulfonyle, halogénoalkyl(C₁-C₈)sulfonyle, alkyl(C₁-C₈)sulfonyl-alkyle(C₁-C₈), alkyl (C₁-C₈) carbonyle, alcoxy (C₁-C₈) carbonyle, alcoxy(C₁-C₈)carbonyl-alkyle(C₁-C₈), aminocarbonyle, alkyl(C₁-C₈)aminocarbonyle, dialkyl(C₁-C₈)aminocarbonyle, aminocarbonylalkyle(C₁-C₈), alkyl(C₁-C₈) aminocarbonylalkyle(C₁-C₈), dialkyl(C₁-C₈)aminocarbonylalkyle(C₁-C₈), carboxy, carboxy-alkyle(C₁-C₈), phényle, phénoxy, phénylthio, phénylamino, phényl-alkyle(C₁-C₈), phényl-alcoxy(C₁-C₈), hétérocyclyle à 3-6 chaînons et hétéroaryle à 5 ou 6 chaînons, les huit radicaux nommés en dernier pouvant pour leur part être partiellement ou totalement halogénés et/ou pouvant porter un à trois substituants choisis dans l'ensemble constitué par les groupes nitro, cyano, alkyle en C₁-C₈, alcoxy en C₁-C₈, halogénoalkyle en C₁-C₈, halogénoalcoxy en C₁-C₈, alkyl(C₁-C₈)thio, halogénoalkyl(C₁-C₈)thio, alkyl(C₁-C₈)sulfinyle, halogénoalkyl(C₁-C₈)sulfinyle, alkyl(C₁-C₈)sulfonyle et halogénoalkyl(C₁-C₈)sulfonyle ; et
qui est lié au squelette isoxazoline en position ortho ou méta par rapport à un atome d'azote.

5. Isoxazolines à substitution 3-hétéroaryle de formule I selon la revendication 1, dans lesquelles
Y représente un groupe phényle qui est halogéné en position 2 et/ou en position 6 par rapport au point de liaison et/ou peut porter un substituant choisi dans l'ensemble constitué par les groupes cyano, alkyle en C₁-C₄ et halogénoalkyle en C₁-C₄.

6. Procédé pour la préparation d'isoxazolines à substitution 3-hétéroaryle de formule I selon la revendication 1, **caractérisé en ce que**
a) on fait réagir des 5-hydroxyméthyloxazolines de formule III 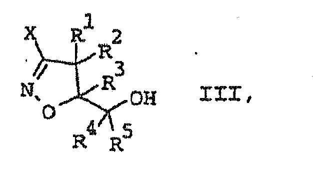 dans laquelle X, R¹-R⁵ ont les significations données dans la revendication 1,
avec des dérivés arylméthyle de formule II 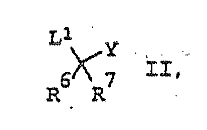 dans laquelle R⁶ et R⁷ ont les significations données dans la revendication 1 et L¹ représente un groupe partant déplaçable par substitution nucléophile, ou
b) on fait réagir des dérivés de méthylisoxazoline de formule IX 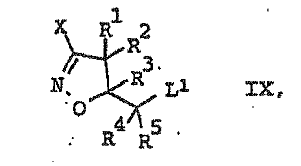
dans laquelle X, R¹-R⁵ ont les significations données dans la revendication 1 et L² représente un groupe partant déplaçable par substitution nucléophile,
avec des alcools arylméthyliques de formule X 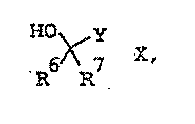 dans laquelle R⁶ et R⁷ ont les significations données dans la revendication 1.

7. Procédé pour la préparation d'isoxazolines à substitution 3-hétéroaryle de formule I selon la revendication 1, **caractérisé en ce qu'**on fait réagir des arylméthylallyléthers de formule XI 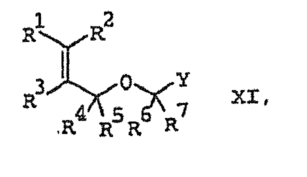 dans laquelle Y, R¹-R⁷ ont les significations données dans la revendication 1,
avec un nitrile-oxyde de formule V 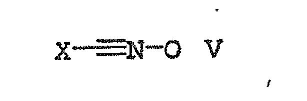 dans laquelle X a les significations données dans la revendication 1.

8. 5-hydroxyméthyloxazolines de formule III 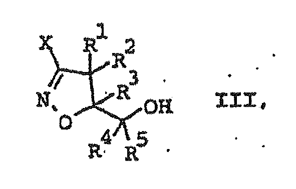 dans laquelle X, R¹-R⁵ ont les significations données dans la revendication 1.

9. Dérivés de méthylisoxazoline de formule IX 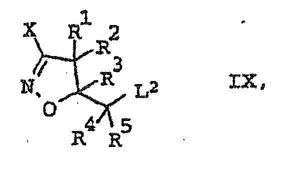 dans laquelle X, R¹-R⁵ ont les significations données dans la revendication 1 et L² représente un atome d'halogène, un groupe alkyl(C₁-C₆)-sulfonyle ou halogénoalkyl(C₁-C₆)sulfonyloxy.

10. Compositions contenant une quantité à effet herbicide d'au moins une isoxazoline à substitution 3-hétéroaryle de formule I ou d'un sel de I, utilisable en agriculture, selon la revendication 1, et des adjuvants usuels pour la formulation de produits phytosanitaires.

11. Procédé pour la préparation de compositions selon la revendication 10, **caractérisé en ce qu'**on mélange une quantité à effet herbicide d'au moins une isoxazoline à substitution 3-hétéroaryle de formule I selon la revendication 1, ou d'un sel de I, utilisable en agriculture, et des adjuvants usuels pour la formulation de produits phytosanitaires.

12. Procédé pour la lutte contre une croissance indésirable de plantes, **caractérisé en ce qu'**on laisse agir sur des plantes, leur habitat et/ou sur des semences une quantité à effet herbicide d'au moins une isoxazoline à substitution 3-hétéroaryle de formule I selon la revendication 1, ou d'un sel de I, utilisable en agriculture.

13. Utilisation des isoxazolines à substitution 3-hétéroaryle de formule I selon la revendication 1 et de leurs sels utilisables en agriculture, en tant qu'herbicides.
